(19) Europäisches Patentamt
European Patent Office
Office européen des brevets

(11) Veröffentlichungsnummer: **0 430 884 B1**

(12) **EUROPÄISCHE PATENTSCHRIFT**

(45) Veröffentlichungstag der Patentschrift: **11.01.95**

(51) Int. Cl.⁶: **C07H 19/01**, A61K 31/70, A01N 43/90

(21) Anmeldenummer: **90810906.9**

(22) Anmeldetag: **22.11.90**

(54) **Insektizide und Parasitizide.**

(30) Priorität: **01.12.89 CH 4298/89**

(43) Veröffentlichungstag der Anmeldung:
**05.06.91 Patentblatt 91/23**

(45) Bekanntmachung des Hinweises auf die Patenterteilung:
**11.01.95 Patentblatt 95/02**

(84) Benannte Vertragsstaaten:
**AT BE CH DE DK ES FR GB GR IT LI LU NL SE**

(56) Entgegenhaltungen:
**EP-A- 0 184 173**
**EP-A- 0 252 879**
**EP-A- 0 357 460**

(73) Patentinhaber: **CIBA-GEIGY AG**
**Klybeckstrasse 141**
**CH-4002 Basel (CH)**

(72) Erfinder: **O'Sullivan, Anthony Cornelius, Dr.**
**Habsburgerstrasse 38**
**CH-4055 Basel (CH)**

**EP 0 430 884 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 13$\beta$-Phenethylthio-Milbemycin-Derivate der nachstehenden Formel I, deren Herstellung und Verwendung zur Bekämpfung von Insekten sowie Ekto- und Endoparasiten am Nutztier. Sie betrifft auch die Verwendung besagter, neuer Milbemycin-Derivate zur Herstellung von Tierarzneimitteln und zur Anwendung bei der Behandlung von Tieren.

Die neuen Verbindungen haben die allgemeine Formel I

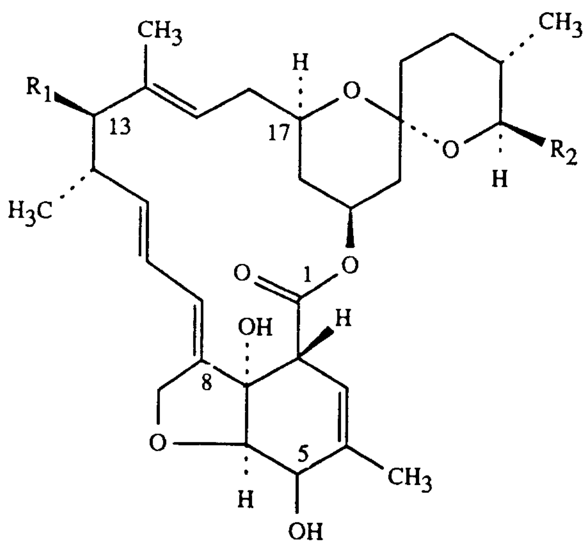

(I),

worin
$R_2$ für Methyl, Ethyl, Isopropyl, sek.Butyl oder für die Gruppe $-C(CH_3)=CHQ$ steht,
wobei
Q Methyl, Ethyl oder Isopropyl bedeutet;
und
$R_1$ die Gruppe

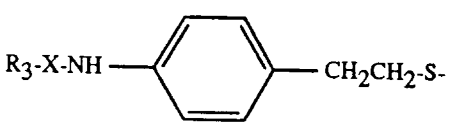

repräsentiert;
wobei X für eine der Gruppen -C(O), -OC(O)-, -NHC(O)-, -NHC(S)- oder $-SO_2-$ steht;
und
$R_3$ für Wasserstoff, $Het_1$, unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl;
unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl; oder für die Gruppe

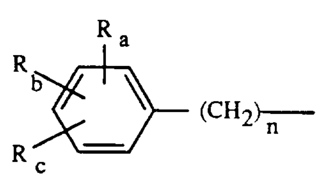

steht, wobei
n für 0, 1 oder 2 steht,
$R_a$, $R_b$ und $R_c$ unabhängig voneinander Wasserstoff, $C_1$-$C_4$-Alkyl, $C_1$-$C_4$-Alkoxy, $C_1$-$C_4$-Alkylthio, $C_1$-$C_4$-

Haloalkyl, $C_1$-$C_4$-Haloalkoxy, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, Halogen, CN, $NO_2$ oder $NH_2$ bedeuten, wobei $R_c$ zusätzlich für $C_3$-$C_8$-Cycloalkyl oder für die Gruppe

$Het_2$-O-

stehen kann, wobei $Het_1$ und $Het_2$ für einen unsubstituierten oder substituierten, über Kohlenstoff gebundenen Heterocyclus stehen, ausgewählt aus der Reihe Benzimidazol, Benzoxazol, Benzthiazol, Imidazol, Oxazol, Thiazol, Oxadiazol, Thiadiazol, Triazol, Pyridin, Pyridazin, Pyrimidin, Pyrazin, Triazin, Chinolin, Isochinolin, Chinoxalin, Phthalazin, Chinazolin und Benzotriazin; unter Einschluss ihrer physiologisch verträglichen Säureadditionssalze.

Wenn $Het_1$ oder $Het_2$ für ein benzokondensiertes System steht, ist stets der heterocyclische Molekülteil mit dem Rest des Gesamtmoleküls verbunden.

Die Heterocyclen $Het_1$ und $Het_2$ sind stets über ein Ringkohlenstoffatom an das restliche Molekül gebunden. Sie können ihrerseits unsubstituiert oder substituiert vorliegen. Verbindungen der Formel I, worin Het, welches sowohl für $Het_1$ wie für $Het_2$ steht, beispielsweise eine der nachfolgend angegebenen Bedeutungen hat, bilden je nach Definition von Het eine der folgenden erfindungsgemässen bevorzugten Gruppen a bis u:

| Gruppe | Het | Typ |
|---|---|---|
| a) | 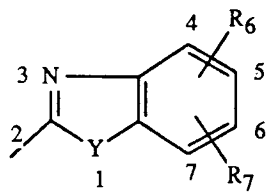 | Benzimidazol-2-yl<br>Benzoxazol-2-yl<br>Benzthiazol-2-yl |
| b) | 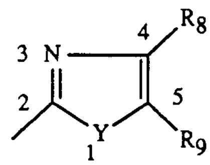 | Imidazol-2-yl<br>Oxazol-2-yl<br>Thiazol-2-yl |
| c) | 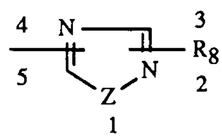 | [1H-1,2,4-Triazol]-5-yl<br>[1,2,4-Oxadiazol]-5-yl<br>[1,2,4-Thiadiazol]-5-yl<br>[1,2,4-Oxadiazol]-3-yl<br>[1,2,4-Thiadiazol]-3-yl |
| d) | 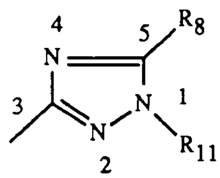 | [1H-1,2,4-Triazol]-3-yl |
| e) | 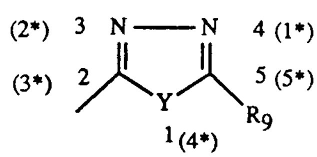 | [1,3,4-Oxadiazol]-2-yl<br>[1,3,4-Thiadiazol]-2-yl<br>* [4H-1,2,4-Triazol]-3-yl |
| f) | 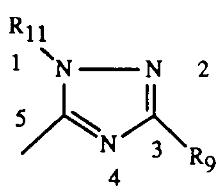 | [1H-1,2,4-Triazol]-5-yl |
| g) | 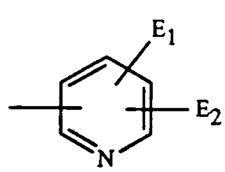 | Pyridin-2-yl<br>Pyridin-3-yl<br>Pyridin-4-yl |

| Gruppe | Het | Typ |
| --- | --- | --- |
| h) | 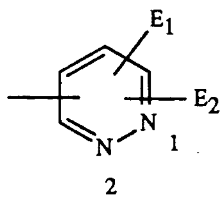 | Pyridazin-3-yl<br>Pyridazin-4-yl |
| i) | 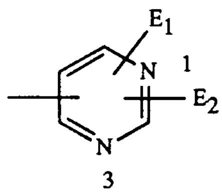 | Pyrimidin-2-yl<br>Pyrimidin-4-yl<br>Pyrimidin-5-yl |
| k) | 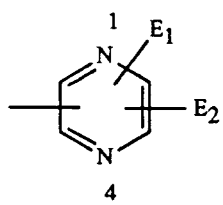 | Pyrazin-2-yl |
| l) | 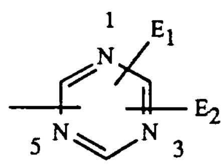 | s-Triazin-2-yl |
| m) | 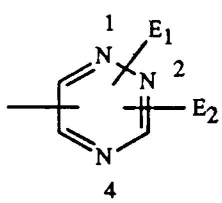 | [1,2,4-Triazin]-3-yl<br>[1,2,4-Triazin]-5-yl<br>[1,2,4-Triazin]-6-yl |
| n) | 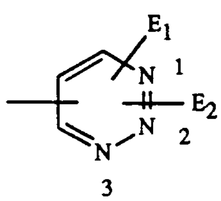 | [1,2,3-Triazin]-4-yl<br>[1,2,3-Triazin]-5-yl |
| o) | 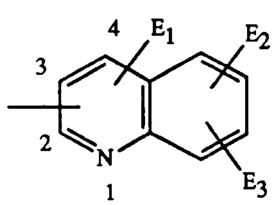 | Chinolin-2-yl<br>Chinolin-3-yl<br>Chinolin-4-yl |

| Gruppe | Het | Typ |
|---|---|---|
| p) | 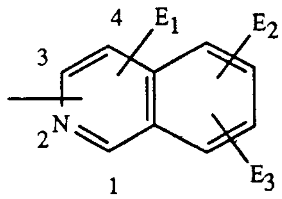 | Isochinolin-1-yl<br>Isochinolin-3-yl<br>Isochinolin-4-yl |
| q) | 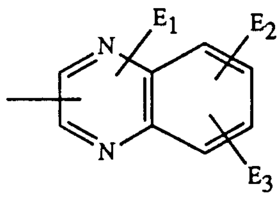 | Chinoxalin-2-yl |
| r) | 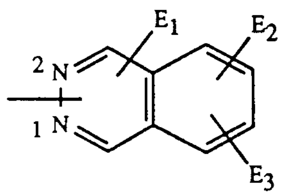 | Phthalazin-3-yl |
| s) | 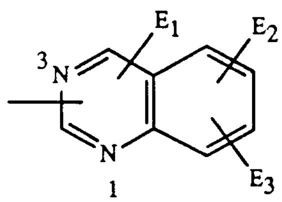 | Chinazolin-2-yl<br>Chinazolin-4-yl |
| t) | 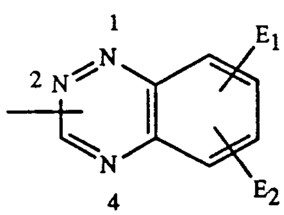 | [1,2,4-Benzotriazin]-3-yl |
| u) | 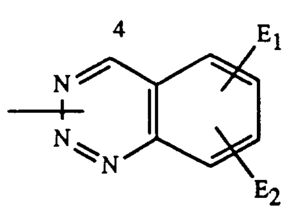 | [1,2,3-Benzotriazin]-4-yl |

wobei

Y für Sauerstoff, Schwefel oder $NR_{10}$ steht;

Z für Sauerstoff, Schwefel oder NH steht;

$R_6$ und $R_7$ unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, Halogen, Nitro oder Cyano bedeuten;

$R_8$ und $R_9$ unabhängig voneinander für Wasserstoff, $C_1$-$C_6$-Alkyl, $C_1$-$C_6$-Haloalkyl, $C_1$-$C_6$-Alkoxy, $C_1$-$C_6$-

Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen;
$R_{10}$ Wasserstoff oder $C_1$-$C_6$-Alkyl bedeutet;
$R_{11}$ für $C_1$-$C_6$-Alkyl steht;
und
$E_1$, $E_2$ und $E_3$ unabhängig voneinander für Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, $C_3$-$C_8$-Cycloalkyl, Halogen, CN, $NO_2$ oder $NH_2$ stehen.

Innerhalb der Bedeutungen für $E_1$, $E_2$ und $E_3$ gelten als besonders bevorzugt: Wasserstoff, Methyl, Ethyl, $CF_3$, Methoxy, $OCF_3$, Methylthio, $CH_3OCH_2O$, Cyclopropyl, Fluor, Chlor und Brom.

Innerhalb der Gruppen b bis f sind diejenigen Verbindungen der Formel I interessant, worin Y, Z, $R_{10}$ und $R_{11}$ wie oben definiert sind und $R_8$ und $R_9$ unabhängig voneinander für unsubstituiertes oder durch Halogen oder $C_1$-$C_3$-Alkyl substituiertes $C_3$-$C_7$-Cycloalkyl, vorzugsweise Cyclopropyl oder Cyclohexyl, stehen.

Ausgewählte Beispiele für den Substituenten Het in den Gruppen a bis f sind:

a')

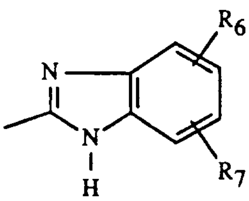 , 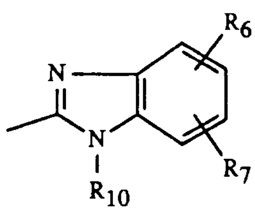 , 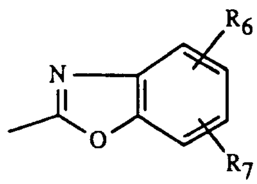 , 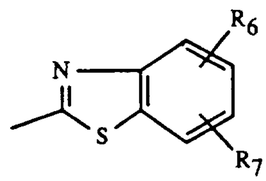 ,

b') 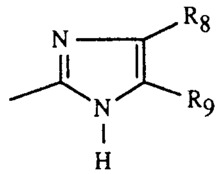 , 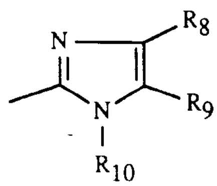 , 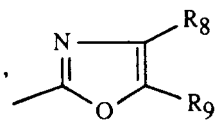 , 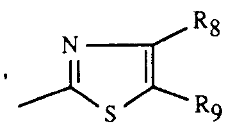 ,

c') 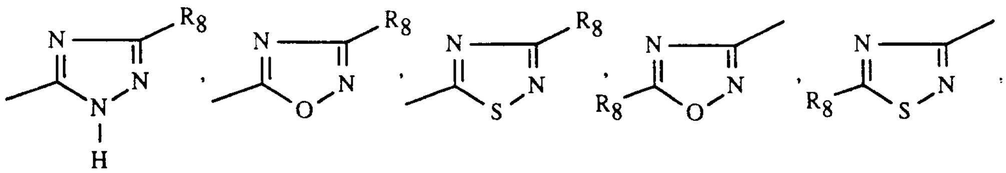

d') 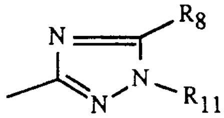

e') 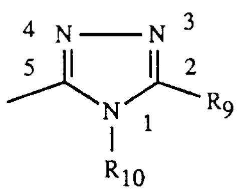 , 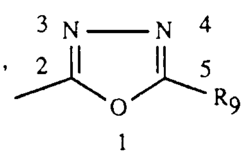 , 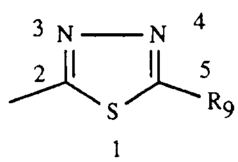 ,

f') 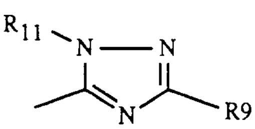 ,

wobei die Substituenten $R_6$ bis $R_{11}$ wie vorstehend definiert sind.

Als weiteres Beispiel (v) ist

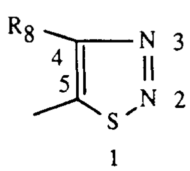

(v)

zu erwähnen, worin $R_8$ die vorstehend angegebenen Bedeutungen hat

Bevorzugt sind alle Gruppen a) bis u) von Verbindungen der Formel I, worin $R_2$ für Methyl oder Ethyl, vor allem aber für Ethyl, steht.

Innerhalb der Gruppe a') sind diejenigen Verbindungen der Formel I zusätzlich bevorzugt, worin $R_6$ und $R_7$ unabhängig voneinander für Wasserstoff, Methyl, $CF_3$, Methoxy, Halomethoxy, Fluor, Chlor oder Brom stehen und die übrigen Substituenten wie für Formel I definiert sind.

Innerhalb der Gruppen b') bis f') sind diejenigen Verbindungen der Formel I zusätzlich bevorzugt, worin $R_8$ und $R_9$ unabhängig voneinander für Wasserstoff, Methyl, Ethyl, $C_3$-$C_7$-Cycloalkyl, $CF_3$, $C_2F_5$, $C_3F_7$, $CCl_3$, $CHCl_2$, $CH_2Cl$, $SCH_3$ oder Halogen stehen; $R_{10}$ Wasserstoff oder Methyl bedeutet und $R_{11}$ für $C_1$-$C_4$-Alkyl, insbesondere Methyl steht.

Besonders bemerkenswert sind Verbindungen der Formel I, worin die Gruppe $Het_2$-O- in para-Stellung an den Phenylrest gebunden ist.

Eine weitere bevorzugte Gruppe bilden diejenigen Verbindungen der Formel I, in denen $R_2$ und $R_3$ unabhängig voneinander für Methyl, Ethyl oder Isopropyl stehen und X die unter Formel I angegebenen Bedeutungen hat.

Unter dem Begriff Alkyl selbst oder als Bestandteil eines anderen Substituenten sind im Rahmen vorliegender Erfindung je nach Anzähl der angegebenen Kohlenstoffatome beispielsweise folgende geradkettige und verzweigte Gruppen zu verstehen: Methyl, Ethyl, n-Propyl, Isopropyl, n-Butyl, sek.-Butyl, tert.Butyl, Isobutyl, etc.. Haloalkyl selbst oder als Bestandteil von Haloalkoxy oder Haloalkylthio steht für einen mono- bis perhalogenierten Alkylsubstituenten, wie z.B. für $CH_2Cl$, $CHCl_2$, $CCl_3$, $CH_2F$, $CHF_2$, $CF_3$, $CH_2Br$, $CHBr_2$, $CBr_3$, $CH_2J$, $CJ_3$, CHClF, CHBrCl, CFBrCl, $C_2F_5$, $CH_2CH_2Cl$, $CHClCH_3$, $C_2Cl_5$, $CHFCHCl_2$, etc., vorzugsweise für $CF_3$. Unter Halogen soll hier und im folgenden Fluor, Chlor, Brom oder Jod, vorzugsweise Fluor, Chlor oder Brom, vor allem jedoch Chlor verstanden werden.

Alkoxycarbonyl steht für eine R-OC(O)-Gruppe, in der R einen Alkylrest repräsentiert, so z.B. für $CH_3OC(O)$-, $C_2H_5OC(O)$-, tert.Butoxy-C(O)-, etc.. Beispiele für $C_2$-$C_6$-Alkoxyalkoxy sind: $CH_3OCH_2O$, $C_2H_5OCH_2O$, $C_2H_5OCH_2CH_2O$, tert.Butoxy-$CH_2CH_2O$, etc..

Cycloalkyl selbst oder als Bestandteil eines Substituenten steht je nach Anzahl der angegebenen Kohlenstoffatome für Cyclopropyl, Cyclobutyl, Cyclopentyl, Cyclohexyl, etc. Cyanoalkyl steht für eine Alkylgruppe, worin ein Wasserstoffatom durch CN substituiert ist, vorzugsweise für eine Alkylgruppe, worin sich die CN-Gruppe am terminalen Kohlenstoffatom befindet.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie z.B. Essigsäure, Trifluoressigsäure, Trichloressigsäure, Propionsäure, Glykolsäure, Thiocyansäure, Milchsäure, Bernsteinsäure, Zitronensäure, Ameisensäure, Benzolsulfonsäure, p-Toluolsulfonsäure, Methansulfonsäure, Salicylsäure, p-Aminosalicylsäure, Phthalsäure, 2-Phenoxybenzoesäure oder 2-Acetoxybenzoesäure.

Die Verbindungen der Formel I liegen bei Raumtemperatur überwiegend als Feststoffe vor. Sie besitzen sehr wertvolle insektizide und parasitizide Eigenschaften und lassen sich zur kurativen und präventiven Bekämpfung einer Reihe von Parasiten bei Warmblütern, insbesondere bei Haus- und Nutztieren, vor allem aus der Klasse der Säugetiere einsetzen.

Eine bevorzugte Untergruppe bilden Verbindungen der Formel I, worin
$R_2$ für Methyl, Ethyl, Isopropyl oder sek.Butyl steht;
$R_1$ die Gruppe

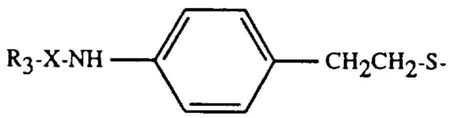

repräsentiert;
wobei X für eine der Gruppen -C(O)-, -OC(O)-, -NHC(O)-, -NHC(S)- oder -$SO_2$- steht;
und
$R_3$ für $C_1$-$C_8$-Alkyl, vorzugsweise $C_1$-$C_4$-Alkyl, oder $C_3$-$C_6$-Cycloalkyl steht.

Eine weitere bevorzugte Untergruppe bilden Verbindungen der Formel I, worin
$R_2$ für Methyl, Ethyl, Isopropyl oder sek.Butyl steht;
$R_1$ die Gruppe

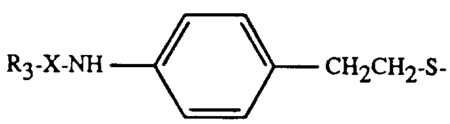

repräsentiert;
wobei X für eine der Gruppen -C(O)-, -OC(O)-, -NHC(O)-, -NHC(S)- oder -$SO_2$- steht;
und
$R_3$ für die Gruppe

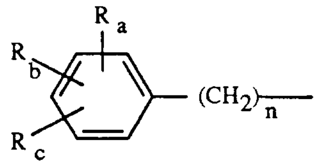

steht, wobei
n für 0 oder 1 steht und
$R_a$, $R_b$ und $R_c$ unabhängig voneinander für Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten und $R_c$ zusätzlich für die Gruppe $Het_2$-O- stehen kann, wobei $Het_2$ die unter Formel I angegebenen Gruppen repräsentiert.

Eine weitere bevorzugte Untergruppe bilden Verbindungen der Formel I, worin
$R_2$ für Methyl, Ethyl, Isopropyl oder sek.Butyl steht;
$R_1$ die Gruppe

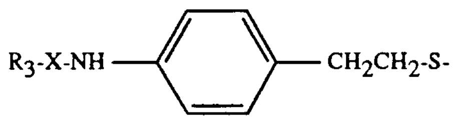

repräsentiert;
wobei X für eine der Gruppen -C(O)-, -OC(O)- oder -NHC(O)- steht; und
$R_3$ für $Het_1$, unsubstituiertes oder durch $C_1$-$C_4$-Alkoxy, $C_1$-$C_4$-Alkylthio oder Halogen substituiertes $C_1$-$C_6$-Alkyl, unsubstituiertes oder durch $C_1$-$C_2$-Alkoxy, $C_1$-$C_2$-Alkylthio oder Halogen substituiertes $C_3$-$C_6$-Cycloalkyl oder die Gruppe

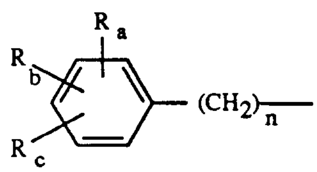

steht, wobei n für 0 oder 1 steht;
$R_a$, $R_b$ und $R_c$ unabhängig voneinander Wasserstoff, Fluor, Chlor, Brom, Methyl, Methoxy, Methylthio, $CF_3$ oder $NO_2$ bedeuten, wobei $R_c$ zusätzlich für die Gruppe

$Het_2$-O-

stehen kann, wobei $Het_1$ und $Het_2$ für eine der vorstehend für Het genannten Gruppen a') bis f') stehen.

Eine weitere bevorzugte Untergruppe bilden Verbindungen der Formel I, worin
$R_2$ für Methyl, Ethyl, Isopropyl oder sek.Butyl steht;
$R_1$ die Gruppe

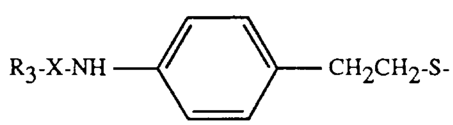

repräsentiert,
wobei X für -OC(O)- steht; und
$R_3$ für unsubstituiertes oder durch $C_1$-$C_4$-Alkoxy, $C_1$-$C_4$-Alkylthio oder Halogen substituiertes $C_1$-$C_6$-Alkyl steht.

Eine andere bevorzugte Untergruppe bilden Verbindungen der Formel I, worin
$R_2$ für Methyl, Ethyl, Isopropyl oder sek.Butyl steht;

$R_1$ die Gruppe

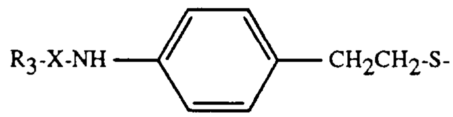

repräsentiert,
wobei X für -C(O)-, -OC(O)- oder -NHC(O)- steht; und
$R_3$ für Wasserstoff; unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl; unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl steht.

Eine weitere bevorzugte Untergruppe bilden Verbindungen der Formel I, worin
$R_2$ für Methyl, Ethyl, Isopropyl oder sek.Butyl steht;
$R_1$ die Gruppe

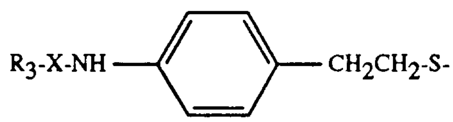

repräsentiert,
wobei X für -C(O)-, -OC(O)- oder -NHC(O)- steht; und $R_3$ für die Gruppe

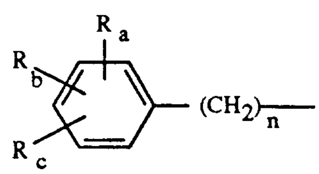

steht, wobei n für 0 oder 1 steht und
$R_a$, $R_b$ und $R_c$ unabhängig voneinander Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten und
$R_c$ zusätzlich für die Gruppe

$Het_2$-O-

stehen kann, wobei $Het_2$ für eine der vorstehend für Het genannten Gruppen a') bis f') steht.

Eine weitere bevorzugte Untergruppe bilden Verbindungen der Formel I, worin
$R_2$ für Methyl, Ethyl, Isopropyl oder sek.Butyl steht;
$R_1$ die Gruppe

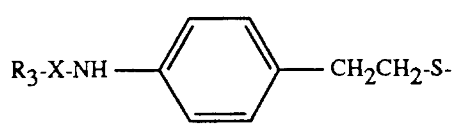

repräsentiert,
wobei X für -C(O)-, -OC(O)- oder -NHC(O)- steht; und $R_3$ für die Gruppe

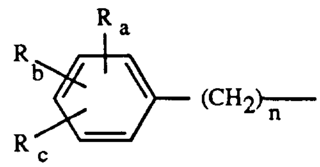

steht, wobei n für 0 oder 1 steht und

$R_a$, $R_b$ und $R_c$ unabhängig voneinander für Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten.

Eine weitere bevorzugte Untergruppe bilden Verbindungen der Formel I, worin

$R_2$ für Methyl oder Ethyl steht;

$R_1$ die Gruppe

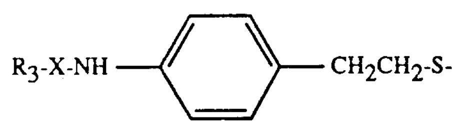

repräsentiert,

wobei X für -OC(O)- steht und $R_3$ $C_1$-$C_6$-Alkyl bedeutet.

Eine weitere bevorzugte Untergruppe bilden Verbindungen der Formel I, worin

$R_2$ für Methyl oder Ethyl steht;

$R_1$ die Gruppe

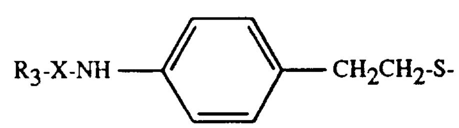

repräsentiert,

wobei X für -OC(O)- steht und $R_3$ für Phenyl oder Benzyl steht.

Zu den bevorzugten Einzelsubstanzen der Formel I gehören folgende Verbindungen:

13β-[2-(4-Acetaminophenyl)ethylthio]milbemycin A4;
13β-(2-[4-(4-Heptylcarbonylamino)phenyl]ethylthio)milbemycin A4;
13β-(2-[4-(2,6-Dichlorpyridin-4-ylcarbonylamino)phenyl]ethylthio)-milbemycin A4;
13β-(2-[4-Tosylphenyl]ethylthio)milbemycin A4;
13β-(2-[4-(Methylaminothiocarbonylamino)phenyl]ethylthio)milbemycin A4;
13β-(2-[4-(Benzoylaminocarbonylamino)phenyl]ethylthio)milbemycin A4;
13β-(2-[4-(Cyclohexylaminocarbonylamino)phenyl]ethylthio)milbemycin A4;
13β-(2-[4-((4-Methoxyphenyl)aminocarbonylamino)phenyl]ethylthio)milbemycin A4;
13β-(2-[4-(Carboxymethylaminocarbonylamino)phenyl]ethylthio)milbemycin A4; und
13β-(2-[4-(Methoxycarbonylamino)phenyl]ethylthio)milbemycin A4.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycinen ($R_2$ = $CH_3$, $C_2H_5$ oder iso$C_3H_7$) ist die 13-Position anstelle der Thioethergruppe ($R_1$) der erfindungsgemässen Verbindungen der Formel I ausschliesslich mit Wasserstoff besetzt, wie die nachstehende Formel XXX zeigt:

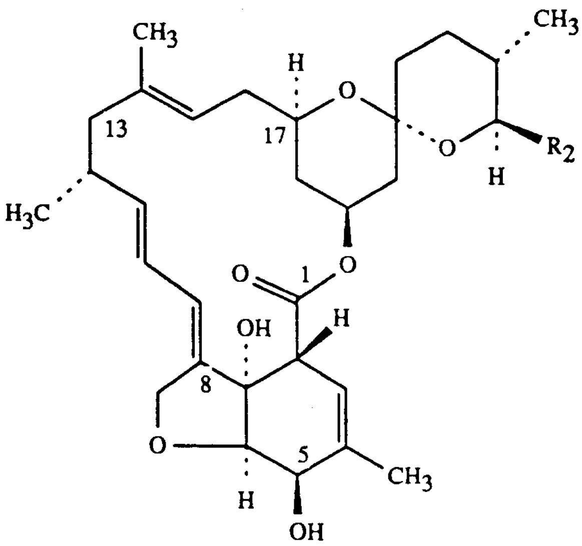

(XXX)

| | |
|---|---|
| $R_2$ = $CH_3$ | Milbemycin $A_3$ |
| $R_2$ = $C_2H_5$ | Milbemycin $A_4$ |
| $R_2$ = $isoC_3H_7$ | Milbemycin D |
| $R_2$ = $sec.C_4H_9$ | 13-Deoxi-22,23-dihydro-C-076-Bla-aglycon. |

Bei Avermectinen dagegen steht in der 13-Position ein $\alpha$-L-Oleandrosyl-$\alpha$-L-oleandrose-Rest, der über Sauerstoff in $\alpha$-Konfiguration mit dem Makrolid-Molekül verknüpft ist. Avermectine unterschieden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C = C-Doppelbindung befindliche 13$\alpha$-Hydroxy-Gruppe besitzen. Die Avermectinen-Aglykone sind, wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor und der Substituent in 13-Position ist immer $\beta$-ständig.

Verbindungen der Formel I, worin $R_2$ für die Gruppe

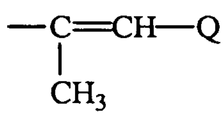

steht und

Q Methyl, Ethyl oder Isopropyl bedeutet, lassen sich als 23-Deoxy-Abkömmlinge der natürlich vorkommenden Antibiotika S541 auffassen, enthalten aber nunmehr in 13-Position eine $\beta$-Thioethergruppe. Auch diese Vettreter der Formel I werden hier und im folgenden einfachheitshalber als Milbemycine bezeichnet.

Die Konstitution von natürlichen Antibiotika S541 ist aus der DE-OS 35 32 794 bekannt und sieht wie folgt aus:

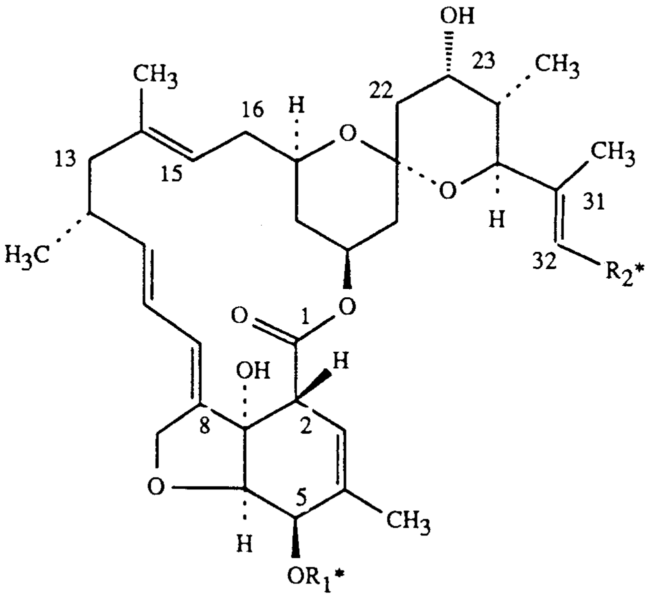

(Antibiotika S541)

| | | |
|---|---|---|
| Faktor A | $R_2^*$ = iso$C_3H_7$ | $R_1^*$ = H |
| Faktor B | $R_2^*$ = $CH_3$ | $R_1^*$ = $CH_3$ |
| Faktor C | $R_2^*$ = $CH_3$ | $R_1^*$ = H |
| Faktor D | $R_2^*$ = $C_2H_5$ | $R_1^*$ = H |
| Faktor E | $R_2^*$ = $C_2H_5$ | $R_1^*$ = $CH_3$ |
| Faktor F | $R_2^*$ = iso$C_3H_7$ | $R_1^*$ = $CH_3$ |

Milbemycine mit einer 13β-Alkylthiogruppe sind als Insektizide und Parasitizide aus der Europäischen Offenlegungsschrift EP-0,184,173 bzw. dem korrespondierenden Britischen Patent GB-2,167,751B bekannt. Die biologische Aktivität der 13β-Ethylthiomilbemycine kann man jedoch überraschenderweise durch Einführung der erfindungsgemässen, terminalen

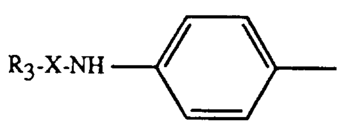

-Gruppe,

(worin $R_3$ und X die unter Formel I angegebenen Bedeutungen haben) in die 2-Position der 13β-Ethylgruppe signifikant erhöhen. Vergleicht man z.B. die Verbindung Nr. 1.14 aus obigen Publikationen

[Substanz A]

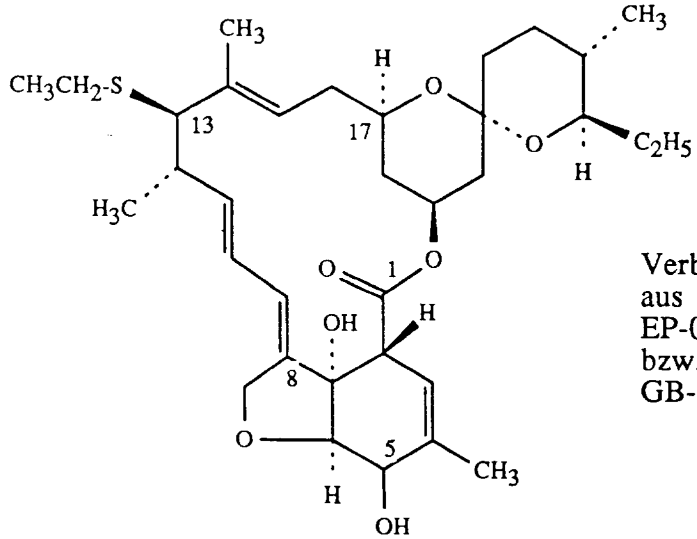

Verb. Nr. 1.14
aus
EP-0,184,173
bzw.
GB-2,167,751B

mit der erfindungsgemässen Substanz Nr. 1.13 (vgl. Tab. 1)

[Substanz B]

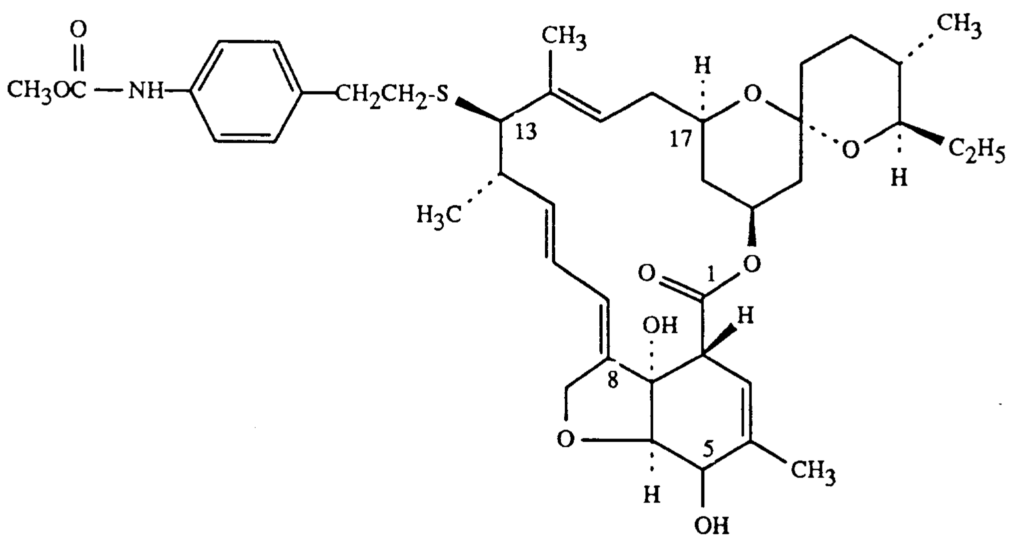

gemäss dem weiter unten beschriebenen biologischen Beispiel B-3 an Schafen, die mit den Nematoden Haemonchus contortus und Trichostrongylus colubriformis infiziert wurden, so findet man bei einmaliger intraruminaler Applikation von 0,1 mg Aktivsubstanz/kg Körpergewicht folgende Resultate (angegeben in Reduktion der Wurmeier im Kot verglichen mit der Zahl der Eier bei infizierten, aber unbehandelten Schafen):

| | Versuch 1 | Versuch 2 (Wiederholung von 1) | Kontrolle |
|---|---|---|---|
| Substanz A des Standes der Technik | 50 % | 58 % | 0 |
| erfindungsgemässe Substanz B | 99 % | 93 % | 0 |

Eine Reduktion von über 90 % entspricht praktisch einer 100 %igen Wirkung insbesondere, da die vereinzelt im Kot aufgefundenen Eier sich als nicht lebensfähig erwiesen, während die Reduktion bei Behandlung mit Substanz A mit weniger als 60 % völlig unzureichend ist und ein Grossteil der im Kot verbleibenden Eier zudem schlupffähig war.

Die Verbindungen der Formel I werden erfindungsgemäss aus Verbindungen der Formel II

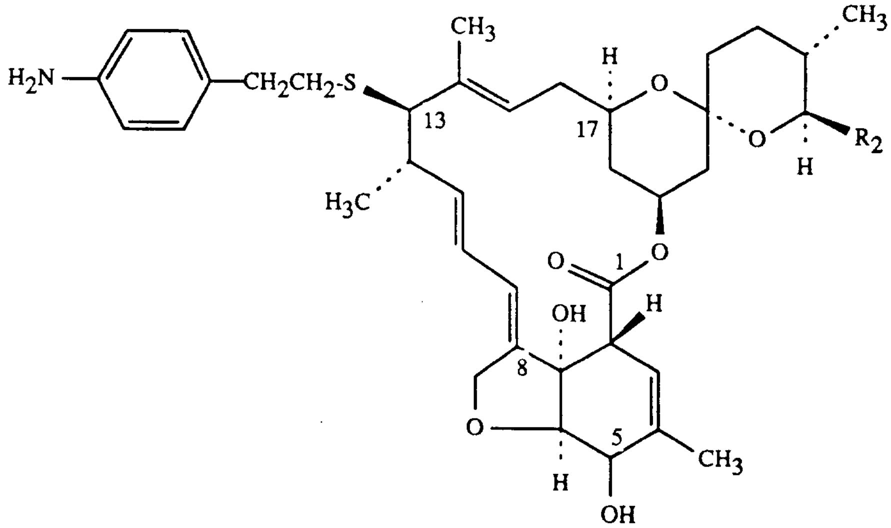

(II),

worin $R_2$ die unter Formel I angegebenen Bedeutungen hat, hergestellt, indem man

α) in den Fällen, in denen X in der Formel I für -C(O)- oder -OC(O)- steht, besagte Verbindung der Formel II in Abwesenheit oder vorzugsweise in Anwesenheit eines Neutralisationsmittels mit einer Verbindung der Formel III

$R_3$-X-T (III)

umsetzt, wobei $R_3$ die unter Formel I angegebenen Bedeutungen hat, X für -C(O)- oder -OC(O)- steht und T eine Abgangsgruppe repräsentiert.

Beispiele von brauchbaren Abgangsgruppen T sind vor allem Halogen, insbesondere Chlor, Brom oder Jod. Als Abgangsgruppe T kommt gegebenenfalls auch eine OH-Gruppe in Betracht.

Dazu gehören ferner auch Gruppen, wie Benzolsulfonyloxy, p-Tosyloxy, Trifluoracetyloxy oder Niederalkylsulfonyloxy, wie z.B. Mesyloxy, oder wenn X in Formel III für -C(O)- steht, der entsprechende Rest -O-C(O)-$R_3$ des Anhydrids einer Carbonsäure, wie beispielsweise der Essigsäure.

Das Verfahren wird im allgemeinen in einem reaktions-inerten Lösungsmittel oder in einem der beteiligten Reaktanden, sofern diese flüssig sind, durchgeführt. Geeignete Lösungsmittel sind z.B.: Ether und etherartige Verbindungen wie Dialkylether (Diethylether, Diisopropylether, tert.-Butylmethylether, Dimethoxyethan, Dioxan, Tetrahydrofuran, Anisol, usw.); halogenierte Kohlenwasserstoffe wie Chlorbenzol, Methylenchlorid, bevorzugt Ethylenchlorid, Chloroform, Tetrachlorkohlenstoff, Tetrachlorethylen, usw.; wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgasatmosphäre (z.B. Argon, Helium, Stickstoff, etc.) und/oder in absoluten Lösungsmitteln durchgeführt werden. Gewünschtenfalls können die Endprodukte auf übliche Art und Weise gereinigt werden, z.B. durch Waschen, Digerieren, Extraktion, Umkristallisation, Chromatographie usw..

Die Reaktion von Verbindungen der Formel II mit Verbindungen der Formel III wird normalerweise in den obengenannten reaktionsinerten Lösungsmitteln im allgemeinen bei Temperaturen von -20° bis 100°C, vorzugsweise von 0° bis 50°C, durchgeführt. Zum Abfangen der dabei als Nebenprodukt gebildeten Säuren findet die Reaktion zweckmässigerweise in Gegenwart eines Neutralisationsmittels statt.

Als solche kommen organische Basen in Betracht, z.B. tertiäre Amine wie Trialkylamine (Trimethylamin, Triethylamin, Diisopropylmethylamin, Tripropylamin, usw.), Pyridin und Pyridinbasen (4-Dimethylaminopyridin, 4-Pyrrolidylaminopyridin usw.), bevorzugt ist Pyridin. Das Neutralisationsmittel wird üblicherweise in mindestens äquimolarer Menge in bezug auf die Ausgangsstoffe eingesetzt.

β) In den Fällen, in denen X in der Formel I für -NH-C(O)- steht, setzt man erfindungsgemäss die Verbindung der Formel II mit einem Isocyanat der Formel IV

$R_3$-N = C = O (IV)

oder mit einem Aminoacylierungsmittel der Formel V

$R_3$-NH-C(O)-D (V)

um, wobei $R_3$ jeweils die unter Formel I angegebenen Bedeutungen hat und D für eine unter T genannte Abgangsgruppe oder vorzugsweise für unsubstituiertes oder substituiertes Phenoxy steht. Auch diese Reaktion wird vorzugsweise in einem der unter α) genannten, inerten Lösungsmittel und in dem dort angegebenen Temperaturbereich durchgeführt.

γ) In den Fällen, in denen X in der Formel I für -NH-C(S)- steht, geht man wie unter β) vor, mit der Massgabe, dass statt des Isocyanats IV ein Isothiocyanat der Formel VI

$R_3$-N = C = S (VI),

worin $R_3$ die unter Formel I angegebenen Bedeutungen hat, eingesetzt wird.

δ) In den Fällen, in denen X in der Formel I für $SO_2$ steht, geht man wie unter α) vor, mit der Massgabe, dass statt des Acylierungsmittels der Formel III ein Sulfonierungsmittel der Formel VIa eingesetzt wird

$R_3$-$SO_2$-T (VIa),

worin $R_3$ die unter Formel I angegebenen Bedeutungen hat und T wie unter α) definiert ist.

ε) Ein weiteres erfindungsgemässes Verfahren zur Herstellung von Verbindungen der Formel I, in denen X in der Formel I für -NH-C(O)- steht, besteht darin, dass man eine Verbindung der Formel I, in welcher X für -OC(O)- steht und $R_3$ 4-Nitrophenyl ist, wie beispielsweise Verbindung Nr. 1.17 (Tab. 1), mit einem Amin der Formel VIb

$R_3$-$NH_2$ (VIb)

worin $R_3$ die unter Formel I angegebenen Bedeutungen hat, umsetzt.

Die Ausgangsverbindungen der Formel II sind aus Verbindungen der Formel VII

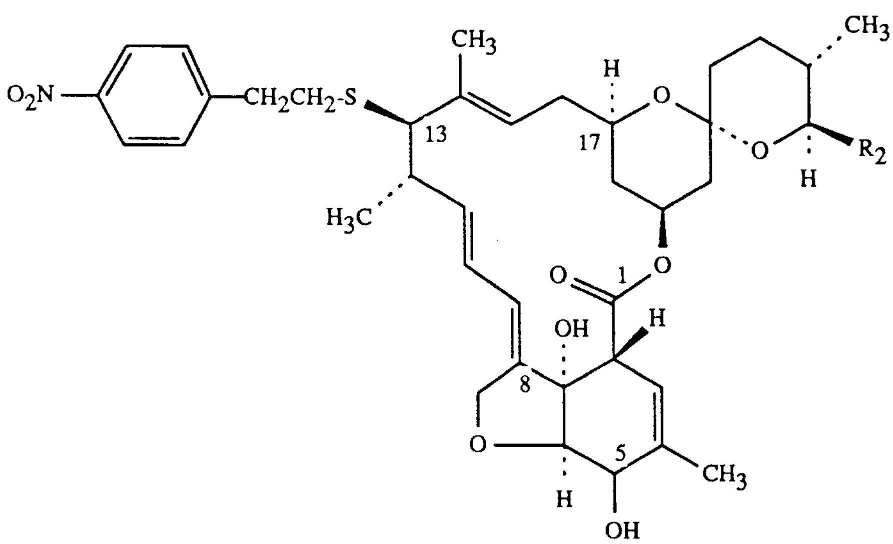

(VII)

durch Reduktion der endständigen Nitrogruppe zugänglich.

Hierzu kann jede beliebige zur Reduktion von $NO_2$ zu $NH_2$ geeignete Methode eingesetzt werden, so z.B. auch die katalytische Hydrierung mittels Palladiumkatalysatoren oder die Reduktion mit Zink in Gegenwart von Essigsäure bei Temperaturen zwischen 0° und 50°C. Es kann dabei vorteilhaft sein, die OH-Gruppe in 5-Position vor der Reduktion der Nitrogruppe mit einer Schutzgruppe zu maskieren.

Unter OH-Schutzgruppen sollen hier und im folgenden die in der organischen Chemie üblichen Schutzfunktionen verstanden werden. Dabei handelt es sich insbesondere um Acyl- und Silylgruppen.

Geeignete Acylgruppen sind beispielsweise die Reste $R_t$-C(O)-, wobei $R_t$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituierte Gruppe aus der Reihe Phenyl und Benzyl steht und vorzugsweise $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet. Als geeignete Silylgruppen zum Schutz von OH kommt der Rest -Si($R_x$)($R_y$)($R_z$)in Frage, wobei $R_x$, $R_y$ und $R_z$ vorzugsweise unabhängig voneinander für $C_1$-$C_4$-Alkyl, Benzyl oder Phenyl stehen und beispielsweise zusammen mit dem Siliziumatom eine der Gruppen Trimethylsilyl, Diphenyl-tert.-butylsilyl, bis(Isopropyl)-methylsilyl, Triphenylsilyl usw. und insbesondere tert.-Butyldimethylsilyl bilden. Die 5-OH-Gruppe kann auch als Benzylether oder Methoxiethoximethylether vorliegen.

Die Schutzgruppe lässt sich hydrolytisch wieder abspalten.

Die Verbindung der Formel VII ist aus der zugrundeliegenden Mercapto-Verbindung VIII

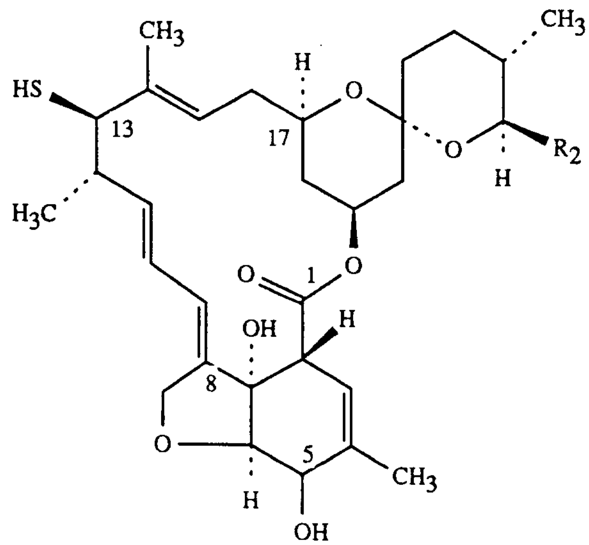

(VIII)

durch Reaktion mit einer Verbindung der Formel IX

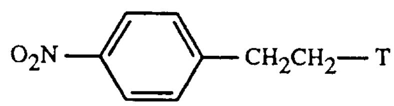

(IX)

zugänglich, wobei T für eine unter Formel III angegebene Abgangsgruppe, vorzugsweise für Jod steht. Man arbeitet bei 20° bis 150°C, vorzugsweise 80° bis 110°C in einem reaktionsinerten Lösungsmittel, vorzugsweise in Dimethylformamid, und setzt ein organisches Amin, bevorzugt Ethyldiisopropylamin, als Neutralisationsmittel zu.

Die Mercapto-Verbindung VIII ist aus der Europäischen Offenlegungsschrift EP-0,184,173 bzw. der korrespondierenden Britischen Patentschrift GB-2,167,751B bekannt.

Die Ausgangsverbindungen der Formeln III, IV, V, VI, VIa, VIb und IX sind bekannt oder lassen sich analog zu den bekannten Vertretern herstellen.

Die Verbindungen der Formel I eignen sich ausgezeichnet zur Bekämpfung aller Entwicklungsstadien von Schädlingen an Tieren und Pflanzen, darunter insbesondere von tierparasitären Ekto-Parasiten. Zu letzteren zählen unter der Ordnung Acarina insbesondere Schädlinge der Familien Ixodidae, Dermanyssidae, Sarcoptidae und Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae und Gastrophilidae.

Die Verbindungen der Formel I sind auch einsetzbar gegen Hygiene-Schädlinge, insbesondere solche der Ordnung Diptera mit den Familien Sarcophagidae, Anophilidae und Culicidae; der Ordnung Orthoptera, der Ordnung Dictyoptera (z.B. Familie Blattidae) und der Ordnung Hymenoptera (z.B. Familie Formicidae).

Die Verbindungen der Formel I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten); der Ordnungen Hemiptera, Heteroptera und Thysanoptera; sowie bei den pflanzenfressenden Insekten der Ordnungen Lepidoptera, Coleoptera, Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Gattungen Meloidogyne, Heterodera, Pratylenchus, Ditylenchus, Radopholus, Rizoglyphus und andere.

Ferner sind die Verbindungen gegen Helminthen in allen Entwicklungsstadien wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen und Ziervögeln. Typische Nematoden dieser Indikation sind: Haemonchus, Trichostrongylus, Ostertagia, Nematodirus, Cooperia, Ascaris, Bunostomum, Oesophagostomum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von human- pathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert Wenn die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich. Ueber geschlossenen Kultur-Anbauflächen werden sie zweckmässigerweise in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit guten Emulgier-, Dispergier- und Netzeigenschaften in Betracht Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergrüppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben: "1986 International Mc Cutcheon's Emulsifiers and Detergents" The Manufacturing Confectioner Publishing

Co., Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

A. Herstellung der Ausgangsprodukte

Beispiel A1: Herstellung von 5-O-tert.Butyldimethylsilyl-13$\beta$-(2-[4-nitrophenyl]ethylthio)milbemycin A4

Eine Lösung bestehend aus 2 g (2,9 mmol) 5-O-tert.Butyldimethylsilyl-13$\beta$-mercaptomilbemycin A4, 1,6 g (5,82 mmol) 2-(4-Nitrophenyl)-ethyljodid und 2,5 ml (1,9 g; 14,55 mmol) Diisopropylethylamin in 6 ml Dimethylformamid wird bei 100 °C während 60 Minuten gerührt. Das Reaktionsgut wird nach Abkühlung auf Raumtemperatur in 100 ml n-Hexan aufgenommen, zuerst mit 2M wässriger HCl-Lösung zweimal und anschliessend einmal mit 5 %iger wässriger $NaHCO_3$-Lösung gewaschen, über $MgSO_4$ getrocknet und eingeengt. Man erhält 1,8 g der Titelsubstanz nach Säulenchromatographie an Kieselgel.
$^1$H-NMR (300 MHz, $CDCl_3$, TMS)
3,04 (d, J = 10 Hz) $C_{13}H$

Beispiel A2: Herstellung von 13$\beta$-(2-[4-Nitrophenyl]ethylthio)milbemycin A4

1,7 g (20,3 mmol) 5-O-tert.Butyldimethylsilyl-13$\beta$-(2-[4-nitrophenyl]ethylthio)milbemycin A4 werden mit 20 ml einer 1 %igen methanolischen p-Toluolsulfonsäurelösung behandelt Nach 2 Stunden wird das Reaktionsprodukt in Ether aufgenommen, mit 5 %iger wässriger $NaHCO_3$ gewaschen, über $MgSO_4$ getrocknet und eingeengt. Nach Säulenchromatographie an Kieselgel (Essigester/n-Hexan/1:1) erhält man 1,53 g der Titelsubstanz.
$^1$H-NMR (300 MHz, $CDCl_3$)(TMS)
3,04 (d, J = 10 Hz)($C_{13}H$)
7,32 (d, J = 8 Hz)(aromatische H)
8,17 (d, J = 8 Hz)(aromatische H)
Massenspektrum (FD)m/e: 723 ($C_{40}H_{53}NO_9S$)

Beispiel A3: Herstellung von 13$\beta$-(2-[4-Aminophenyl]ethylthio)milbemycin A4

Zu einer Lösung von 4 g (5,53 mmol) 13$\beta$-(2-[4-Nitrophenyl]ethylthio)-milbemycin A4 in 40 ml Essigsäure werden bei Raumtemperatur unter Rühren 4 g Zinkstaub zugegeben und das resultierende Gemenge gerührt. Nach 16 Stunden wird das Reaktionsgemisch mit 150 ml Essigester verdünnt und filtriert. Das Filtrat wird dreimal mit Wasser gewaschen, mit $MgSO_4$ getrocknet, eingeengt und an Kieselgel (Essigester/Hexan/2:1) chromatographiert. Man erhält 2,25 g 13$\beta$-(2-[4-Aminophenyl]ethylthio)milbemycin A4.
$^1$H-NMR (300 MHz, $CDCl_3$, TMS)
3,03 (d J = 10 Hz)($C_{13}H$)
6,63 (d, J = 8 Hz)(aromatische H)
6,96 (d, J = 8 Hz)(aromatische H)
Massenspektrum (FD) m/e: 693 ($C_{40}H_{55}NO_7S$)

B. Herstellung der Endprodukte

Beispiel H1: Herstellung von 13β-(2-[4-Acetylaminophenyl]ethylthio)milbemycin A4

Zu einer Lösung von 40 mg (58 µmol) 13β-(2-[4-Aminophenyl]ethylthio)milbemycin A4, 5,8 µl Pyridin und 47 µl (37 mg, 1,15 mmol) Methanol in 0,3 ml Dichlormethan werden bei Raumtemperatur unter Rühren 7 µl (7 mg, 72 µmol) Essigsäureanhydrid zugegeben. Nach 2 Stunden Rühren bei Raumtemperatur wird mit Essigester und Wasser aufgearbeitet, mit 0,5M Zitronensäure und anschliessend 5 %iger wässriger $NaHCO_3$ gewaschen, und an Kieselgel (Essigester/Hexan/2:1) chromatographiert. Man erhält 35 mg 13β-(2-[4-Acetylaminophenyl]ethylthio)milbemycin A4.
$^1$H-NMR (300 MHz, $CDCl_3$, TMS)
2,17 ($CH_3CO$)
3,04 (d, J = 13 Hz)($C_{13}H$)
7,11 (d, J = 8 Hz)(aromatische H)
7,41 (d, J = 8 Hz)(aromatische H)

Beispiel H2: Herstellung von 13β-(2-[4-(4-Heptylcarbonylamino)phenyl]ethylthio)-milbemycin A4

Zu einer Lösung von 40 mg (58 µmol) 13β-(2-[4-Aminophenyl]ethylthio)milbemycin A4, 6 µl (73 µmol) Pyridin und 47 µl (37 mg, 1,15 mmol) Methanol in 0,3 ml Dichlormethan werden unter Rühren bei Raumtemperatur 12 mg (72 µmol) 4-Heptylcarbonylchlorid zugegeben. Nach 2 Stunden wird zwischen Ether und 1M Zitronensäure aufgearbeitet, mit 5 %iger wässriger $NaHCO_3$-Lösung gewaschen, mit $MgSO_4$ getrocknet und an Kieselgel (Essigester/Hexan/1:1) chromatographiert. Man erhält 32 mg 13β-(2-[4-(4-Heptylcarbonylamino)phenyl]ethylthio)milbemycin A4.

Beispiel H3: Herstellung von 13β-(2-[4-(Formylamino)phenyl]ethylthio)milbemycin A4

Zu einer Lösung von 40 mg (58 µmol) 13β-(2-[4-Aminophenyl]ethylthio)milbemycin A4, 6 µl Pyridin und 47 µl (1,15 mmol) Methanol in 0,3 ml Dichlormethan wird ein Gemisch von 7 µl Essigsäureanhydrid und 3 µl Ameisensäure bei Raumtemperatur zugegeben. Nach 4 Stunden wird das Reaktionsgemisch mit Essigester und 1M Zitronensäure aufgearbeitet, mit Wasser und 1M wässriger $NaHCO_3$ gewaschen und an Kieselgel (Essigester/Hexan/2:1) chromatographiert. Man erhält 30 mg 13β-(2-[4-(Formylamino)phenyl]-ethylthio)milbemycin A4.
$^1$H-NMR (300 MHz, $CDCl_3$, TMS)
8,37 (d, J = 1 Hz)(Formyl H)
8,64 (d, J = 12 Hz)(Formyl H)

Beispiel H4: Herstellung von 13β-(2-[4-(4-Toluolsulfonylamino)phenyl]ethylthio)-milbemycin A4

Zu einer Lösung von 40 mg (58 µmol) 13β-(2-[4-Aminophenyl]ethylthio)milbemycin A4, 6 µl (73 µmol) Pyridin und 47 µl (37 mg, 1,15 mmol) Methanol in 0,3 ml Dichlormethan werden unter Rühren bei Raumtemperatur 14 mg (72 µmol) Toluol-4-sulfochlorid zugegeben. Nach 2 Stunden wird mit Ether und 1M Zitronensäure aufgearbeitet, mit 5 %iger wässriger $NaHCO_3$-Lösung gewaschen, mit $MgSO_4$ getrocknet und an Kieselgel (Essigester/Hexan/2:1) chromatographiert. Man erhält 41 mg 13β-(2-[4-(4-Toluolsulfonylamino)phenyl]ethylthio)milbemycin A4.
$^1$H-NMR (300 MHz; $CDCl_3$, TMS)
6,97 (d, J = 8 Hz)(Aromatische H)
7,04 (d, J = 8 Hz)(Aromatische H)
7,23 (d, J = 8 Hz)(Aromatische H)
7,63 (d, J = 8 Hz)(Aromatische H)
Massenspektrum (CI-ve) m/e: 847 ($C_{47}H_{61}NO_9S_2$)

Beispiel H5: Herstellung von 13β-(2-[4-(Methylaminothiocarbonylamino)phenyl]ethylthio)milbemycin A4

Zu einer Lösung von 40 mg (58 µmol) 13β-(2-[4-Aminophenyl]ethylthio)milbemycin A4, 6 µl (73 µmol) Pyridin und 47 µl (1,15 mmol) Methanol in 0,3 ml Dichlormethan wird 5,3 mg (72 µmol) Methylisothiocyanat unter Rühren bei Raumtemperatur zugegeben. Nach 16 Stunden wird mit Ether und 1M wässriger Zitronensäure aufgearbeitet, mit 5 %iger wässriger $NaHCO_3$ gewaschen, mit $MgSO_4$ getrocknet und an

Kieselgel (Essigester/Hexan/2:1) chromatographiert. Man erhält 38 g 13β-(2-[4-(Methylaminothiocarbonylamino)phenyl]ethylthio)milbemycin A4.
$^{1}$H-NMR (300 MHz, $CDCl_3$, TMS)
3,14(d, J = 5 Hz, $NHCH_3$)

Beispiel H6: Herstellung von 13β-(2-[4-(Cyclohexylaminocarbonylamino)phenyl]ethylthio)milbemycin A4

Zu einer Lösung von 40 mg (58 µmol) 13β-(2-[4-Aminophenyl]ethylthio)milbemycin A4, 6 µl (73 µmol) Pyridin und 47 µl (1,15 mmol) Methanol in 0,3 ml Tetrahydrofuran werden 9 µl (8 mg, 72 µmol) Cyclohexylisocyanat bei Raumtemperatur unter Rühren zugegeben. Nach 3 Stunden bei Raumtemperatur wird mit Essigester und mit 5 %iger wässriger $NaHCO_3$ aufgearbeitet, mit $MgSO_4$ getrocknet und an Kieselgel (Essigester/Hexan/4:1) chromatographiert. Man erhält 46 mg 13β-(2-[4-(Cyclohexylaminocarbonylamino)phenyl]ethylthio)milbemycin A4.
$^{1}$H-NMR (300 MHz, $CDCl_3$, TMS)
4,50 (d, J = 8 Hz)(NH)
Massenspektrum (Cl - ve): m/e 818 ($C_{47}H_{65}N_2O_8S$)

Beispiel H7: Herstellung von 13β-(2-[4-(Carboxymethylaminocarbonylamino)phenyl]-ethylthio)milbemycin A4

Eine Lösung von 50 mg (58 µmol) 13β-(2-[4-(4-Nitrophenoxi)carbonylaminophenyl]ethylthio)milbemycin A4 (hergestellt nach Beispiel B2) und 4,4 mg (58 µmol) Glycin in 2ml Dimethylformamid werden bei 70 °C gerührt. Nach 2 Stunden wird mit Essigester und Wasser aufgearbeitet, mit $MgSO_4$ getrocknet und an Kieselgel (Dichlormethan/Methanol/4:1) chromatographiert. Man erhält 22 mg 13β-(2-[4-(Carboxymethylaminocarbonylamino)phenyl]ethylthio)milbemycin A4.
$^{1}$H-NMR (300 MHz, $D_6$-DMSO, TMS)
4,67 (d, J = 8 Hz)(NH)
9,09 (br, s)(COOH)
Massenspektrum (Cl + ve): m/e 794 ($C_{43}H_{58}N_2O_{10}S$)
Analog zu den beschriebenen Arbeitsweisen werden auch die nachfolgend aufgelisteten Verbindungen der Formel I hergestellt.

Tabelle 1: Typische Vertreter von Verbindungen der Formel I

mit $R_1$ =

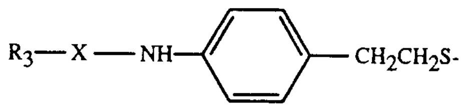

| Verb.Nr. | X | $R_3$ | $R_2$ | Physik. Daten |
|---|---|---|---|---|
| 1.1 | CO | $C_2H_5$ | $C_2H_5$ | m/e = 749 (CI-ve) |
| 1.2 | CO | Phenyl | $C_2H_5$ | m/e = 797 (FD) |
| 1.3 | CO | $CH_3$ | $C_2H_5$ | m/e = 735 (FD) |
| 1.4 | CO | H | $C_2H_5$ | m/e = 721 (CI-ve) |
| 1.5 | CO | tert.Butyl | $C_2H_5$ | m/e = 777 (CI-ve) |
| 1.6 | CO | $(H_3CCH_2CH_2)_2CH$- | $C_2H_5$ | m/e = 819 (CI-ve) |
| 1.7 | CO | $ClCH_2$ | $C_2H_5$ | m/e = 769 (CI-ve) |
| 1.8 | CO | 2,6-Dichlorpyrid-4-yl | $C_2H_5$ | m/e = 866 (CI-ve) |
| 1.9 | CO | $Cl_2CH$ | $C_2H_5$ | m/e = 804 (CI-ve) |
| 1.10 | CO | $Cl_3C$ | $C_2H_5$ | m/e = 839 (CI-ve) |
| 1.11 | CO | $F_3C$ | $C_2H_5$ | m/e = 789 (CI+ve) |
| 1.12 | CO | 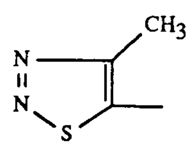 | $C_2H_5$ | m/e = 837 (CI+ve $NH_3$) |
| 1.13 | OCO | $CH_3$ | $C_2H_5$ | m/e = 751 (FD) |
| 1.14 | OCO | $C_2H_5$ | $C_2H_5$ | m/e = 765 (FD) |
| 1.15 | OCO | Benzyl | $C_2H_5$ | m/e = 827 (FD) |
| 1.16 | OCO | Phenyl | $C_2H_5$ | m/e = 831 (CI+ve $NH_3$) |
| 1.17 | OCO | p-Nitrophenyl | $C_2H_5$ | m/e = 858 (FD) |
| 1.18 | OCO | $CH_3CH_2CH_2CH_2$ | $C_2H_5$ | m/e = 793 (CI-ve) |
| 1.19 | $SO_2$ | $CH_3$ | $C_2H_5$ | m/e = 771 (CI-ve) |
| 1.20 | $SO_2$ | p-Tolyl | $C_2H_5$ | m/e = 847 (CI-ve) |

Fortsetzung von Tabelle 1:

| Verb.Nr. | X | $R_3$ | $R_2$ | Physik. Daten |
|---|---|---|---|---|
| 1.21 | $SO_2$ | 2-Fluorphenyl | $C_2H_5$ | m/e = 869 (CI+ve $NH_3$) |
| 1.22 | $SO_2$ | 2-(2-Methoxyethoxy-)phenyl | $C_2H_5$ | m/e = 925 (CI+ve $NH_3$) |
| 1.23 | $SO_2$ | 2-Difluormethylthiophenyl | $C_2H_5$ | m/e = 933 (CI+ve $NH_3$) |
| 1.24 | NH-CS | $C_2H_5$ | $C_2H_5$ | m/e = 778 (CI-ve) |
| 1.25 | NH-CS | 3-Fluorphenyl | $C_2H_5$ | m/e = 846 (CI-ve) |
| 1.26 | NH-CS | $CH_3$ | $C_2H_5$ | m/e = 776 (CI-ve) |
| 1.27 | NH-CO | $CH_3$ | $C_2H_5$ | m/e = 750 (FD) |
| 1.28 | NH-CO | $C_2H_5$ | $C_2H_5$ | m/e = 764 (CI+ve) |
| 1.29 | NH-CO | Cyclohexyl | $C_2H_5$ | m/e = 818 (CI-ve) |
| 1.30 | NH-CO | p-Tolyl | $C_2H_5$ | m/e = 826 (CI-ve) |
| 1.31 | NH-CO | 2-Fluorphenyl | $C_2H_5$ | m/e = 830 (CI-ve) |
| 1.32 | NH-CO | Phenyl | $C_2H_5$ | m/e = 812 (CI-ve) |
| 1.33 | NH-CO | p-Methoxyphenyl | $C_2H_5$ | m/e = 842 (CI-ve) |
| 1.34 | NH-CO | $HOOCCH(CH_3)$- | $C_2H_5$ | m/e = 808 (CI-ve) |
| 1.35 | NH-CO | $HOOCCH_2$- | $C_2H_5$ | m/e = 794 (CI+ve) |
| 1.36 | NH-CO | HOOCCH(isopropyl)- | $C_2H_5$ | m/e = 836 (CI-ve) |
| 1.37 | CO | $CH_3$ | $CH_3$ | m/e = 721 (CI-ve) |
| 1.38 | CO | $CH_3$ | Isopropyl | m/e = 749 (CI-ve) |
| 1.39 | OCO | $CH_3$ | $CH_3$ | m/e = 737 (CI-ve) |
| 1.40 | OCO | $C_2H_5$ | sek.Butyl | m/e = 779 (CI-ve) |
| 1.41 | $SO_2$ | $CH_3$ | $CH_3$ | m/e = 757 (CI-ve) |
| 1.42 | NH-CS | $CH_3$ | Isopropyl | m/e = 780 (CI-ve) |
| 1.43 | NH-CO | $CH_3$ | $CH_3$ | m/e = 736 (CI-ve) |

Fortsetzung von Tabelle 1:

| Verb.Nr. | X | $R_3$ | $R_2$ | Physik. Daten |
|---|---|---|---|---|
| 1.44 | NH-CO | 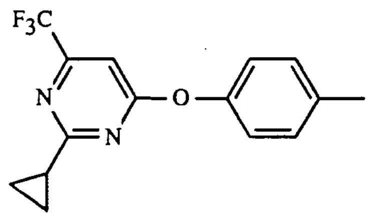 | $C_2H_5$ | m/e = 1014 (CI-ve) |
| 1.45 | NH-CO | 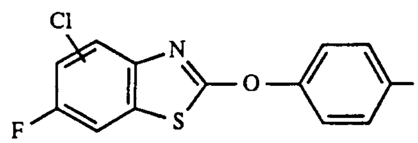 | $C_2H_5$ | m/e = 1019 (CI-ve) |
| 1.46 | NH-CO | 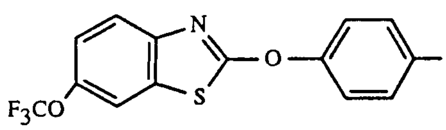 | $C_2H_5$ | m/e = 1045 (CI-ve) |
| 1.47 | NH-CO | 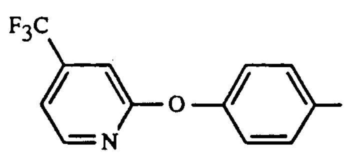 | $C_2H_5$ | m/e = 973 (CI-ve) |
| 1.48 | NH-CO | 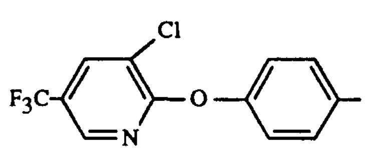 | $C_2H_5$ | m/e = 1007 (CI-ve) |
| 1.49 | NH-CO | 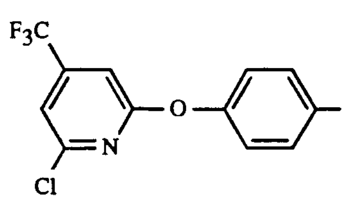 | $C_2H_5$ | m/e = 1007 (CI-ve) |

Formulierungsbeispiele für den Wirkstoff der Formel I
(% = Gewichtsprozent)

| Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus Tabelle 1 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermählen wird.

| Extruder Granulat | |
|---|---|
| Wirkstoff aus Tabelle 1 | 10 % |
| Na-Ligninsulfonat | 2 % |
| Carboximethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Tabletten bzw. Boli | | |
|---|---|---|
| I | Ein Wirkstoff aus Tabelle 1<br>Methylcellulose<br>Kieselsäure hochdispers | 33,0 %<br>0,80 %<br>0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist.<br>Maisstärke<br>mikrokrist. Cellulose<br>Magnesiumstearat | 22,50 %<br>17,00 %<br>16,50 %<br>1,00 % |

Alle 4 Hilfsstoffe gut mischen
Phasen I und II mischen und zu Tabletten oder Boli verpressen.

Injektabiles

A. Oeliges Vehikel (langsame Freisetzung)

| | |
|---|---|
| Ein Wirkstoff aus Tabelle 1 | 0,1-1,0 g |
| Erdnussöl | ad 100 ml |
| Ein Wirkstoff aus Tabelle 1 | 0,1-1,0 g |
| Sesamöl | ad 100 ml |

Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 μm sterilfiltriert.

B. Wassermischbare Lösung (mittlere Freisetzungsgeschwindigkeit)

| | |
|---|---|
| Ein Wirkstoff aus Tabelle 1 | 0,1-1,0 g |
| 4-Hydroxymethyl-1,3-dioxolan (Glycerol Formal) | 40 g |
| 1,2-Propandiol | ad 100 ml |
| Ein Wirkstoff aus Tabelle 1 | 0,1-1,0 g |
| Glycerindimethylketal | 40 g |
| 1,2-Propandiol | ad 100 ml |

Herstellung: Der Wirkstoff wird in einem Teil des Lösungsmittels unter Rühren gelöst, auf das Sollvolumen aufgefüllt und durch ein geeignetes Membranfilter mit 0.22 μm sterilfiltriert.

C. Wässriges Solubilisat (rasche Freisetzung)

| | |
|---|---|
| Ein Wirkstoff aus Tabelle 1 | 0,1-1,0 g |
| Polyäthoxyliertes Ricinusöl (40 Aethylenoxideinheiten)* | 10 g |
| 1,2-Propandiol | 20 g |
| Benzylalkohol | 1 g |
| Aqua ad injekt. | ad 100 ml |

* Im Handel erhältlich unter der Bezeichnung CREMOPHOR® EL (BASF AG);

| | |
|---|---|
| Ein Wirkstoff aus Tabelle 1 | 0,1-1,0 g |
| Polyäthoxyliertes Sorbitanmonooleat (20 Aethylenoxideinheiten)** | 8 g |
| 4-Hydroxymethyl-1,3-dioxolan (Glycerol Formal) | 20 g |
| Benzylalkohol | 1 g |
| Aqua ad injekt. | ad 100 ml |

** Im Handel erhältlich unter der Bezeichnung TWEEN® 80 (ICI);

Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 µm Porendurchmesser.

Die wässrigen Systeme können bevorzugterweise auch für die orale und/oder intraruminale Applikation eingesetzt werden.

Biologische Beispiele

B-1. Wirkung gegen $L_1$-Larven von Lucilia sericata

1 ml einer wässrigen Suspension der zu prüfenden Aktivsubstanz wird somit 3 ml eines speziellen Larvenzuchtmediums bei ca. 50 °C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I, beispielsweise von den Beispielen H1 bis H7, bewirken eine vollständige Abtötung bei 250 ppm.

B-2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine Wirkstoffmenge von 1,0 µg pro Zecke gelöst ist Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formel I, beispielsweise aus Tabelle 1, zeigen eine $IR_{90}$ bei einer Aufwandmenge von 5 µg/g.

B-3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wählweise mit 1 mg oder 0,5 mg/kg Körpergewicht. Die Evaluierung

erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit Verbindungen der Formel I aus Tabelle 1 bei 1 mg/kg behandelt werden, zeigen im Vergleich zu unbehandelten, aber infizierten Vergleichsgruppen keinen Nematodenbefall (= komplette Reduktion der Wurmeier im Kot).

B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formel I aus Tabelle 1 bewirken in diesem Test bei niedriger Konzentration bereits nach einem Tag eine vollständige Abtötung sämtlicher Larven.

B-5. Milbizide Wirkung gegen Dermanyssus gallinae

2 bis 3 ml einer Testlösung mit 100 ppm Aktivsubstanz werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen der Formel I aus Tabelle 1 bewirken bei der angegebenen Konzentration eine Abtötung der Milben.

**Patentansprüche**

**Patentansprüche für folgende Vertragsstaaten : AT, BE, CH, DE, DK, FR, GB, IT, LI, LU, NL, SE**

**1.** Verbindung der Formel I

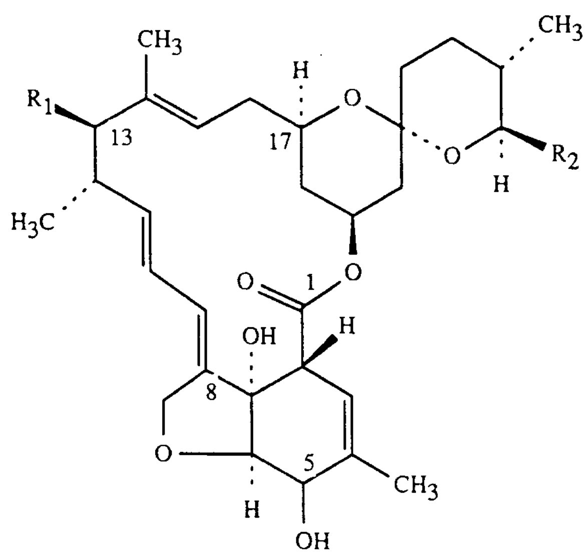

(I),

worin
$R_2$ für Methyl, Ethyl, Isopropyl, sek.Butyl oder für die Gruppe $-C(CH_3)=CHQ$ steht,
wobei
Q Methyl, Ethyl oder Isopropyl bedeutet;
und
$R_1$ die Gruppe

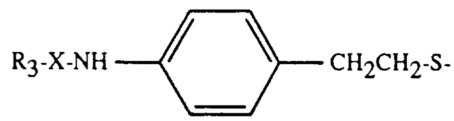

repräsentiert;
wobei X für eine der Gruppen -C(O)-, -OC(O)-, -NHC(O)-, -NHC(S)- oder $-SO_2-$ steht;
und
$R_3$ für Wasserstoff, $Het_1$, unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl;
unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl oder für die Gruppe

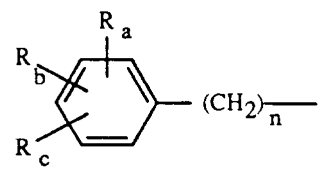

steht, wobei
n für 0, 1 oder 2 steht,
$R_a$, $R_b$ und $R_c$ unabhängig voneinander Wasserstoff, $C_1$-$C_4$-Alkyl, $C_1$-$C_4$-Alkoxy, $C_1$-$C_4$-Alkylthio, $C_1$-$C_4$-Haloalkyl, $C_1$-$C_4$-Haloalkoxy, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, Halogen, CN, $NO_2$ oder $NH_2$ bedeuten, wobei $R_c$ zusätzlich für $C_3$-$C_8$-Cycloalkyl oder für die Gruppe

$Het_2$-O-

stehen kann, wobei $Het_1$ und $Het_2$ für einen unsubstituierten oder substituierten, über Kohlenstoff gebundenen Heterocyclus stehen, ausgewählt aus der Reihe Benzimidazol, Benzoxazol, Benzthiazol, Imidazol, Oxazol, Thiazol, Oxadiazol, Thiadiazol, Triazol, Pyridin, Pyridazin, Pyrimidin, Pyrazin, Triazin, Chinolin, Isochinolin, Chinoxalin, Phthalazin, Chinazolin und Benzotriazin; unter Einschluss ihrer physiologisch verträglichen Säureadditionssalze.

2. Verbindung der Formel I nach Anspruch 1, worin
$R_2$ für Methyl, Ethyl, Isopropyl, sek.Butyl oder für die Gruppe $-C(CH_3)=CHQ$ steht,
wobei
Q Methyl, Ethyl oder Isopropyl bedeutet;
und
$R_1$ die Gruppe

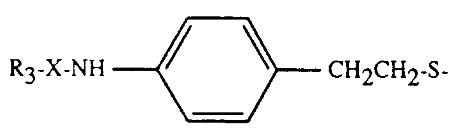

repräsentiert;
wobei X für eine der Gruppen -C(O)-, -OC(O)-, -NHC(O)-, -NHC(S)- oder $-SO_2-$ steht;
und
$R_3$ für Wasserstoff, $Het_1$, unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl;
unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl oder für die Gruppe

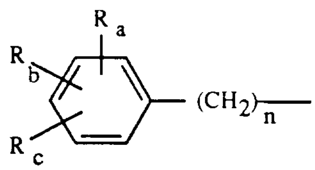

steht, wobei

n für 0, 1 oder 2 steht,

$R_a$, $R_b$ und $R_c$ unabhängig voneinander Wasserstoff, $C_1$-$C_4$-Alkyl, $C_1$-$C_4$-Alkoxy, $C_1$-$C_4$-Alkylthio, $C_1$-$C_4$-Haloalkyl, $C_1$-$C_4$-Haloalkoxy, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, Halogen, CN, $NO_2$ oder $NH_2$ bedeuten, wobei $R_c$ zusätzlich für $C_3$-$C_8$-Cycloalkyl oder für die Gruppe

$Het_2$-O-

stehen kann, wobei $Het_1$ und $Het_2$, welche nachfolgend unter Het zusammengefasst sind, für eine Gruppe ausgewählt aus der Reihe a bis u stehen:

| Gruppe | Het | Typ |
| --- | --- | --- |
| a) | 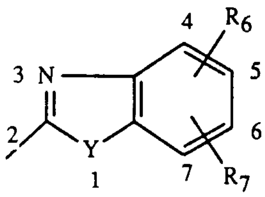 | Benzimidazol-2-yl<br>Benzoxazol-2-yl<br>Benzthiazol-2-yl |
| b) | 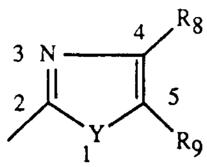 | Imidazol-2-yl<br>Oxazol-2-yl<br>Thiazol-2-yl |
| c) | 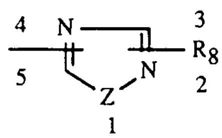 | [1H-1,2,4-Triazol]-5-yl<br>[1,2,4-Oxadiazol]-5-yl<br>[1,2,4-Thiadiazol]-5-yl<br>[1,2,4-Oxadiazol]-3-yl<br>[1,2,4-Thiadiazol]-3-yl |
| d) | 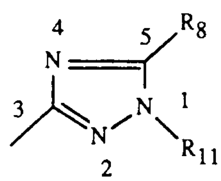 | [1H-1,2,4-Triazol]-3-yl |
| e) | 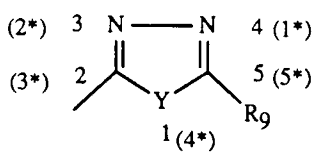 | [1,3,4-Oxadiazol]-2-yl<br>[1,3,4-Thiadiazol]-2-yl<br>* [4H-1,2,4-Triazol]-3-yl |
| f) | 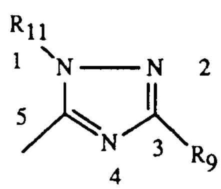 | [1H-1,2,4-Triazol]-5-yl |
| g) | 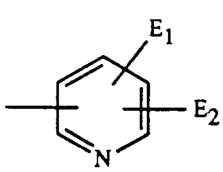 | Pyridin-2-yl<br>Pyridin-3-yl<br>Pyridin-4-yl |

| Gruppe | Het | Typ |
|---|---|---|
| h) | 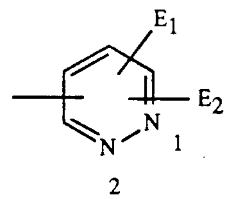 | Pyridazin-3-yl<br>Pyridazin-4-yl |
| i) | 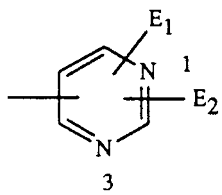 | Pyrimidin-2-yl<br>Pyrimidin-4-yl<br>Pyrimidin-5-yl |
| k) | 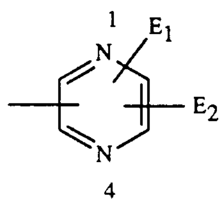 | Pyrazin-2-yl |
| l) | 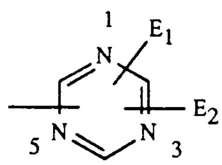 | s-Triazin-2-yl |
| m) | 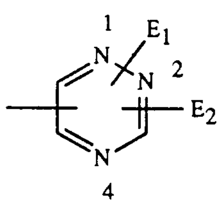 | [1,2,4-Triazin]-3-yl<br>[1,2,4-Triazin]-5-yl<br>[1,2,4-Triazin]-6-yl |
| n) | 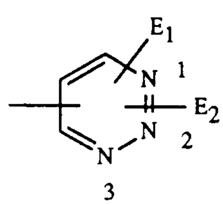 | [1,2,3-Triazin]-4-yl<br>[1,2,3-Triazin]-5-yl |
| o) | 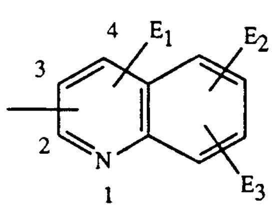 | Chinolin-2-yl<br>Chinolin-3-yl<br>Chinolin-4-yl |

| Gruppe | Het | Typ |
|---|---|---|
| p) | 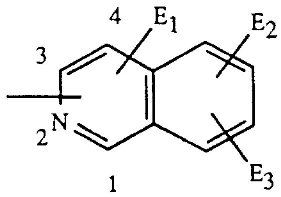 | Isochinolin-1-yl<br>Isochinolin-3-yl<br>Isochinolin-4-yl |
| q) | 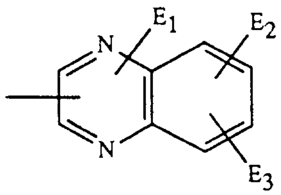 | Chinoxalin-2-yl |
| r) | 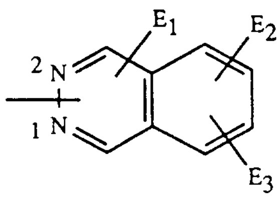 | Phthalazin-3-yl |
| s) | 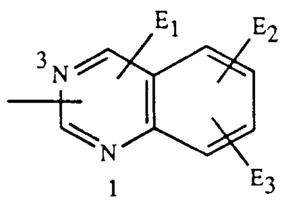 | Chinazolin-2-yl<br>Chinazolin-4-yl |
| t) | 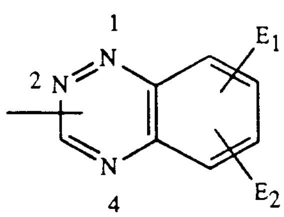 | [1,2,4-Benzotriazin]-3-yl |
| u) | 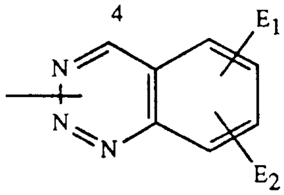 | [1,2,3-Benzotriazin]-4-yl |

wobei

Y für Sauerstoff, Schwefel oder $NR_{10}$ steht;

Z für Sauerstoff, Schwefel oder NH steht;

$R_6$ und $R_7$ unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, Halogen, Nitro oder Cyano bedeuten;

$R_8$ und $R_9$ unabhängig voneinander für Wasserstoff, $C_1$-$C_6$-Alkyl, $C_1$-$C_6$-Haloalkyl, $C_1$-$C_6$-Alkoxy, $C_1$-

$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen;
$R_{10}$ Wasserstoff oder $C_1$-$C_6$-Alkyl bedeutet;
$R_{11}$ für $C_1$-$C_6$-Alkyl steht;
und
$E_1$, $E_2$ und $E_3$ unabhängig voneinander für Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, $C_3$-$C_8$-Cycloalkyl, Halogen, CN, $NO_2$ oder $NH_2$ stehen.

**3.** Verbindung der Formel I nach Anspruch 2, dadurch gekennzeichnet, dass $E_1$, $E_2$ und $E_3$ unabhängig voneinander für Wasserstoff, Methyl, Ethyl, $CF_3$, Methoxy, $OCF_3$, Methylthio, $CH_3OCH_2O$, Cyclopropyl, Fluor, Chlor oder Brom stehen und die übrigen Substituenten wie in Anspruch 2 definiert sind.

**4.** Verbindung der Formel I nach Anspruch 2, dadurch gekennzeichnet, dass in den Gruppen b bis f $R_8$ und $R_9$ unabhängig voneinander für unsubstituiertes oder durch Halogen oder $C_1$-$C_3$-Alkyl substituiertes $C_3$-$C_7$-Cycloalkyl stehen und die übrigen Substituenten sowie Y und Z wie in Anspruch 2 definiert sind.

**5.** Verbindung der Formel I nach Anspruch 2, dadurch gekennzeichnet, dass der Substituent Het für

a')

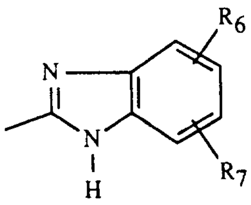 , 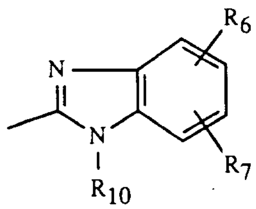 , 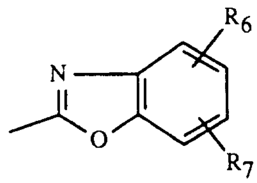 , 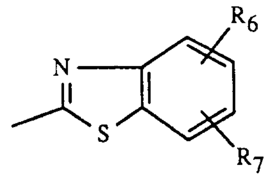 ,

b') 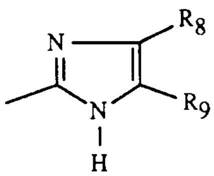 , 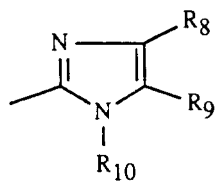 , 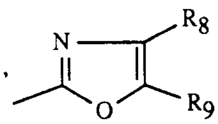 , 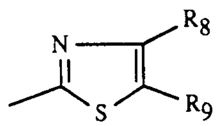 ,

c') 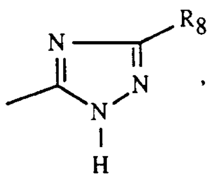 , 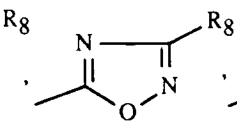 , 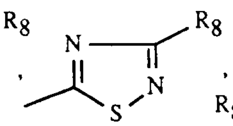 , 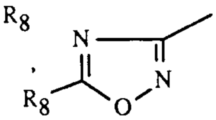 , 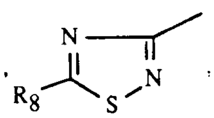 ;

d') 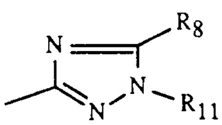

e') 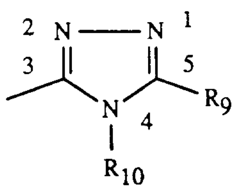 , 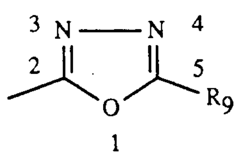 , 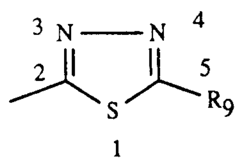 ,

f') 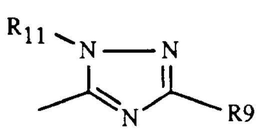 ,

steht und die übrigen Substituenten wie in Anspruch 2 definiert sind.

**6.** Verbindung der Formel I nach einem der Ansprüche 2 bis 5, dadurch gekennzeichnet, dass $R_2$ für Methyl oder Ethyl steht.

**7.** Verbindung der Formel I nach Anspruch 1, dadurch gekennzeichnet, dass $R_2$ und $R_3$ unabhängig voneinander für Methyl, Ethyl oder Isopropyl stehen und X die unter Formel I angegebenen Bedeutungen hat.

**8.** Verbindung der Formel I nach Anspruch 1, dadurch gekennzeichnet, dass $R_2$ für Methyl, Ethyl, Isopropyl oder sek.Butyl steht;
$R_1$ die Gruppe

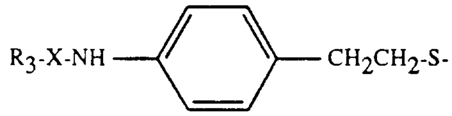

repräsentiert;
wobei X für eine der Gruppen -C(O)-, -OC(O)-, -NHC(O)-, -NHC(S)- oder $-SO_2-$ steht;
und
$R_3$ für $C_1$-$C_8$-Alkyl oder $C_3$-$C_6$-Cycloalkyl steht.

**9.** Verbindung der Formel I nach Anspruch 1, dadurch gekennzeichnet, dass $R_2$ für Methyl, Ethyl, Isopropyl oder sek.Butyl steht;
$R_1$ die Gruppe

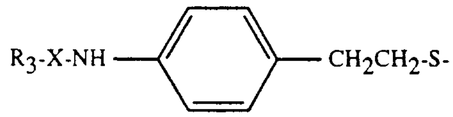

repräsentiert;
wobei X für eine der Gruppen -C(O)-, -OC(O)-, -NHC(O)-, -NHC(S)- oder $-SO_2-$ steht;
und
$R_3$ für die Gruppe

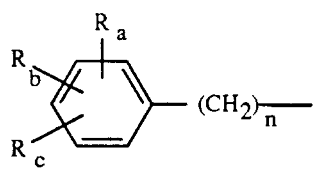

steht, wobei
n für 0 oder 1 steht und
$R_a$, $R_b$ und $R_c$ unabhängig voneinander für Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten und $R_c$ zusätzlich für die Gruppe $Het_2$-O- stehen kann, wobei $Het_2$ die unter Formel I angegebenen Gruppen repräsentiert.

**10.** Verbindung der Formel I nach Anspruch 2, dadurch gekennzeichnet, dass $R_2$ für Methyl, Ethyl, Isopropyl oder sek.Butyl steht;
$R_1$ die Gruppe

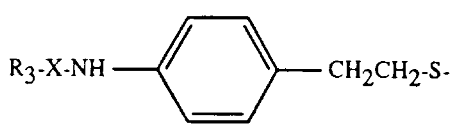

repräsentiert;
wobei X für eine der Gruppen -C(O)-, -OC(O)- oder -NHC(O)- steht; und
$R_3$ für $Het_1$, unsubstituiertes oder durch $C_1$-$C_4$-Alkoxy, $C_1$-$C_4$-Alkylthio oder Halogen substituiertes $C_1$-$C_6$-Alkyl, unsubstituiertes oder durch $C_1$-$C_2$-Alkoxy, $C_1$-$C_2$-Alkylthio oder Halogen substituiertes $C_3$-$C_6$-Cycloalkyl oder die Gruppe

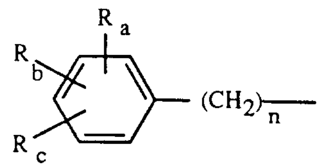

steht, wobei n für 0 oder 1 steht;
$R_a$, $R_b$ und $R_c$ unabhängig voneinander Wasserstoff, Fluor, Chlor, Brom, Methyl, Methoxy, Methylthio, $CF_3$ oder $NO_2$ bedeuten, wobei $R_c$ zusätzlich für die Gruppe

$Het_2$-O-

stehen kann, wobei $Het_1$ und $Het_2$ für eine der in Anspruch 5 für Het definierten Gruppen a') bis f') steht und die übrigen Substituenten wie in Anspruch 2 definiert sind.

**11.** Verbindung der Formel I nach Anspruch 1, dadurch gekennzeichnet, dass $R_2$ für Methyl, Ethyl, Isopropyl oder sek.Butyl steht;
$R_1$ die Gruppe

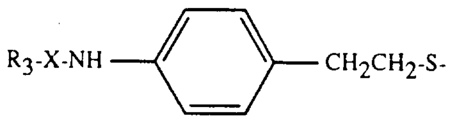

repräsentiert,
wobei X für -OC(O)- steht; und
$R_3$ für unsubstituiertes oder durch $C_1$-$C_4$-Alkoxy, $C_1$-$C_4$-Alkylthio oder Halogen substituiertes $C_1$-$C_6$-Alkyl steht.

**12.** Verbindung der Formel I nach Anspruch 1, dadurch gekennzeichnet, dass $R_2$ für Methyl, Ethyl, Isopropyl oder sek.Butyl steht;
$R_1$ die Gruppe

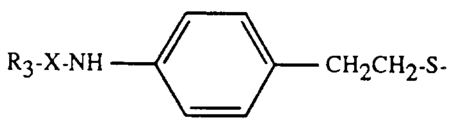

repräsentiert,
wobei X für -C(O)-, -OC(O)- oder -NHC(O)- steht; und
$R_3$ für Wasserstoff; unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl; unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl steht.

**13.** Verbindung der Formel I nach Anspruch 2, dadurch gekennzeichnet, dass $R_2$ für Methyl, Ethyl, Isopropyl oder sek.Butyl steht;
$R_1$ die Gruppe

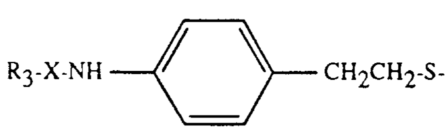

repräsentiert,
wobei X für -C(O)-, -OC(O)- oder -NHC(O)- steht; und $R_3$ für die Gruppe

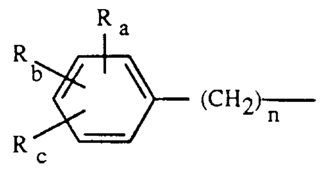

steht, wobei n für 0 oder 1 steht und
$R_a$, $R_b$ und $R_c$ unabhängig voneinander Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten und
$R_c$ zusätzlich für die Gruppe

$Het_2$-O-

stehen kann, wobei $Het_2$ für eine der im Anspruch 5 für Het definierten Gruppen a') bis f') steht und die übrigen Substituenten wie in Anspruch 2 definiert sind.

**14.** Verbindung der Formel I nach Anspruch 1, dadurch gekennzeichnet, dass $R_2$ für Methyl, Ethyl, Isopropyl oder sek.Butyl steht;
$R_1$ die Gruppe

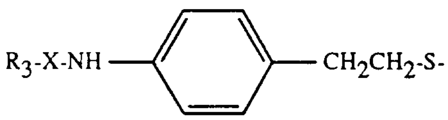

repräsentiert,
wobei X für -C(O)-, -OC(O)- oder -NHC(O)- steht; und $R_3$ für die Gruppe

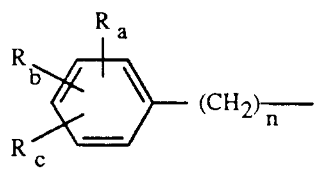

steht, wobei n für 0 oder 1 steht und
$R_a$, $R_b$ und $R_c$ unabhängig voneinander für Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten.

**15.** Verbindung der Formel I nach Anspruch 1, dadurch gekennzeichnet, dass $R_2$ für Methyl oder Ethyl steht;
$R_1$ die Gruppe

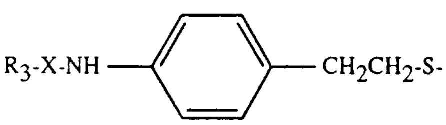

repräsentiert,
wobei X für -OC(O)- steht und $R_3$ $C_1$-$C_6$-Alkyl bedeutet.

**16.** Eine Verbindung der Formel I nach Anspruch 1, ausgewählt aus der Reihe
13β-[2-(4-Acetaminophenyl)ethylthio]milbemycin A4;
13β-(2-[4-(4-Heptylcarbonylamino)phenyl]ethylthio)milbemycin A4;
13β-(2-[4-(2,6-Dichlorpyridin-4-ylcarbonylamino)phenyl]ethylthio)-milbemycin A4;
13β-(2-[4-Tosylphenyl]ethylthio)milbemycin A4;
13β-(2-[4-(Methylaminothiocarbonylamino)phenyl]ethylthio)milbemycin A4;
13β-(2-[4-(Benzoylaminocarbonylamino)phenyl]ethylthio)milbemycin A4;
13β-(2-[4-(Cyclohexylaminocarbonylamino)phenyl]ethylthio)milbemycin A4;
13β-(2-[4-((4-Methoxyphenyl)aminocarbonylamino)phenyl]ethylthio)milbemycin A4;
13β-(2-[4-(Carboxymethylaminocarbonylamino)phenyl]ethylthio)milbemycin A4; und
13β-(2-[4-(Methoxycarbonylamino)phenyl]ethylthio)milbemycin A4.

**17.** Verfahren zur Herstellung einer Verbindung der Formel I nach Anspruch 1, dadurch gekennzeichnet, dass man eine Verbindung der Formel II

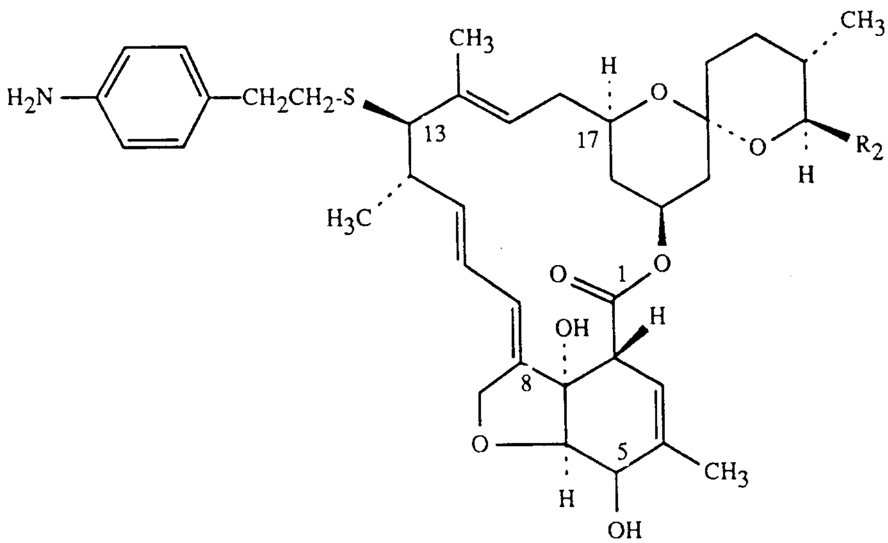

(II),

worin $R_2$ die unter Formel I angegebenen Bedeutungen hat,

α) in den Fällen, in denen X in der Formel I für -C(O)- oder -OC(O)- steht, mit einer Verbindung der Formel III

$R_3$-X-T (III)

in Abwesenheit oder vorzugsweise in Anwesenheit eines Neutralisationsmittels umsetzt, wobei $R_3$ die unter Formel I angegebenen Bedeutungen hat, X für -C(O)- oder -OC(O)-steht und T eine Abgangsgruppe repräsentiert;

β) in den Fällen, in denen X in der Formel I für -NH-C(O)- steht, mit einem Isocyanat der Formel IV

$R_3$-N=C=O (IV)

oder mit einem Aminoacylierungsmittel der Formel V

$R_3$-NH-C(O)-D (V)

umsetzt, wobei $R_3$ jeweils die unter Formel I angegebenen Bedeutungen hat und D für eine unter T genannte Abgangsgruppe oder vorzugsweise für unsubstituiertes oder substituiertes Phenoxy steht;

γ) in den Fällen, in denen X in der Formel I für -NH-C(S)- steht, mit einem Isothiocyanat der Formel VI

$R_3$-N=C=S (VI),

umsetzt, wobei $R_3$ die unter Formel I angegebenen Bedeutungen hat und
δ) in den Fällen, in denen X in der Formel I für $SO_2$ steht, mit einem Sulfonierungsmittel der Formel VIa

$R_3$-$SO_2$-T (VIa),

in Abwesenheit oder vorzugsweise in Anwesenheit eines Neutralisationsmittels umsetzt, wobei $R_3$ die unter Formel I angegebenen Bedeutungen hat und T wie unter α) definiert ist.

**18.** Insektizides und parasitizides Mittel, dadurch gekennzeichnet, dass es als Wirkstoff eine Verbindung der Formel I nach einem der Ansprüche 1 bis 16 enthält.

**19.** Verbindungen der Formel I nach einem der Ansprüche 1 bis 16 zur Anwendung bei der Bekämpfung von auf oder in Tieren parasitierenden Schädlingen.

**20.** Verbindungen der Formel I nach Anspruch 19 zur Anwendung bei der Bekämpfung von Endo- oder Ektoparasiten am Nutztier.

**21.** Verwendung einer Verbindung der Formel I nach einem der Ansprüche 1 bis 16 zur Herstellung eines Arzneimittels zur Bekämpfung von auf oder in Tieren parasitierenden Schädlingen.

**Patentansprüche für folgende Vertragsstaaten : ES, GR**

**1.** Verfahren zur Herstellung einer Verbindung der Formel I

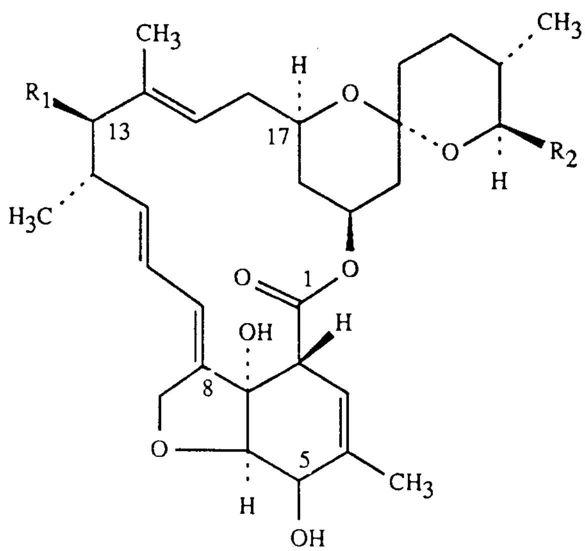

(I),

worin
$R_2$ für Methyl, Ethyl, Isopropyl, sek.Butyl oder für die Gruppe -C($CH_3$) = CHQ steht, wobei
Q Methyl, Ethyl oder Isopropyl bedeutet;
und
$R_1$ die Gruppe

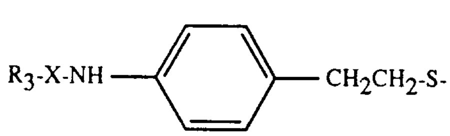

repräsentiert;

wobei X für eine der Gruppen -C(O)-, -OC(O)-, -NHC(O)-, -NHC(S)- oder $-SO_2-$ steht;
und
$R_3$ für Wasserstoff, $Het_1$, unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl;
unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl oder für die Gruppe

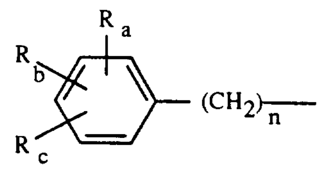

steht, wobei
n für 0, 1 oder 2 steht,
$R_a$, $R_b$ und $R_c$ unabhängig voneinander Wasserstoff, $C_1$-$C_4$-Alkyl, $C_1$-$C_4$-Alkoxy, $C_1$-$C_4$-Alkylthio, $C_1$-$C_4$-Haloalkyl, $C_1$-$C_4$-Haloalkoxy, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, Halogen, CN, $NO_2$ oder $NH_2$ bedeuten, wobei $R_c$ zusätzlich für $C_3$-$C_8$-Cycloalkyl oder für die Gruppe

$Het_2$-O-

stehen kann, wobei $Het_1$ und $Het_2$ für einen unsubstituierten oder substituierten, über Kohlenstoff gebundenen Heterocyclus stehen, ausgewählt aus der Reihe Benzimidazol, Benzoxazol, Benzthiazol, Imidazol, Oxazol, Thiazol, Oxadiazol, Thiadiazol, Triazol, Pyridin, Pyridazin, Pyrimidin, Pyrazin, Triazin, Chinolin, Isochinolin, Chinoxalin, Phthalazin, Chinazolin und Benzotriazin; unter Einschluss ihrer physiologisch verträglichen Säureadditionssalze, dadurch gekennzeichnet, dass man eine Verbindung der Formel II

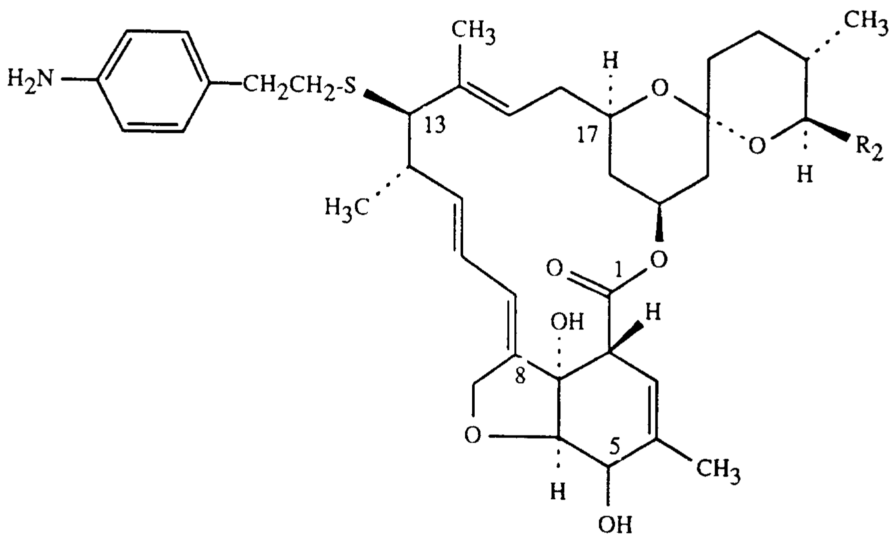

(II),

worin $R_2$ die unter Formel I angegebenen Bedeutungen hat,
α) in den Fällen, in denen X in der Formel I für -C(O)- oder -OC(O)- steht, mit einer Verbindung der Formel III

$R_3$-X-T (III)

in Abwesenheit oder vorzugsweise in Anwesenheit eines Neutralisationsmittels umsetzt,
wobei $R_3$ die unter Formel I angegebenen Bedeutungen hat, X für -C(O)- oder -OC(O)-steht und T eine Abgangsgruppe repräsentiert;

β) in den Fällen, in denen X in der Formel I für -NH-C(O)- steht, mit einem Isocyanat der Formel IV

$R_3$-N = C = O (IV)

oder mit einem Aminoacylierungsmittel der Formel V

$R_3$-NH-C(O)-D (V)

umsetzt, wobei $R_3$ jeweils die unter Formel I angegebenen Bedeutungen hat und D für eine unter T genannte Abgangsgruppe oder vorzugsweise für unsubstituiertes oder substituiertes Phenoxy steht;
γ) in den Fällen, in denen X in der Formel I für -NH-C(S)- steht, mit einem Isothiocyanat der Formel VI

$R_3$-N = C = S (VI),

umsetzt, wobei $R_3$ die unter Formel I angegebenen Bedeutungen hat und
δ) in den Fällen, in denen X in der Formel I für $SO_2$ steht, mit einem Sulfonierungsmittel der Formel VIa

$R_3$-$SO_2$-T (VIa),

in Abwesenheit oder vorzugsweise in Anwesenheit eines Neutralisationsmittels umsetzt,
wobei $R_3$ die unter Formel I angegebenen Bedeutungen hat und T wie unter α) definiert ist.

**2.** Verfahren zur Herstellung einer Verbindung der Formel I nach Anspruch 1, in welcher
$R_2$ für Methyl, Ethyl, Isopropyl, sek.Butyl oder für die Gruppe -C($CH_3$) = CHQ steht,
wobei
Q Methyl, Ethyl oder Isopropyl bedeutet;
und
$R_1$ die Gruppe

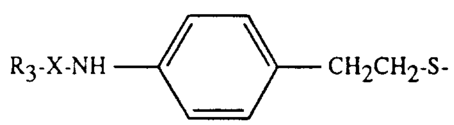

repräsentiert;
wobei X für eine der Gruppen -C(O)-, -OC(O)-, -NHC(O)-, -NHC(S)- oder -$SO_2$- steht;
und
$R_3$ für Wasserstoff, $Het_1$, unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl;
unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl oder für die Gruppe

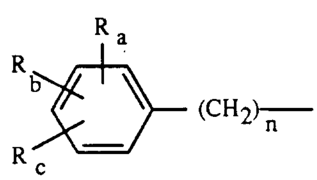

steht, wobei
n für 0, 1 oder 2 steht,
$R_a$, $R_b$ und $R_c$ unabhängig voneinander Wasserstoff, $C_1$-$C_4$-Alkyl, $C_1$-$C_4$-Alkoxy, $C_1$-$C_4$-Alkylthio, $C_1$-$C_4$-Haloalkyl, $C_1$-$C_4$-Haloalkoxy, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, Halogen, CN, $NO_2$ oder $NH_2$ bedeuten, wobei $R_c$ zusätzlich für $C_3$-$C_8$-Cycloalkyl oder für die Gruppe

$Het_2$-O-

stehen kann, wobei $Het_1$ und $Het_2$, welche nachfolgend unter Het zusammengefasst sind, für eine Gruppe ausgewählt aus der Reihe a bis u stehen:

| Gruppe | Het | Typ |
|---|---|---|
| a) | 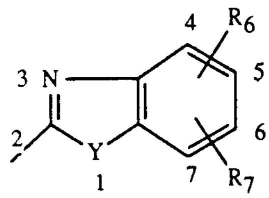 | Benzimidazol-2-yl<br>Benzoxazol-2-yl<br>Benzthiazol-2-yl |
| b) | 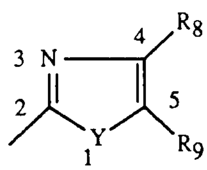 | Imidazol-2-yl<br>Oxazol-2-yl<br>Thiazol-2-yl |
| c) | 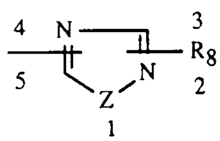 | [1H-1,2,4-Triazol]-5-yl<br>[1,2,4-Oxadiazol]-5-yl<br>[1,2,4-Thiadiazol]-5-yl<br>[1,2,4-Oxadiazol]-3-yl<br>[1,2,4-Thiadiazol]-3-yl |
| d) | 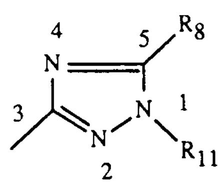 | [1H-1,2,4-Triazol]-3-yl |
| e) | 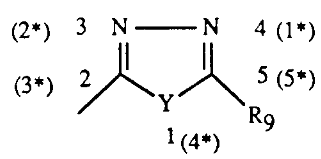 | [1,3,4-Oxadiazol]-2-yl<br>[1,3,4-Thiadiazol]-2-yl<br>* [4H-1,2,4-Triazol]-3-yl |
| f) | 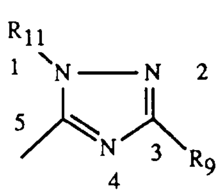 | [1H-1,2,4-Triazol]-5-yl |
| g) | 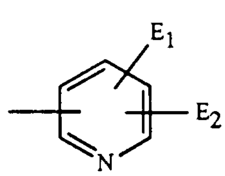 | Pyridin-2-yl<br>Pyridin-3-yl<br>Pyridin-4-yl |

| Gruppe | Het | Typ |
|---|---|---|
| h) | 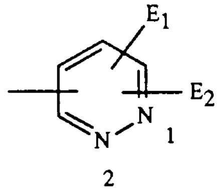 | Pyridazin-3-yl<br>Pyridazin-4-yl |
| i) | 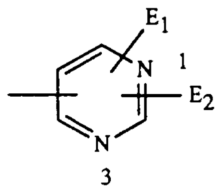 | Pyrimidin-2-yl<br>Pyrimidin-4-yl<br>Pyrimidin-5-yl |
| k) | 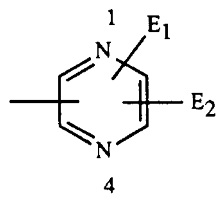 | Pyrazin-2-yl |
| l) | 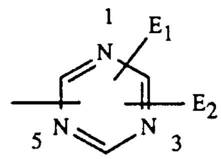 | s-Triazin-2-yl |
| m) | 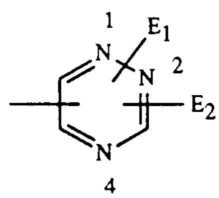 | [1,2,4-Triazin]-3-yl<br>[1,2,4-Triazin]-5-yl<br>[1,2,4-Triazin]-6-yl |
| n) | 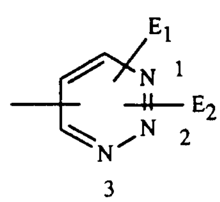 | [1,2,3-Triazin]-4-yl<br>[1,2,3-Triazin]-5-yl |
| o) | 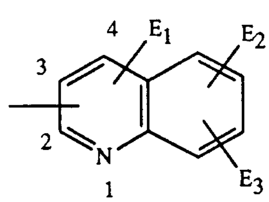 | Chinolin-2-yl<br>Chinolin-3-yl<br>Chinolin-4-yl |

| Gruppe | Het | Typ |
|---|---|---|
| p) | 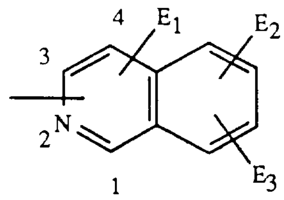 | Isochinolin-1-yl<br>Isochinolin-3-yl<br>Isochinolin-4-yl |
| q) | 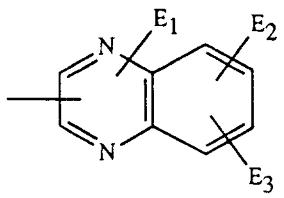 | Chinoxalin-2-yl |
| r) | 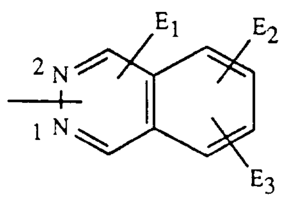 | Phthalazin-3-yl |
| s) | 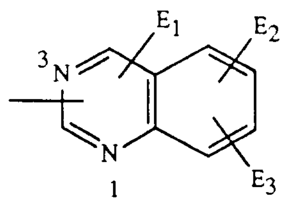 | Chinazolin-2-yl<br>Chinazolin-4-yl |
| t) | 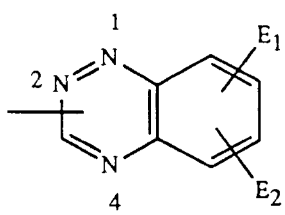 | [1,2,4-Benzotriazin]-3-yl |
| u) | 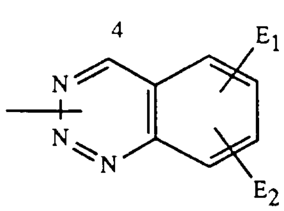 | [1,2,3-Benzotriazin]-4-yl |

wobei

Y für Sauerstoff, Schwefel oder $NR_{10}$ steht;

Z für Sauerstoff, Schwefel oder NH steht;

$R_6$ und $R_7$ unabhängig voneinander Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Alkylsulfonyl, $C_1$-$C_4$-Alkylsulfinyl, $C_1$-$C_4$-Haloalkylsulfonyl, $C_1$-$C_4$-Haloalkylsulfinyl, Halogen, Nitro oder Cyano bedeuten;

$R_8$ und $R_9$ unabhängig voneinander für Wasserstoff, $C_1$-$C_6$-Alkyl, $C_1$-$C_6$-Haloalkyl, $C_1$-$C_6$-Alkoxy, $C_1$-

$C_6$-Alkylthio, Halogen oder Nitro stehen; oder unabhängig voneinander für unsubstituiertes oder durch Halogen oder $C_1$-$C_3$-Alkyl substituiertes $C_3$-$C_7$-Cycloalkyl stehen;
$R_{10}$ Wasserstoff oder $C_1$-$C_6$-Alkyl bedeutet;
$R_{11}$ für $C_1$-$C_6$-Alkyl steht;
und
$E_1$, $E_2$ und $E_3$ unabhängig voneinander für Wasserstoff, $C_1$-$C_4$-Alkyl, $C_1$-$C_4$-Haloalkyl, $C_1$-$C_4$-Alkoxy, $C_1$-$C_4$-Haloalkoxy, $C_1$-$C_4$-Alkylthio, $C_1$-$C_4$-Haloalkylthio, $C_2$-$C_6$-Alkoxyalkoxy, $C_1$-$C_5$-Alkanoyloxy, $C_1$-$C_5$-Alkoxycarbonyl, $C_3$-$C_8$-Cycloalkyl, Halogen, CN, $NO_2$ oder $NH_2$ stehen.

**3.** Verfahren zur Herstellung einer Verbindung der Formel I nach Anspruch 2, worin $E_1$, $E_2$ und $E_3$ unabhängig voneinander für Wasserstoff, Methyl, Ethyl, $CF_3$, Methoxy, $OCF_3$, Methylthio, $CH_3OCH_2O$, Cyclopropyl, Fluor, Chlor oder Brom stehen und die übrigen Substituenten wie in Anspruch 2 definiert sind.

**4.** Verfahren zur Herstellung einer Verbindung der Formel I nach Anspruch 2, in welcher innerhalb der Gruppen b bis f $R_8$ und $R_9$ unabhängig voneinander für unsubstituiertes oder durch Halogen oder $C_1$-$C_3$-Alkyl substituiertes $C_3$-$C_7$-Cycloalkyl stehen und die übrigen Substituenten sowie Y und Z wie in Anspruch 2 definiert sind.

**5.** Verfahren zur Herstellung einer Verbindung der Formel I nach Anspruch 2, in welcher der Substituent Het für

a') 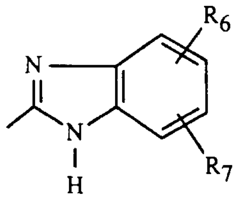 , 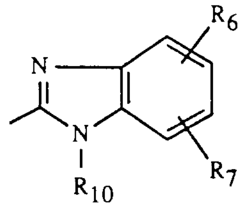 , 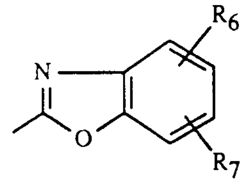 , 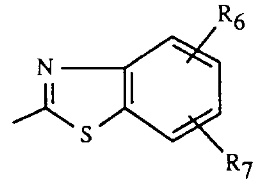

b') 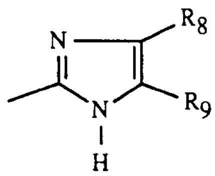 , 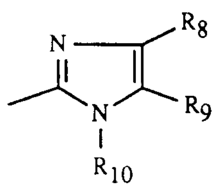 , 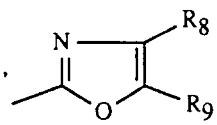 , 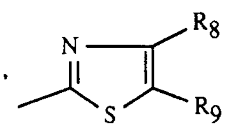 ,

c') 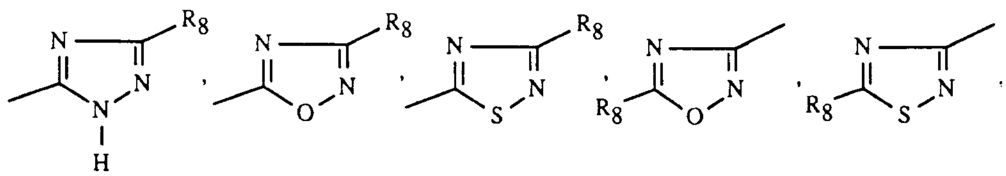 ,

d') 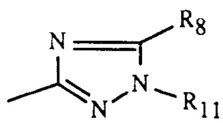

e') 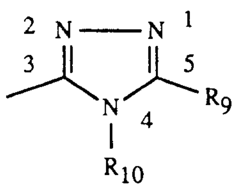 , 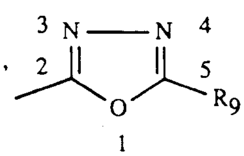 , 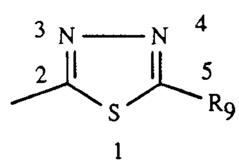 ,

f') 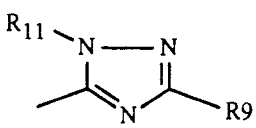 ,

steht und die übrigen Substituenten wie in Anspruch 2 definiert sind.

**6.** Verfahren zur Herstellung einer Verbindung der Formel I nach einem der Ansprüche 2 bis 5, in welcher $R_2$ für Methyl oder Ethyl steht.

**7.** Verfahren zur Herstellung einer Verbindung der Formel I nach Anspruch 1, in welcher $R_2$ und $R_3$ unabhängig voneinander für Methyl, Ethyl oder Isopropyl stehen und X die unter Formel I angegebenen Bedeutungen hat.

**8.** Verfahren zur Herstellung einer Verbindung der Formel I nach Anspruch 1, in welcher
$R_2$ für Methyl, Ethyl, Isopropyl oder sek.Butyl steht;
$R_1$ die Gruppe

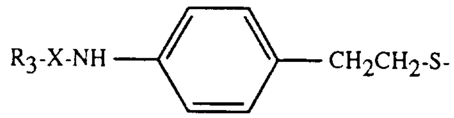

repräsentiert;
wobei X für eine der Gruppen -C(O)-, -OC(O)-, -NHC(O)-, -NHC(S)- oder $-SO_2-$ steht;
und
$R_3$ für $C_1$-$C_8$-Alkyl oder $C_3$-$C_6$-Cycloalkyl steht.

**9.** Verfahren zur Herstellung einer Verbindung der Formel I nach Anspruch 1, in welcher
$R_2$ für Methyl, Ethyl, Isopropyl oder sek.Butyl steht;
$R_1$ die Gruppe

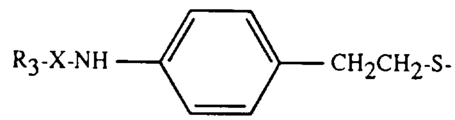

repräsentiert;
wobei X für eine der Gruppen -C(O)-, -OC(O)-, -NHC(O)-, -NHC(S)- oder $-SO_2-$ steht;
und
$R_3$ für die Gruppe

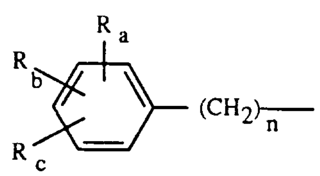

steht, wobei
n für 0 oder 1 steht und
$R_a$, $R_b$ und $R_c$ unabhängig voneinander für Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten und $R_c$ zusätzlich für die Gruppe $Het_2$-O- stehen kann, wobei $Het_2$ die unter Formel I angegebenen Gruppen repräsentiert.

**10.** Verfahren zur Herstellung einer Verbindung der Formel I nach Anspruch 2, in welcher $R_2$ für Methyl, Ethyl, Isopropyl oder sek.Butyl steht;
$R_1$ die Gruppe

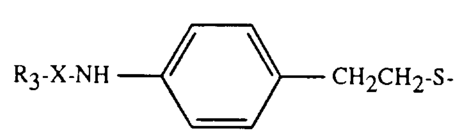

repräsentiert;
wobei X für eine der Gruppen -C(O)-, -OC(O)- oder -NHC(O)- steht; und
$R_3$ für $Het_1$, unsubstituiertes oder durch $C_1$-$C_4$-Alkoxy, $C_1$-$C_4$-Alkylthio oder Halogen substituiertes $C_1$-$C_6$-Alkyl, unsubstituiertes oder durch $C_1$-$C_2$-Alkoxy, $C_1$-$C_2$-Alkylthio oder Halogen substituiertes $C_3$-$C_6$-Cycloalkyl oder die Gruppe

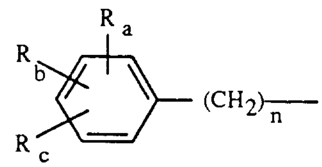

steht, wobei n für 0 oder 1 steht;
$R_a$, $R_b$ und $R_c$ unabhängig voneinander Wasserstoff, Fluor, Chlor, Brom, Methyl, Methoxy, Methylthio, $CF_3$ oder $NO_2$ bedeuten, wobei $R_c$ zusätzlich für die Gruppe

$Het_2$-O-

stehen kann, wobei $Het_1$ und $Het_2$ für eine der in Anspruch 5 für Het definierten Gruppen a') bis f') steht und die übrigen Substituenten wie in Anspruch 2 definiert sind.

**11.** Verfahren zur Herstellung einer Verbindung der Formel I nach Anspruch 1, in welcher $R_2$ für Methyl, Ethyl, Isopropyl oder sek.Butyl steht;
$R_1$ die Gruppe

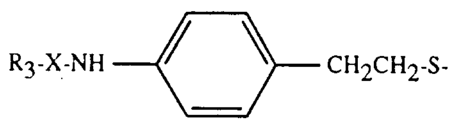

repräsentiert,
wobei X für -OC(O)- steht; und
$R_3$ für unsubstituiertes oder durch $C_1$-$C_4$-Alkoxy, $C_1$-$C_4$-Alkylthio oder Halogen substituiertes $C_1$-$C_6$-Alkyl steht.

**12.** Verfahren zur Herstellung einer Verbindung der Formel I nach Anspruch 1, in welcher $R_2$ für Methyl, Ethyl, Isopropyl oder sek.Butyl steht;
$R_1$ die Gruppe

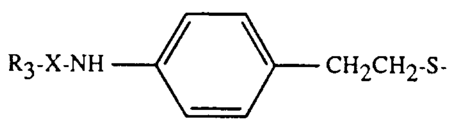

repräsentiert,
wobei X für -C(O)-, -OC(O)- oder -NHC(O)- steht; und
$R_3$ für Wasserstoff; unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_1$-$C_8$-Alkyl; unsubstituiertes oder durch $C_1$-$C_4$-Alkoxy, $C_1$-$C_4$-Alkylthio, $C_2$-$C_5$-Alkoxycarbonyl, Halogen, CN, $NO_2$, COOH oder $NH_2$ substituiertes $C_3$-$C_8$-Cycloalkyl steht.

**13.** Verfahren zur Herstellung einer Verbindung der Formel I nach Anspruch 2, in welcher $R_2$ für Methyl, Ethyl, Isopropyl oder sek.Butyl steht;
$R_1$ die Gruppe

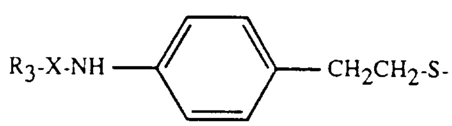

repräsentiert,
wobei X für -C(O)-, -OC(O)- oder -NHC(O)- steht; und $R_3$ für die Gruppe

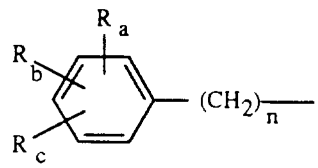

steht, wobei n für 0 oder 1 steht und
$R_a$, $R_b$ und $R_c$ unabhängig voneinander Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten und
$R_c$ zusätzlich für die Gruppe

$Het_2$-O-

stehen kann, wobei $Het_2$ für eine der im Anspruch 5 für Het definierten Gruppen a') bis f') steht und die übrigen Substituenten wie in Anspruch 2 definiert sind.

**14.** Verfahren zur Herstellung einer Verbindung der Formel I nach Anspruch 1, in welcher $R_2$ für Methyl, Ethyl, Isopropyl oder sek.Butyl steht;
$R_1$ die Gruppe

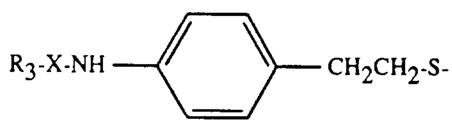

repräsentiert,
wobei X für -C(O)-, -OC(O)- oder -NHC(O)- steht; und $R_3$ für die Gruppe

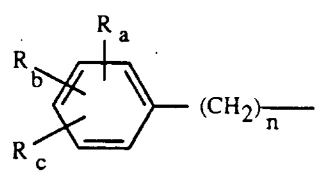

steht, wobei n für 0 oder 1 steht und
$R_a$, $R_b$ und $R_c$ unabhängig voneinander für Wasserstoff, $C_1$-$C_2$-Alkyl, $C_1$-$C_2$-Alkoxy, $C_1$-$C_2$-Alkylthio, $C_1$-$C_2$-Haloalkyl, $C_1$-$C_2$-Haloalkoxy, $C_1$-$C_2$-Haloalkylthio, Halogen oder $NO_2$ bedeuten.

**15.** Verfahren zur Herstellung einer Verbindung der Formel I nach Anspruch 1, in welcher $R_2$ für Methyl oder Ethyl steht;
$R_1$ die Gruppe

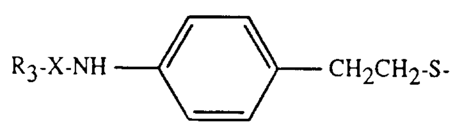

repräsentiert,
wobei X für -OC(O)- steht und $R_3$ $C_1$-$C_6$-Alkyl bedeutet.

**16.** Verfahren zur Herstellung einer Verbindung der Formel I nach Anspruch 1, ausgewählt aus der Reihe
13β-[2-(4-Acetaminophenyl)ethylthio]milbemycin A4;
13β-(2-[4-(4-Heptylcarbonylamino)phenyl]ethylthio)milbemycin A4;
13β-(2-[4-(2,6-Dichlorpyridin-4-ylcarbonylamino)phenyl]ethylthio)-milbemycin A4;
13β-(2-[4-Tosylphenyl]ethylthio)milbemycin A4;
13β-(2-[4-(Methylaminothiocarbonylamino)phenyl]ethylthio)milbemcyin A4;
13β-(2-[4-(Benzoylaminocarbonylamino)phenyl]ethylthio)milbemycin A4;
13β-(2-[4-(Cyclohexylaminocarbonylamino)phenyl]ethylthio)milbemcyin A4;
13β-(2-[4-((4-Methoxyphenyl)aminocarbonylamino)phenyl]ethylthio)milbemycin A4;
13β-(2-[4-(Carboxymethylaminocarbonylamino)phenyl]ethylthio)milbemycin A4; und
13β-(2-[4-(Methoxycarbonylamino)phenyl]ethylthio)milbemycin A4.

**Claims**

**Claims for the following Contracting States : AT, BE, CH, DE, DK, FR, GB, IT, LI, LU, NL, SE**

**1.** A compound of formula I

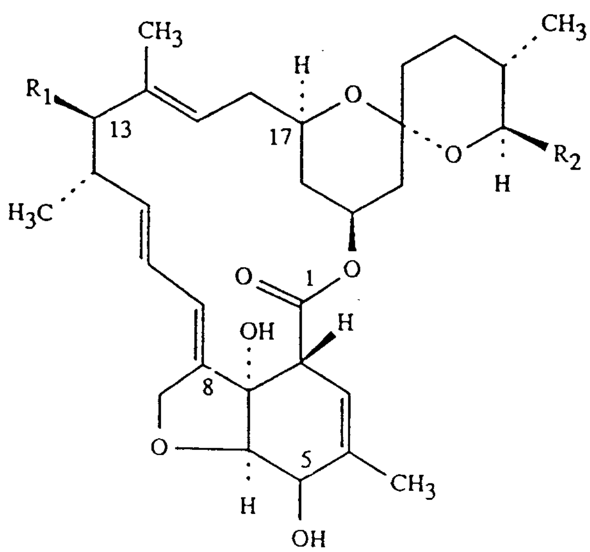

(I),

wherein
$R_2$ is methyl, ethyl, isopropyl, sec-butyl or the group $-C(CH_3)=CHQ$
wherein
Q is methyl, ethyl or isopropyl;
and $R_1$ is the group

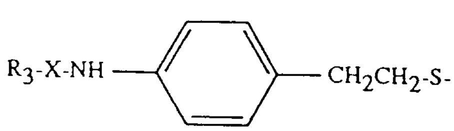

wherein X is a group selected from -C(O)-, -OC(O)-, -NHC(O)-, -NHC(S)- and $-SO_2-$; and
$R_3$ is hydrogen, $Het_1$, unsubstituted $C_1$-$C_8$alkyl or $C_1$-$C_8$alkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is unsubstituted $C_3$-$C_8$cycloalkyl or $C_3$-$C_8$cycloalkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is the group

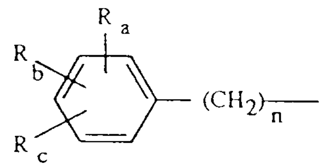

wherein
n is 0, 1 or 2,
$R_a$, $R_b$ and $R_c$ are each independently of one another hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, $C_1$-$C_4$haloalkylthio, $C_2$-$C_6$alkoxyalkoxy, $C_1$-$C_5$alkanoyloxy, $C_1$-$C_5$alkoxycarbonyl, halogen, CN, $NO_2$ or $NH_2$, and $R_c$ may additionally be $C_3$-$C_8$cycloalkyl or the group

$Het_2$-O-

and $Het_1$ and $Het_2$ are each an unsubstituted or substituted heterocycle which is bonded *via* carbon and is selected from the series benzimidazole, benzoxazole, benzthiazole, imidazole, oxazole, thiazole, oxadiazole, thiadiazole, triazole, pyridine, pyridazine, pyrimidine, pyrazine, triazine, quinoline, isoquinoline, quinoxaline, phthalazine, quinazoline and benzotriazine; or a physiologically acceptable acid addition salt thereof.

**2.** A compound of formula I according to claim 1, wherein
$R_2$ is methyl, ethyl, isopropyl, sec-butyl or the group $-C(CH_3)=CHQ$
wherein
Q is methyl, ethyl or isopropyl;
and $R_1$ is the group

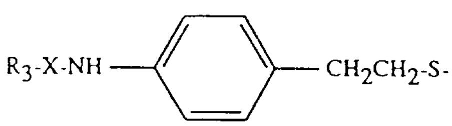

wherein X is a group selected from -C(O)-, -OC(O)-, -NHC(O)-, -NHC(S)- and $-SO_2$-; and
$R_3$ is hydrogen, $Het_1$, unsubstituted $C_1$-$C_8$alkyl or $C_1$-$C_8$alkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is unsubstituted $C_3$-$C_8$cycloalkyl or $C_3$-$C_8$cycloalkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is the group

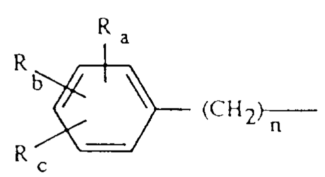

wherein
n is 0, 1 or 2,
$R_a$, $R_b$ and $R_c$ are each independently of one another hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, $C_1$-$C_4$haloalkylthio, $C_2$-$C_6$alkoxyalkoxy, $C_1$-$C_5$alkanoyloxy, $C_1$-$C_5$alkoxycarbonyl, halogen, CN, $NO_2$ or $NH_2$, and $R_c$ may additionally be $C_3$-$C_8$cycloalkyl or the group

$Het_2$-O-,

and $Het_1$ and $Het_2$, hereinafter comprised under Het, are each a group selected from the series a to u:

| Group | Het | Type |
|---|---|---|
| a) | 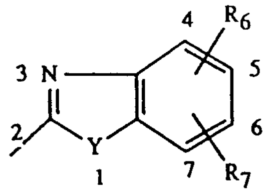 | benzimidazol-2-yl<br>benzoxazol-2-yl<br>benzthiazol-2-yl |
| b) | 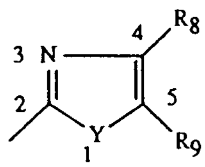 | imidazol-2-yl<br>oxazol-2-yl<br>thiazol-2-yl |
| c) | 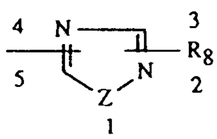 | [1H-1,2,4-triazol]-5-yl<br>[1,2,4-oxadiazol]-5-yl<br>[1,2,4-thiadiazol]-5-yl<br>[1,2,4-oxadiazol]-3-yl<br>[1,2,4-thiadiazol]-3-yl |
| d) | 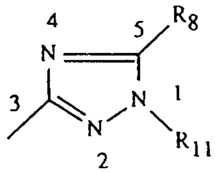 | [1H-1,2,4-triazol]-3-yl |
| e) | 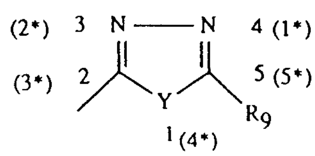 | [1,3,4-oxadiazol]-2-yl<br>[1,3,4-thiadiazol]-2-yl<br>* [4H-1,2,4-triazol]-3-yl |
| f) | 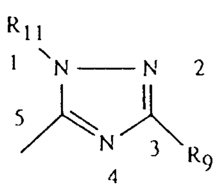 | [1H-1,2,4-triazol]-5-yl |
| g) | 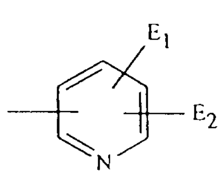 | pyridin-2-yl<br>pyridin-3-yl<br>pyridin-4-yl |

| Group | Het | Type |
|---|---|---|
| h) | 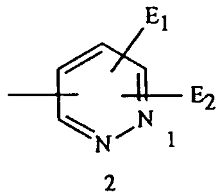 | pyridazin-3-yl<br>pyridazin-4-yl |
| i) | 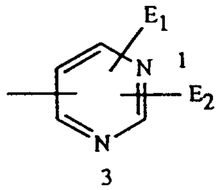 | pyrimidin-2-yl<br>pyrimidin-4-yl<br>pyrimidin-5-yl |
| k) | 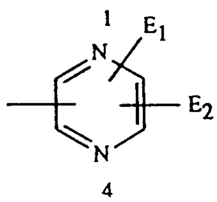 | pyrazin-2-yl |
| l) | 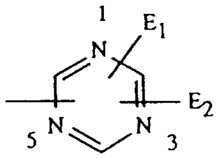 | s-triazin-2-yl |
| m) | 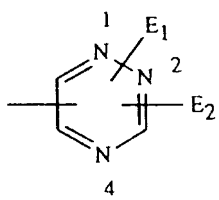 | [1,2,4-triazin]-3-yl<br>[1,2,4-triazin]-5-yl<br>[1,2,4-triazin]-6-yl |
| n) | 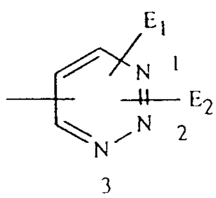 | [1,2,3-triazin]-4-yl<br>[1,2,3-triazin]-5-yl |
| o) | 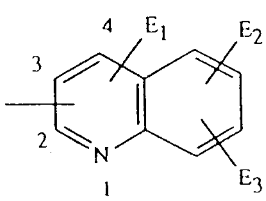 | quinolin-2-yl<br>quinolin-3-yl<br>quinolin-4-yl |

| Group | Het | Type |
| --- | --- | --- |
| p) | 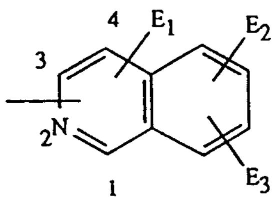 | isoquinolin-1-yl<br>isoquinolin-3-yl<br>isoquinolin-4-yl |
| q) | 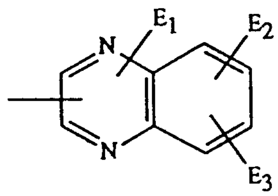 | quinoxalin-2-yl |
| r) | 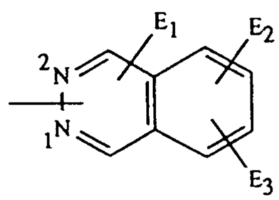 | phthalazin-3-yl |
| s) | 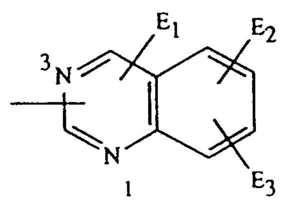 | quinazolin-2-yl<br>quinazolin-4-yl |
| t) | 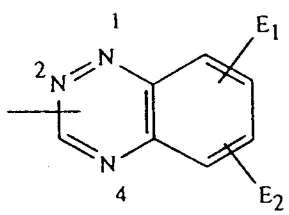 | [1,2,4-benzotriazin]-3-yl |
| u) | 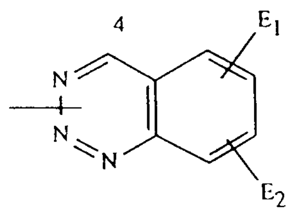 | [1,2,3-benzotriazin]-4-yl |

in which formulae
Y is oxygen, sulfur or $NR_{10}$;
Z is oxygen, sulfur or NH;
$R_6$ and $R_7$ are each independently of the other hydrogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, $C_1$-$C_4$alkylsulfonyl, $C_1$-$C_4$alkylsulfinyl, $C_1$-$C_4$haloalkylsulfonyl, $C_1$-$C_4$haloalkylsulfinyl, halogen, nitro or cyano;
$R_8$ and $R_9$ are each independently of the other hydrogen, $C_1$-$C_6$alkyl, $C_1$-$C_6$haloalkyl, $C_1$-$C_6$alkoxy, $C_1$-$C_6$alkylthio, halogen or nitro; or are each independently of the other unsubstituted $C_3$-$C_7$cycloalkyl or $C_3$-$C_7$cycloalkyl which is substituted by halogen or $C_1$-$C_3$alkyl;
$R_{10}$ is hydrogen or $C_1$-$C_6$alkyl;

$R_{11}$ is $C_1$-$C_6$alkyl:
and
$E_1$, $E_2$ and $E_3$ are each independently of one another hydrogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, $C_2$-$C_6$alkoxyalkoxy, $C_1$-$C_5$alkanoyloxy, $C_1$-$C_5$alkoxycarbonyl, $C_3$-$C_8$cycloalkyl, halogen, CN, $NO_2$ or $NH_2$.

**3.** A compound of formula I according to claim 2, wherein $E_1$, $E_2$ and $E_3$ are each independently of one another hydrogen, methyl, ethyl, $CF_3$, methoxy, $OCF_3$, methylthio, $CH_3OCH_2O$, cyclopropyl, fluoro, chloro or bromo, and the other substituents are as defined in claim 2.

**4.** A compound of formula I according to claim 2, wherein $R_8$ and $R_9$ in the groups b to f are each independently of the other $C_3$-$C_7$cycloalkyl which is unsubstituted or substituted by halogen or $C_1$-$C_3$alkyl and the other substituents and Y and Z are as defined in claim 2.

**5.** A compound of formula I according to claim 2, wherein the substituent Het is

a')

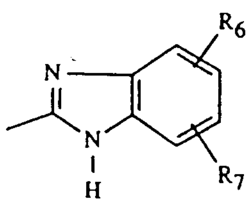 , 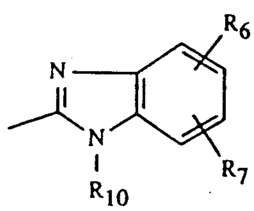 , 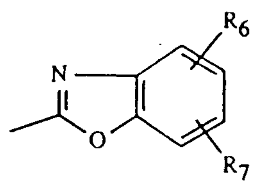 , 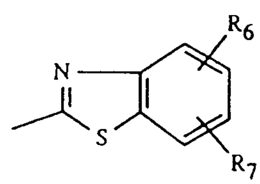 ,

b') 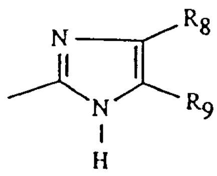 , 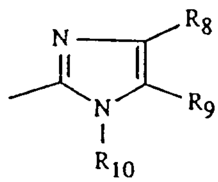 , 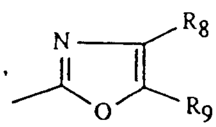 , 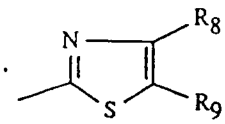 ,

c') 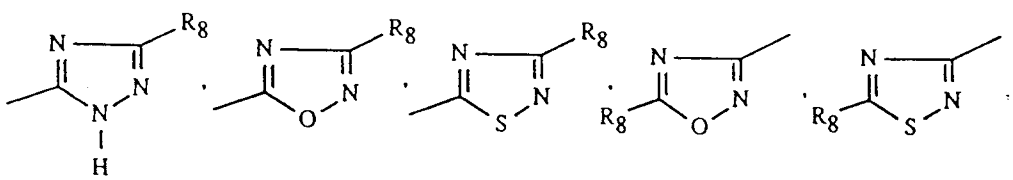

d') 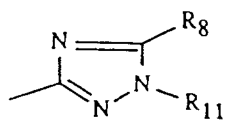

e') 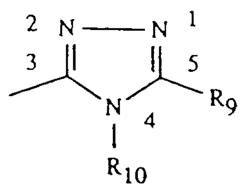 , 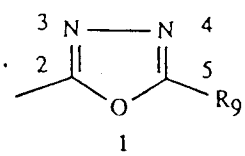 , 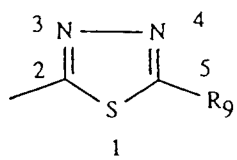 ,

f') 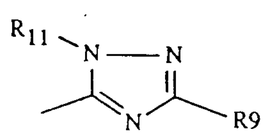 ,

and the other substituents are as defined in claim 2.

6. A compound of formula I according to any one of claims 2 to 5, wherein $R_2$ is methyl or ethyl.

7. A compound of formula I according to claim 1, wherein $R_2$ and $R_3$ are each independently of the other methyl, ethyl or isopropyl, and X is as defined for formula I.

8. A compound of formula I according to claim 1, wherein $R_2$ is methyl, ethyl, isopropyl or sec-butyl; $R_1$ is the group

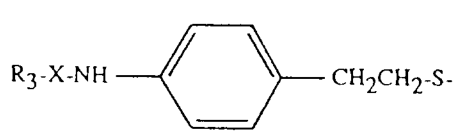

wherein X is a group selected from -C(O)-, -OC(O)-, -NHC(O)-, -NHC(S)- and -$SO_2$-; and $R_3$ is $C_1$-$C_8$alkyl or $C_3$-$C_6$cycloalkyl.

9. A compound of formula I according to claim 1, wherein $R_2$ is methyl, ethyl, isopropyl or sec-butyl; $R_1$ is the group

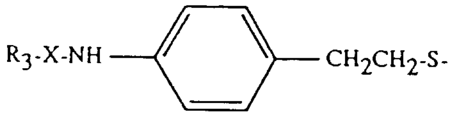

wherein X is a group selected from -C(O)-, -OC(O)-, -NHC(O)-, -NHC(S)- and $-SO_2-$; and
$R_3$ is the group

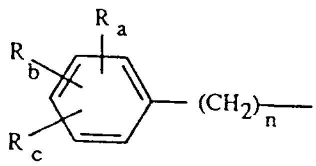

wherein
n is 0 or 1, and
$R_a$, $R_b$ and $R_c$ are each independently of one another hydrogen, $C_1$-$C_2$alkyl, $C_1$-$C_2$alkoxy, $C_1$-$C_2$alkylthio, $C_1$-$C_2$haloalkyl, $C_1$-$C_2$haloalkoxy, $C_1$-$C_2$haloalkylthio, halogen or $NO_2$, and $R_c$ may additionally be the group $Het_2$-O-, wherein $Het_2$ denotes the groups as indicated for formula I.

**10.** A compound of formula I according to claim 2, wherein $R_2$ is methyl, ethyl, isopropyl or sec-butyl;
$R_1$ is the group

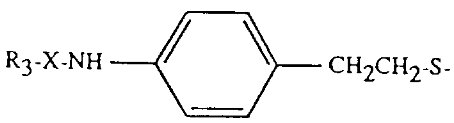

wherein X is a group selected from -C(O)-, -OC(O)- or -NHC(O)-; and
$R_3$ is $Het_1$, unsubstituted $C_1$-$C_6$alkyl or $C_1$-$C_6$alkyl which is substituted by $C_1$-$C_4$alkoxy, $C_1$-$C_4$alkylthio or halogen; or is unsubstituted $C_3$-$C_6$cycloalkyl or $C_3$-$C_6$cycloalkyl which is substituted by $C_1$-$C_2$alkoxy, $C_1$-$C_2$alkylthio or halogen; or is the group

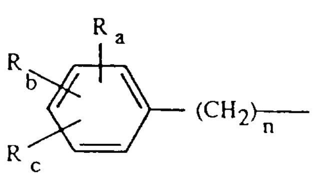 ,

wherein n is 0 or 1;
$R_a$, $R_b$ and $R_c$ are each independently of one another hydrogen, fluoro, chloro, bromo, methyl, methoxy, methylthio, $CF_3$ or $NO_2$, and $R_c$ may additionally be the group

$Het_2$-O-

and $Het_1$ and $Het_2$ are one of the groups a') to f') defined for Het in claim 5, and the other substituents are as defined for claim 2.

**11.** A compound of formula I according to claim 1, wherein
$R_2$ is methyl, ethyl, isopropyl or sec-butyl;
$R_1$ is the group

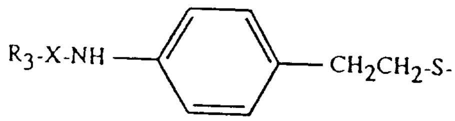

wherein X is -OC(O)-; and
$R_3$ is unsubstituted $C_1$-$C_6$alkyl or $C_1$-$C_6$alkyl which is substituted by $C_1$-$C_4$alkoxy, $C_1$-$C_4$alkylthio or halogen.

**12.** A compound of formula I according to claim 1, wherein
$R_2$ is methyl, ethyl, isopropyl or sec-butyl;
$R_1$ is the group

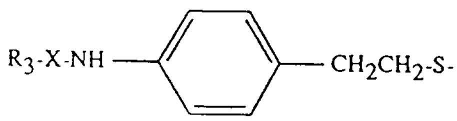

wherein X is -C(O)-, -OC(O)- or -NHC(O)-; and
$R_3$ is hydrogen; unsubstituted $C_1$-$C_8$alkyl or $C_1$-$C_8$alkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is unsubstituted $C_3$-$C_8$cycloalkyl or $C_3$-$C_8$cycloalkyl which is substituted by $C_1$-$C_4$alkoxy, $C_1$-$C_4$alkylthio, $C_2$-$C_5$alkoxycarbonyl, halogen, CN, $NO_2$, COOH or $NH_2$.

**13.** A compound of formula I according to claim 2, wherein
$R_2$ is methyl, ethyl, isopropyl or sec-butyl;
$R_1$ is the group

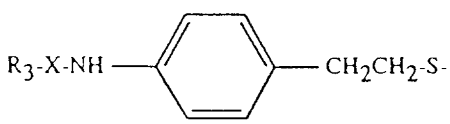

wherein X is -C(O)-, -OC(O)- or -NHC(O)-; and $R_3$ is the group

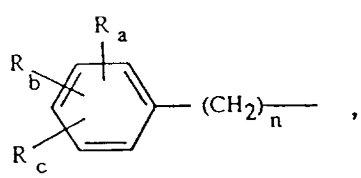

wherein n is 0 or 1, and
$R_a$, $R_b$ and $R_c$ are each independently of one another hydrogen, $C_1$-$C_2$alkyl, $C_1$-$C_2$alkoxy, $C_1$-$C_2$alkylthio, $C_1$-$C_2$haloalkyl, $C_1$-$C_2$haloalkoxy, $C_1$-$C_2$haloalkylthio, halogen or $NO_2$, and
$R_c$ may additionally be the group

$Het_2$-O-,

wherein $Het_2$ is one of the groups a') to f') defined for Het in claim 5 and the other substituents are as defined in claim 2.

**14.** A compound of formula I according to claim 1, wherein
$R_2$ is methyl, ethyl, isopropyl or sec-butyl;
$R_1$ is the group

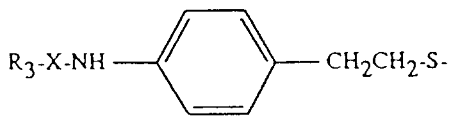

wherein X is -C(O)-, -OC(O)- or -NHC(O)-; and $R_3$ is the group

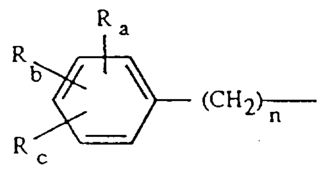

,

wherein n is 0 or 1, and
$R_a$, $R_b$ and $R_c$ are each independently of one another hydrogen, $C_1$-$C_2$alkyl, $C_1$-$C_2$alkoxy, $C_1$-$C_2$alkylthio, $C_1$-$C_2$haloalkyl, $C_1$-$C_2$haloalkoxy, $C_1$-$C_2$haloalkylthio, halogen or $NO_2$.

**15.** A compound of formula I according to claim 1, wherein
$R_2$ is methyl or ethyl;
$R_1$ is the group

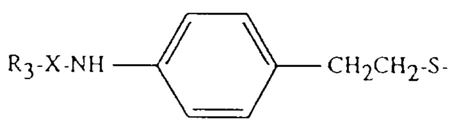

wherein X is -OC(O)-, and $R_3$ is $C_1$-$C_6$alkyl.

**16.** A compound of formula I according to claim 1, selected from the series:
13$\beta$-[2-(4-acetaminophenyl)ethylthio]milbemycin A4;
13$\beta$-(2-[4-(4-heptylcarbonylamino)phenyl]ethylthio)milbemycin A4;
13$\beta$-(2-[4-(2,6-dichloropyridin-4-ylcarbonylamino)phenyl]ethylthio)-milbemycin A4;
13$\beta$-(2-[4-tosylphenyl]ethylthio)milbemycin A4;
13$\beta$-(2-[4-(methylaminothiocarbonylamino)phenyl]ethylthio)milbemycin A4;
13$\beta$-(2-[4-(benzoylaminocarbonylamino)phenyl]ethylthio)milbemcyin A4;
13$\beta$-(2-[4-(cyclohexylaminocarbonylamino)phenyl]ethylthio)milbemcyin A4;
13$\beta$-(2-[4-((4-methoxyphenyl)aminocarbonylamino)phenyl]ethylthio)milbemycin A4;
13$\beta$-(2-[4-(carboxymethylaminocarbonylamino)phenyl]ethylthio)milbemycin A4; and
13$\beta$-(2-[4-(methoxycarbonylamino)phenyl]ethylthio)milbemycin A4.

**17.** A process for the preparation of a compound of formula I according to claim 1, which comprises reacting a compound of formula II

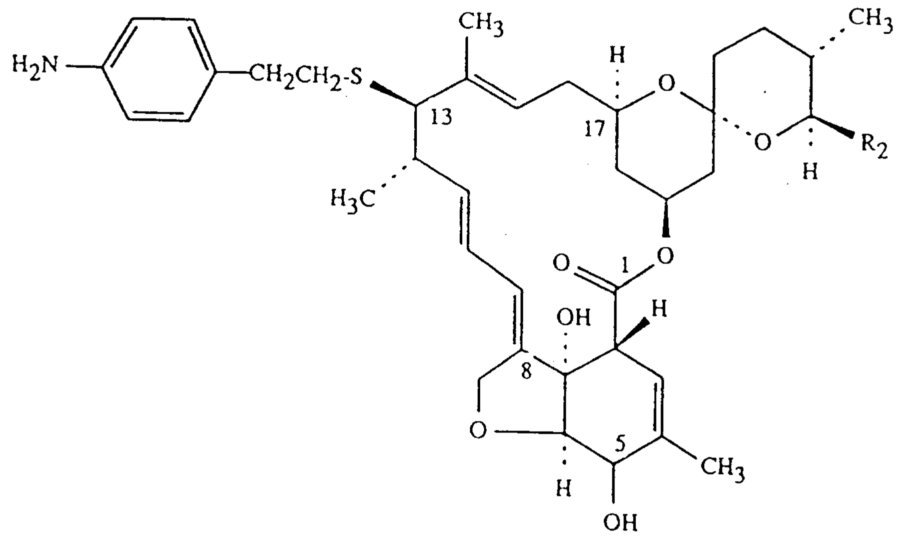

(II),

wherein $R_2$ is as defined for formula I,

α) where X in formula I is -C(O)- or -OC(O)-, in the absence, or preferably in the presence, of a neutralising agent, with a compound of formula III

$R_3$-X-T (III)

wherein $R_3$ is as defined for formula I, X is -C(O)- or -OC(O)-, and T is a leaving group;

β) where X in formula I is -NH-C(O)-, with an isocyanate of formula IV

$R_3$-N=C=O (IV)

or with an aminoacylating agent of formula V

$R_3$-NH-C(O)-D (V)

wherein $R_3$ in formulae (IV) and (V) is as defined for formula I, and D is a leaving group as defined for T or is preferably unsubstituted or substituted phenoxy;

γ) where X in formula I is -NH-C(S)-, with an isothiocyanate of formula VI

$R_3$-N=C=S (VI),

wherein $R_3$ is as defined for formula I; and

δ) where X in formula I is $SO_2$, with a sulfonating agent of formula VIa

$R_3$-$SO_2$-T (VIa),

wherein $R_3$ is as defined for formula I and T is as defined in α), in the absence, or preferably in the presence, of a neutralising agent.

**18.** An insecticidal and parasiticidal composition which comprises, as active ingredient, a compound of formula I as claimed in any one of claims 1 to 16.

**19.** A compound of formula I according to any one of claims 1 to 16 for use in the control of parasitic pests on or in animals.

**20.** A compound of formula I according to claim 19 for use in the control of endo- or ecto-parasites of productive livestock.

**21.** The use of a compound of formula I according to any one of claims 1 to 16 in the preparation of a medicament for controlling parasitic pests on or in animals.

**Claims for the following Contracting States : ES, GR**

**1.** A process for the preparation of a compound of formula I

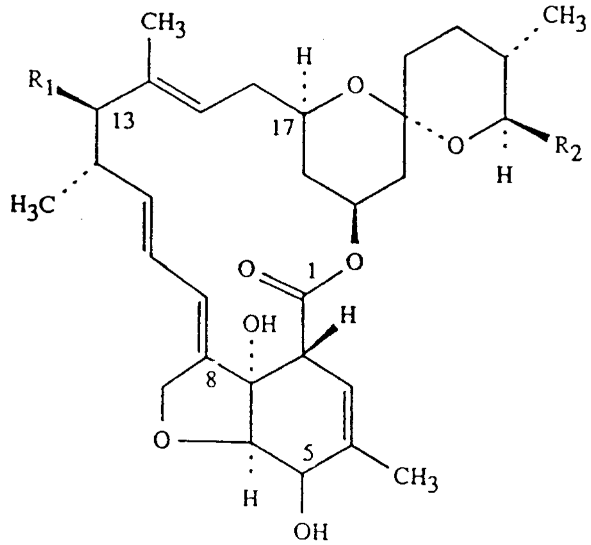

(I),

wherein
$R_2$ is methyl, ethyl, isopropyl, sec-butyl or the group $-C(CH_3)=CHQ$
wherein
Q is methyl, ethyl or isopropyl;
and $R_1$ is the group

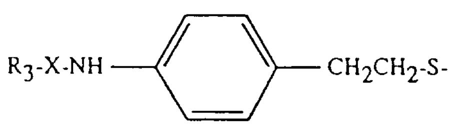

wherein X is a group selected from -C(O)-, -OC(O)-, -NHC(O)-, -NHC(S)- and $-SO_2-$; and
$R_3$ is hydrogen, $Het_1$, unsubstituted $C_1$-$C_8$alkyl or $C_1$-$C_8$alkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is unsubstituted $C_3$-$C_8$cycloalkyl or $C_3$-$C_8$cycloalkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is the group

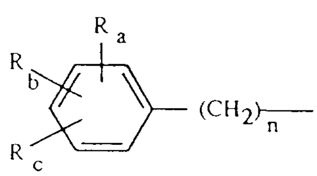

wherein
n is 0, 1 or 2,
$R_a$, $R_b$ and $R_c$ are each independently of one another hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, $C_1$-$C_4$haloalkylthio, $C_2$-$C_6$alkoxyalkoxy, $C_1$-$C_5$alkanoyloxy, $C_1$-$C_5$alkoxycarbonyl, halogen, CN, $NO_2$ or $NH_2$, and $R_c$ may additionally be $C_3$-$C_8$cycloalkyl or the group

$Het_2$-O-

and $Het_1$ and $Het_2$ are an unsubstituted or substituted heterocycle which is bonded *via* carbon and is selected from the series benzimidazole, benzoxazole, benzthiazole, imidazole, oxazole, thiazole, oxadiazole, thiadiazole, triazole, pyridine, pyridazine, pyrimidine, pyrazine, triazine, quinoline, isoquinoline, quinoxaline, phthalazine, quinazoline and benzotriazine; or a physiologically acceptable acid addition salt thereof, which comprises reacting a compound of formula II

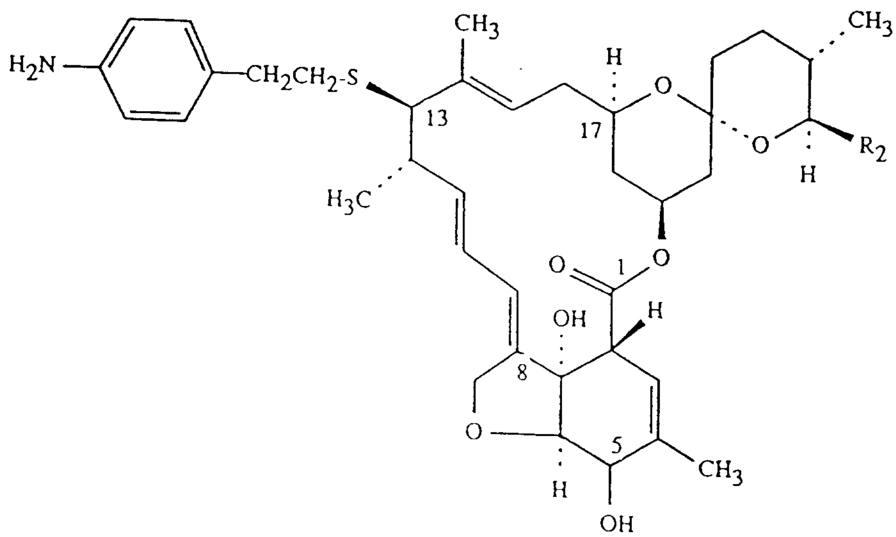

(II),

wherein $R_2$ is as defined for formula I,

α) where X in formula I is -C(O)- or -OC(O)-, in the absence, or preferably in the presence, of a neutralising agent, with a compound of formula III

$R_3$-X-T (III)

wherein $R_3$ is as defined for formula I, X is -C(O)- or -OC(O)-, and T is a leaving group;

β) where X in formula I is -NH-C(O)-, with an isocyanate of formula IV

$R_3$-N = C = O (IV)

or with an aminoacylating agent of formula V

$R_3$-NH-C(O)-D (V)

wherein $R_3$ in formulae (IV) and (V) is as defined for formula I, and D is a leaving group as defined for T or is preferably unsubstituted or substituted phenoxy;

γ) where X in formula I is -NH-C(S)-, with an isothiocyanate of formula VI

$R_3$-N = C = S (VI),

wherein $R_3$ is as defined for formula I; and

δ) where X in formula I is $SO_2$, with a sulfonating agent of formula VIa

$R_3$-$SO_2$-T (VIa),

wherein $R_3$ is as defined for formula I and T is as defined in α), in the absence, or preferably in the presence, of a neutralising agent.

**2.** A process for the preparation of a compound of formula I according to claim 1, wherein $R_2$ is methyl, ethyl, isopropyl, sec-butyl or the group $-C(CH_3)=CHQ$
wherein
Q is methyl, ethyl or isopropyl;
and $R_1$ is the group

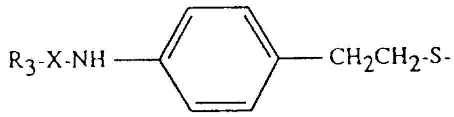

wherein X is a group selected from -C(O)-, -OC(O)-, -NHC(O)-, -NHC(S)- and $-SO_2-$; and
$R_3$ is hydrogen, $Het_1$, unsubstituted $C_1$-$C_8$alkyl or $C_1$-$C_8$alkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is unsubstituted $C_3$-$C_8$cycloalkyl or $C_3$-$C_8$cycloalkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is the group

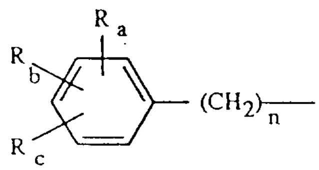

wherein
n is 0, 1 or 2,
$R_a$, $R_b$ and $R_c$ are each independently of one another hydrogen, $C_1$-$C_4$alkyl, $C_1$-$C_4$alkoxy, $C_1$-$C_4$alkylthio, $C_1$-$C_4$haloalkyl, $C_1$-$C_4$haloalkoxy, $C_1$-$C_4$haloalkylthio, $C_2$-$C_6$alkoxyalkoxy, $C_1$-$C_5$alkanoyloxy, $C_1$-$C_5$alkoxycarbonyl, halogen, CN, $NO_2$ or $NH_2$, and $R_c$ may additionally be $C_3$-$C_8$cycloalkyl or the group

$Het_2$-O-,

and $Het_1$ and $Het_2$, hereinafter comprised under Het, are each a group selected from the series a to u:

| Group | Het | Type |
|---|---|---|
| a) | 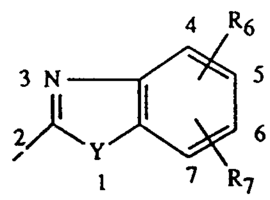 | benzimidazol-2-yl<br>benzoxazol-2-yl<br>benzthiazol-2-yl |
| b) | 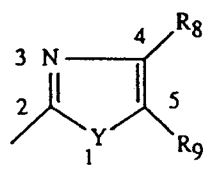 | imidazol-2-yl<br>oxazol-2-yl<br>thiazol-2-yl |
| c) | 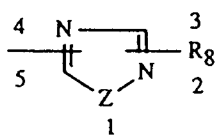 | [1H-1,2,4-triazol]-5-yl<br>[1,2,4-oxadiazol]-5-yl<br>[1,2,4-thiadiazol]-5-yl<br>[1,2,4-oxadiazol]-3-yl<br>[1,2,4-thiadiazol]-3-yl |
| d) | 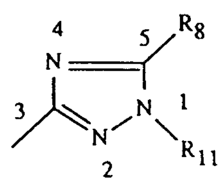 | [1H-1,2,4-triazol]-3-yl |
| e) | 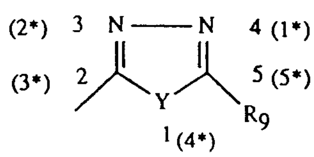 | [1,3,4-oxadiazol]-2-yl<br>[1,3,4-thiadiazol]-2-yl<br>* [4H-1,2,4-triazol]-3-yl |
| f) | 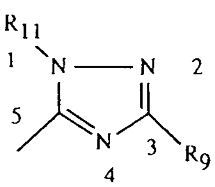 | [1H-1,2,4-triazol]-5-yl |
| g) | 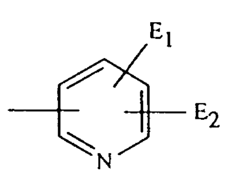 | pyridin-2-yl<br>pyridin-3-yl<br>pyridin-4-yl |

| Group | Het | Type |
|---|---|---|
| h) | 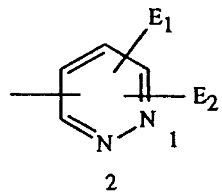 | pyridazin-3-yl<br>pyridazin-4-yl |
| i) | 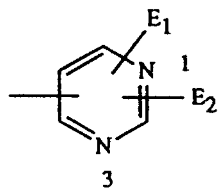 | pyrimidin-2-yl<br>pyrimidin-4-yl<br>pyrimidin-5-yl |
| k) | 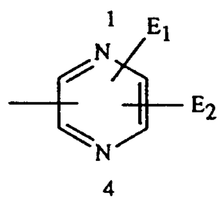 | pyrazin-2-yl |
| l) | 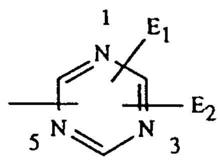 | s-triazin-2-yl |
| m) | 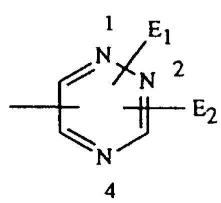 | [1,2,4-triazin]-3-yl<br>[1,2,4-triazin]-5-yl<br>[1,2,4-triazin]-6-yl |
| n) | 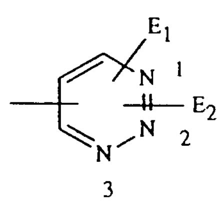 | [1,2,3-triazin]-4-yl<br>[1,2,3-triazin]-5-yl |
| o) | 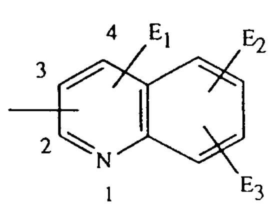 | quinolin-2-yl<br>quinolin-3-yl<br>quinolin-4-yl |

| Group | Het | Type |
|---|---|---|
| p) | 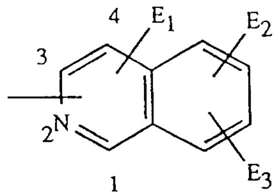 | isoquinolin-1-yl<br>isoquinolin-3-yl<br>isoquinolin-4-yl |
| q) | 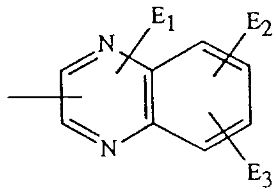 | quinoxalin-2-yl |
| r) | 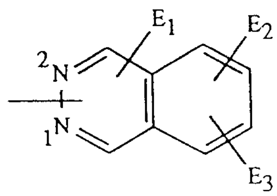 | phthalazin-3-yl |
| s) | 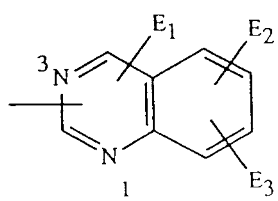 | quinazolin-2-yl<br>quinazolin-4-yl |
| t) | 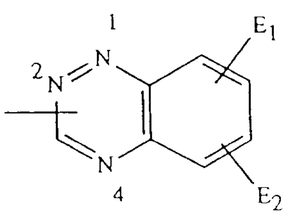 | [1,2,4-benzotriazin]-3-yl |
| u) | 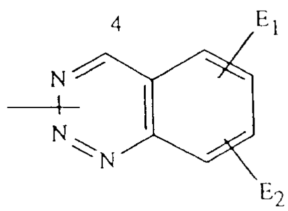 | [1,2,3-benzotriazin]-4-yl |

in which formulae
Y is oxygen, sulfur or $NR_{10}$;
Z is oxygen, sulfur or NH;
$R_6$ and $R_7$ are each independently of the other hydrogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, $C_1$-$C_4$alkylsulfonyl, $C_{1-4}$alkylsulfinyl, $C_1$-$C_4$haloalkylsulfonyl, $C_1$-$C_4$haloalkylsulfinyl, halogen, nitro or cyano;
$R_8$ and $R_9$ are each independently of the other hydrogen, $C_1$-$C_6$alkyl, $C_1$-$C_6$haloalkyl, $C_1$-$C_6$alkoxy, $C_1$-$C_6$alkylthio, halogen or nitro; or are each independently of the other unsubstituted $C_3$-$C_7$cycloalkyl or $C_3$-$C_7$cycloalkyl which is substituted by halogen or $C_1$-$C_3$alkyl;
$R_{10}$ is hydrogen or $C_1$-$C_6$alkyl;

$R_{11}$ is $C_1$-$C_6$alkyl;
and
$E_1$, $E_2$ and $E_3$ are each independently of one another hydrogen, $C_1$-$C_4$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, $C_2$-$C_6$alkoxyalkoxy, $C_1$-$C_5$alkanoyloxy, $C_1$-$C_5$alkoxycarbonyl, $C_3$-$C_8$cycloalkyl, halogen, CN, $NO_2$ or $NH_2$.

**3.** A process for the preparation of a compound of formula I according to claim 2, wherein $E_1$, $E_2$ and $E_3$ are each independently of one another hydrogen, methyl, ethyl, $CF_3$, methoxy, $OCF_3$, methylthio, $CH_3OCH_2O$, cyclopropyl, fluoro, chloro or bromo, and the other substituents are as defined in claim 2.

**4.** A process for the preparation of a compound of formula I according to claim 2, wherein $R_8$ and $R_9$ in the groups b to f are each independently of the other $C_3$-$C_7$cycloalkyl which is unsubstituted or substituted by halogen or $C_1$-$C_3$alkyl and the other substituents and Y and Z are as defined in claim 2.

**5.** A process for the preparation of a compound of formula I according to claim 2, wherein the substituent Het is

a') 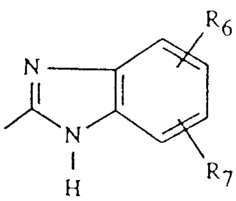 , 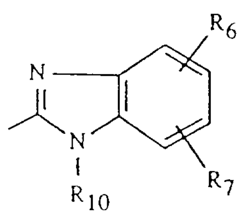 , 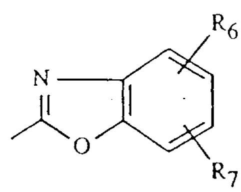 , 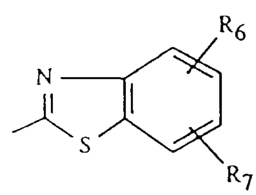

b') 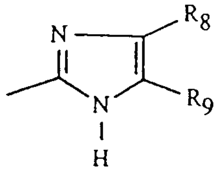 , 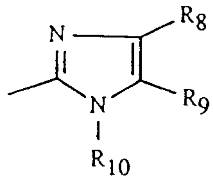 , 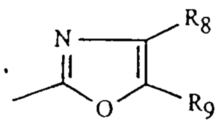 , 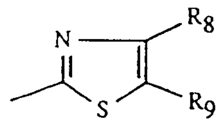 ,

c') 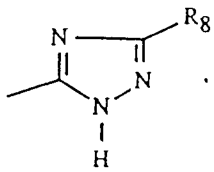 , 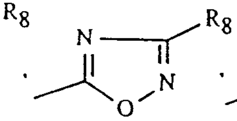 , 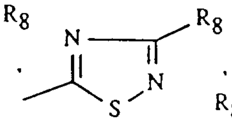 , 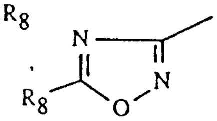 , 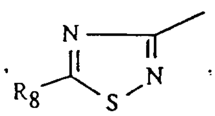 ,

d') 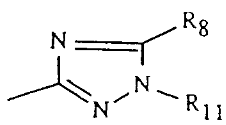

e') 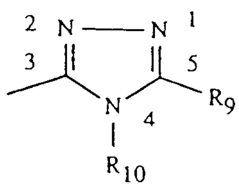 , 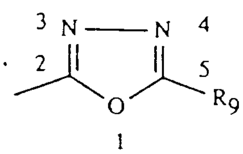 , 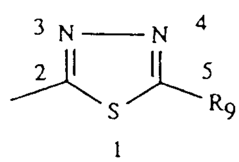 ,

f') 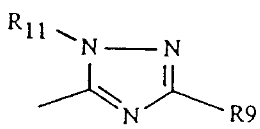 .

and the other substituents are as defined in claim 2.

**6.** A process for the preparation of a compound of formula I according to any one of claims 2 to 5, wherein $R_2$ is methyl or ethyl.

**7.** A process for the preparation of a compound of formula I according to claim 1, wherein $R_2$ and $R_3$ are each independently of the other methyl, ethyl or isopropyl, and X is as defined for formula I.

**8.** A process for the preparation of a compound of formula I according to claim 1, wherein
$R_2$ is methyl, ethyl, isopropyl or sec-butyl;
$R_1$ is the group

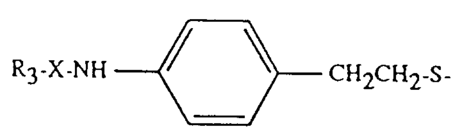

wherein X is a group selected from -C(O)-, -OC(O)-, -NHC(O)-, -NHC(S)- and $-SO_2-$; and
$R_3$ is $C_1$-$C_8$alkyl or $C_3$-$C_6$cycloalkyl.

**9.** A process for the preparation of a compound of formula I according to claim 1, wherein
$R_2$ is methyl, ethyl, isopropyl or sec-butyl:

$R_1$ is the group

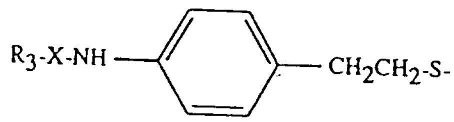

wherein X is a group selected from -C(O)-, -OC(O)-, -NHC(O)-, -NHC(S)- and $-SO_2-$; and
$R_3$ is the group

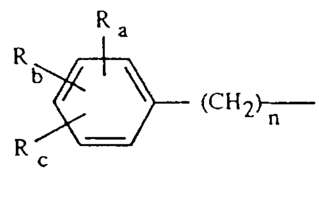

wherein
n is 0 or 1, and
$R_a$, $R_b$ and $R_c$ are each independently of one another hydrogen, $C_1$-$C_2$alkyl, $C_1$-$C_2$alkoxy, $C_1$-$C_2$alkylthio, $C_1$-$C_2$haloalkyl, $C_1$-$C_2$haloalkoxy, $C_1$-$C_2$haloalkylthio, halogen or $NO_2$, and $R_c$ may additionally be the group $Het_2$-O-, wherein $Het_2$ denotes the groups as indicated for formula I.

**10.** A process for the preparation of a compound of formula I according to claim 2,
wherein $R_2$ is methyl, ethyl, isopropyl or sec-butyl;
$R_1$ is the group

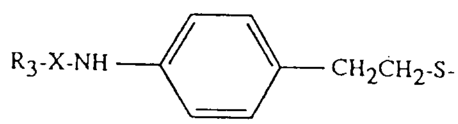

wherein X is a group selected from -C(O)-, -OC(O)- or -NHC(O)-; and
$R_3$ is $Het_1$, unsubstituted $C_1$-$C_6$alkyl or $C_1$-$C_6$alkyl which is substituted by $C_1$-$C_4$alkoxy, $C_1$-$C_4$alkylthio or halogen; or is unsubstituted $C_3$-$C_6$cycloalkyl or $C_3$-$C_6$cycloalkyl which is substituted by $C_1$-$C_2$alkoxy, $C_1$-$C_2$alkylthio or halogen; or is the group

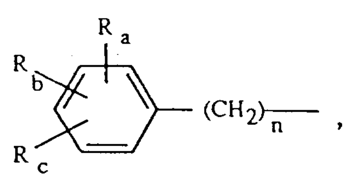

wherein n is 0 or 1;
$R_a$, $R_b$ and $R_c$ are each independently of one another hydrogen, fluoro, chloro, bromo, methyl, methoxy, methylthio, $CF_3$ or $NO_2$, and $R_c$ may additionally be the group

$Het_2$-O-

and $Het_1$ and $Het_2$ are one of the groups a') to f') defined for Het in claim 5, and the other substituents are as defined for claim 2.

**11.** A process for the preparation of a compound of formula I according to claim 1,
wherein
$R_2$ is methyl, ethyl, isopropyl or sec-butyl;

$R_1$ is the group

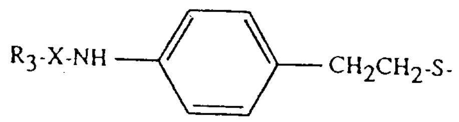

wherein X is -OC(O)-; and
$R_3$ is unsubstituted $C_1$-$C_6$alkyl or $C_1$-$C_6$alkyl which is substituted by $C_1$-$C_4$alkoxy, $C_1$-$C_4$alkylthio or halogen.

**12.** A process for the preparation of a compound of formula I according to claim 1,
wherein
$R_2$ is methyl, ethyl, isopropyl or sec-butyl;
$R_1$ is the group

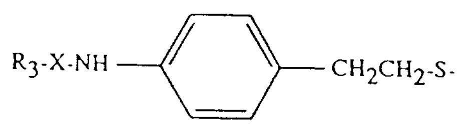

wherein X is -C(O)-, -OC(O)- or -NHC(O)-; and
$R_3$ is hydrogen; unsubstituted $C_1$-$C_8$alkyl or $C_1$-$C_8$alkyl which is substituted by $C_1$-$C_4$alkoxy, $C_1$-$C_4$alkylthio, $C_2$-$C_5$alkoxycarbonyl, halogen, CN, $NO_2$, COOH or $NH_2$;
or is unsubstituted $C_3$-$C_8$cycloalkyl or $C_3$-$C_8$cycloalkyl which is substituted by $C_1$-$C_4$alkoxy, $C_1$-$C_4$alkylthio, $C_2$-$C_5$alkoxycarbonyl, halogen, CN, $NO_2$, COOH or $NH_2$.

**13.** A process for the preparation of a compound of formula I according to claim 2,
wherein
$R_2$ is methyl, ethyl, isopropyl or sec-butyl;
$R_1$ is the group

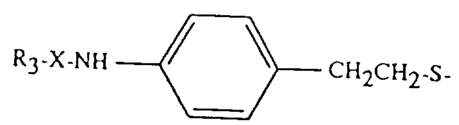

wherein X is -C(O)-, -OC(O)- or -NHC(O)-; and $R_3$ is the group

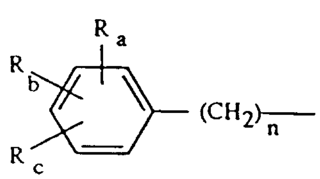,

wherein n is 0 or 1, and
$R_a$, $R_b$ and $R_c$ are each independently of one another hydrogen, $C_1$-$C_2$alkyl, $C_1$-$C_2$alkoxy, $C_1$-$C_2$alkylthio, $C_1$-$C_2$haloalkyl, $C_1$-$C_2$haloalkoxy, $C_1$-$C_2$haloalkylthio, halogen or $NO_2$, and
$R_c$ may additionally be the group

$Het_2$-O-,

wherein $Het_2$ is one of the groups a') to f') defined for Het in claim 5 and the other substituents are as defined in claim 2.

**14.** A process for the preparation of a compound of formula I according to claim 1, wherein
$R_2$ is methyl, ethyl, isopropyl or sec-butyl;
$R_1$ is the group

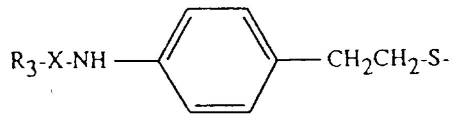

wherein X is -C(O)-, -OC(O)- or -NHC(O)-; and $R_3$ is the group

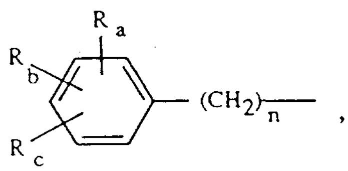

wherein n is 0 or 1, and
$R_a$, $R_b$ and $R_c$ are each independently of one another hydrogen, $C_1$-$C_2$alkyl, $C_1$-$C_2$alkoxy, $C_1$-$C_2$alkylthio, $C_1$-$C_2$haloalkyl, $C_1$-$C_2$haloalkoxy, $C_1$-$C_2$haloalkylthio, halogen or $NO_2$.

**15.** A process for the preparation of a compound of formula I according to claim 1, wherein
$R_2$ is methyl or ethyl;
$R_1$ is the group

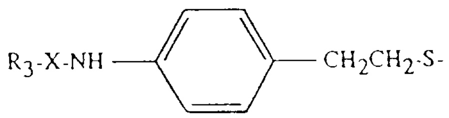

wherein X is -OC(O)-, and $R_3$ is $C_1$-$C_6$alkyl.

**16.** A process for the preparation of a compound of formula I according to claim 1, selected from the series:
13$\beta$-[2-(4-acetaminophenyl)ethylthio]milbemycin A4;
13$\beta$-(2-[4-(4-heptylcarbonylamino)phenyl]ethylthio)milbemycin A4;
13$\beta$-(2-[4-(2,6-dichloropyridin-4-ylcarbonylamino)phenyl]ethylthio)-milbemycin A4;
13$\beta$-(2-[4-tosylphenyl]ethylthio)milbemycin A4;
13$\beta$-(2-[4-(methylaminothiocarbonylamino)phenyl]ethylthio)milbemycin A4;
13$\beta$-(2-[4-(benzoylaminocarbonylamino)phenyl]ethylthio)milbemycin A4;
13$\beta$-(2-[4-(cyclcohexylaminocarbonylamino)phenyl]ethylthio)milbemycin A4;
13$\beta$-(2-[4-((4-methoxyphenyl)aminocarbonylamino)phenyl]ethylthio)milbemycin A4;
13$\beta$-(2-[4-(carboxymethylaminocarbonylamino)phenyl]ethylthio)milbemycin A4; and
13$\beta$-(2-[4-(methoxycarbonylamino)phenyl]ethylthio)milbemycin A4.

## Revendications

## Revendications pour les Etats contractants suivants : AT, BE, CH, DE, DK, FR, GB, IT, LI, LU, NL, SE

**1.** Composé de formule

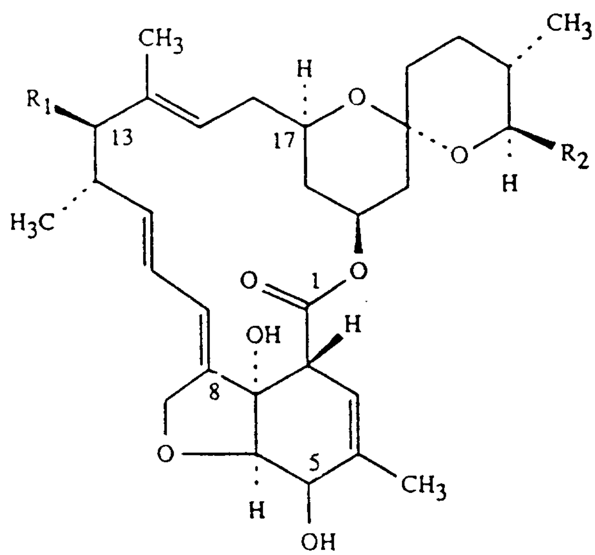

(I),

où

$R_2$ représente le méthyle, l'éthyle, l'isopropyle, le sec-butyle ou le groupe $-C(CH_3)=CHQ$,

Q représentant le méthyle, l'éthyle ou l'isopropyle;

et

$R_1$ représente le groupe

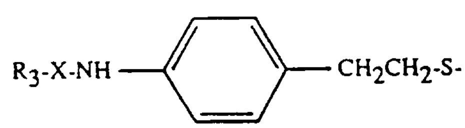

X représentant l'un des groupes -C(O)-, -OC(O)-, -NHC(O)-, -NHC(S)- ou $-SO_2-$;

et

$R_3$ représentant l'hydrogène, $Het_1$, un alkyle en $C_1$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$; un cycloalkyle en $C_3$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$ ou le groupe

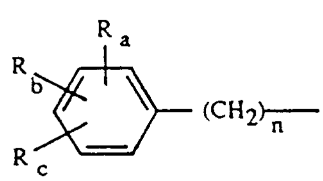

n étant égal à 0, 1 ou 2,

$R_a$, $R_b$ et $R_c$ représentant, indépendamment les uns des autres, l'hydrogène, un alkyle en $C_1$-$C_4$, un alcoxy en $C_1$-$C_4$, un alkyle en $C_1$-$C_4$ thio, un haloalkyle en $C_1$-$C_4$, un haloalcoxy en $C_1$-$C_4$, un haloalkyle en $C_1$-$C_4$ thio, un alcoxyalcoxy en $C_2$-$C_6$, un alcanoyle en $C_1$-$C_5$ oxy, un alcoxy en $C_1$-$C_5$ carbonyle, un halogène, CN, $NO_2$ ou $NH_2$, $R_c$ pouvant représenter en plus un cycloalkyle en $C_3$-$C_8$ ou le groupe $Het_2$-O-, $Het_1$ et $Het_2$ représentant un hétérocycle non substitué ou substitué, lié par l'intermédiaire du carbone, choisi dans le groupe constitué par le benzimidazole, le benzoxazole, le benzothiazole, l'imidazole, l'oxazole, le thiazole, l'oxadiazole, le thiadiazole, le triazole, la pyridine, la

pyridazine, la pyrimidine, la pyrazine, la triazine, la quinoléine, l'isoquinoléine, la quinoxaline, la phtalazine, la quinazoline et la benzotriazine; y compris ses sels d'addition avec des acides physiologiquement compatibles.

**2.** Composé de formule I selon la revendication 1 où
$R_2$ représente le méthyle, l'éthyle, l'isopropyle, le sec-butyle ou le groupe $-C(CH_3)=CHQ$,
Q représentant le méthyle, l'éthyle ou l'isopropyle; et $R_1$ représente le groupe

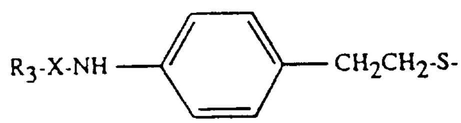

X représentant l'un des groupes -C(O)-, -OC(O)-, -NHC(O)-, -NHC(S)- ou $-SO_2-$;
et
$R_3$ représentant l'hydrogène, $Het_1$, un alkyle en $C_1$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$; un cycloalkyle en $C_3$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$ ou représentant le groupe

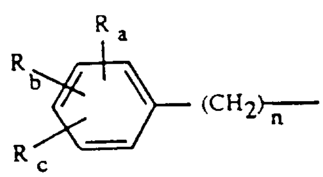

n étant égal à 0, 1 ou 2,
$R_a$, $R_b$ et $R_c$ représentant, indépendamment les uns des autres, l'hydrogène, un alkyle en $C_1$-$C_4$, un alcoxy en $C_1$-$C_4$, un alkyle en $C_1$-$C_4$ thio, un haloalkyle en $C_1$-$C_4$, un haloalcoxy en $C_1$-$C_4$, un haloalkyle en $C_1$-$C_4$ thio, un alcoxyalcoxy en $C_2$-$C_6$, un alcanoyle en $C_1$-$C_5$ oxy, un alcoxy en $C_1$-$C_5$ carbonyle, un halogène, CN, $NO_2$ ou $NH_2$, $R_c$ représentant en plus un cycloalkyle en $C_3$-$C_8$ ou le groupe

$Het_2$-O-

$Het_1$ et $Het_2$, étant ci-après rassemblés sous la désignation Het, représentant un groupe choisi parmi l'une des séries a à u :

| Groupe | Het | Type |
|---|---|---|
| a) | 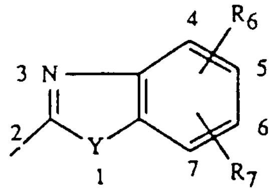 | benzimidazol-2-yle<br>benzoxazol-2-yle<br>benzothiazol-2-yle |
| b) | 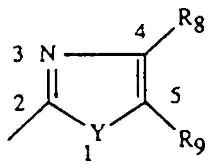 | imidazol-2-yle<br>oxazol-2-yle<br>thiazol-2-yle |
| c) | 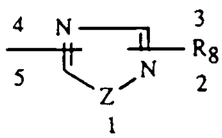 | [1H-1,2,4-triazol]-5-yle<br>[1,2,4-oxadiazol]-5-yle<br>[1,2,4-thiadiazol]-5-yle<br>[1,2,4-oxadiazol]-3-yle<br>[1,2,4-thiadiazol]-3-yle |
| d) | 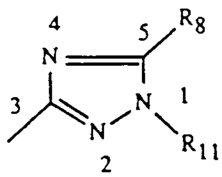 | [1H-1,2,4-triazol]-3-yle |
| e) | 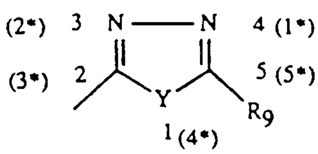 | [1,3,4-oxadiazol]-2-yle<br>[1,3,4-thiadiazol]-2-yle<br>* [4H-1,2,4-triazol]-3-yle |
| f) | 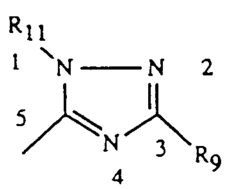 | [1H-1,2,4-triazol]-5-yle |
| g) | 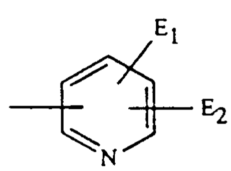 | pyridin-2-yle<br>pyridin-3-yle<br>pyridin-4-yle |

| Groupe | Het | Type |
|---|---|---|
| h) | 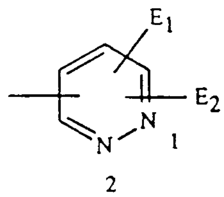 | pyridazin-3-yle<br>pyridazin-4-yle |
| i) | 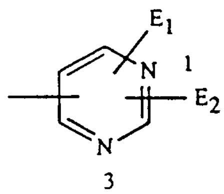 | pyrimidin-2-yle<br>pyrimidin-4-yle<br>pyrimidin-5-yle |
| k) | 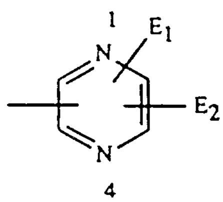 | pyrazin-2-yle |
| l) | 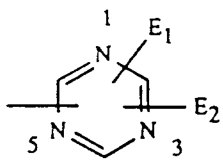 | s-triazin-2-yle |
| m) | 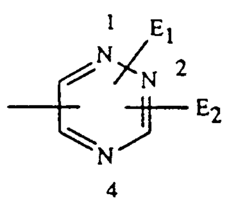 | [1,2,4-triazin]-3-yle<br>[1,2,4-triazin]-5-yle<br>[1,2,4-triazin]-6-yle |
| n) | 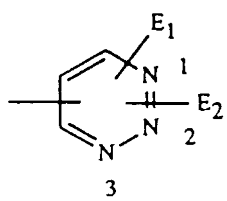 | [1,2,3-triazin]-4-yle<br>[1,2,3-triazin]-5-yle |
| o) | 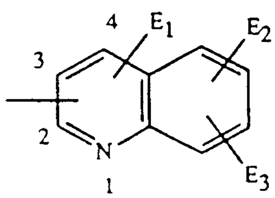 | quinoléin-2-yle<br>quinoléin-3-yle<br>quinoléin-4-yle |

| Groupe | Het | Type |
| --- | --- | --- |
| p) | 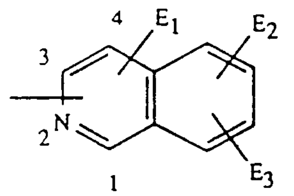 | isoquinoléin-1-yle<br>isoquinoléin-3-yle<br>isoquinoléin-4-yle |
| q) | 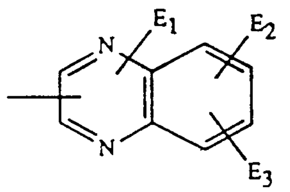 | quinoxalin-2-yle |
| r) | 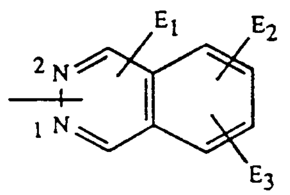 | phtalazin-3-yle |
| s) | 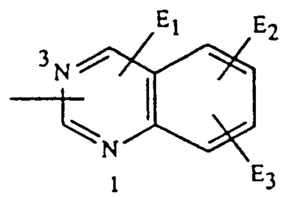 | quinazolin-2-yle<br>quinazolin-4-yle |
| t) | 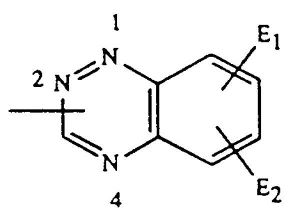 | [1,2,4-benzotriazin]-3-yle |
| u) | 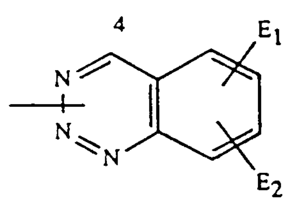 | [1,2,3-benzotriazin]-4-yle |

où

Y représente l'oxygène, le soufre ou $NR_{10}$;

Z représente l'oxygène, le soufre ou NH;

$R_6$ et $R_7$ représentent, indépendamment l'un de l'autre, l'hydrogène, un alkyle en $C_1$-$C_4$, un haloalkyle en $C_1$-$C_4$, un alcoxy en $C_1$-$C_4$, un haloalcoxy en $C_1$-$C_4$, un alkyle en $C_1$-$C_4$ thio, un alkyle en $C_1$-$C_4$ sulfonyle, un alkyle en $C_1$-$C_4$ sulfinyle, un haloalkyle en $C_1$-$C_4$ sulfonyle, un haloalkyle en $C_1$-$C_4$ sulfinyle, un halogène, le nitro ou le cyano;

$R_8$ et $R_9$ représentent, indépendamment l'un de l'autre, l'hydrogène, un alkyle en $C_1$-$C_6$, un haloalkyle

en $C_1$-$C_6$, un alcoxy en $C_1$-$C_6$, un alkyle en $C_1$-$C_6$ thio, un halogène ou le nitro; ou indépendamment l'un de l'autre, un cycloalkyle en $C_3$-$C_7$ non substitué ou substitué par un halogène ou par un alkyle en $C_1$-$C_3$;
$R_{10}$ représente l'hydrogène ou un alkyle en $C_1$-$C_6$;
$R_{11}$ représente un alkyle en $C_1$-$C_6$;
et
$E_1$, $E_2$ et $E_3$, représentent, indépendamment les uns des autres, l'hydrogène, un alkyle en $C_1$-$C_4$, un haloalkyle en $C_1$-$C_4$, un alcoxy en $C_1$-$C_4$, un haloalcoxy en $C_1$-$C_4$, un alkyle en $C_1$-$C_4$ thio, un haloalkyle en $C_1$-$C_4$ thio, un alcoxyalcoxy en $C_2$-$C_6$, un alcanoyle en $C_1$-$C_5$ oxy, un alcoxy en $C_1$-$C_5$ carbonyle, un cycloalkyle en $C_3$-$C_8$, un halogène, CN, $NO_2$ ou $NH_2$.

**3.** Composé de formule I selon la revendication 2, caractérisé en ce que $E_1$, $E_2$ et $E_3$ représentent, indépendamment les uns des autres, l'hydrogène, le méthyle, l'éthyle, $CF_3$, le méthoxy, $OCF_3$, le méthylthio, $CH_3OCH_2O$, le cyclopropyle, le fluor, le chlore ou le brome et en ce que les autres substituants sont définis comme dans la revendication 2.

**4.** Composé de formule I selon la revendication 2, caractérisé en ce que dans les groupes b à f $R_8$ et $R_9$ représentent, indépendamment l'un de l'autre, un cycloalkyle en $C_3$-$C_7$ non substitué ou substitué par un halogène ou par un alkyle en $C_1$-$C_3$ et les autres substituants, ainsi que Y et Z sont définis comme dans la revendication 2.

5. Composé de formule I selon la revendication 2, caractérisé en ce que le substituant Het représente

a')

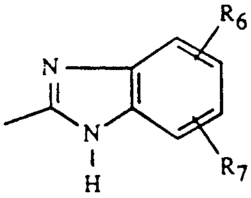 , 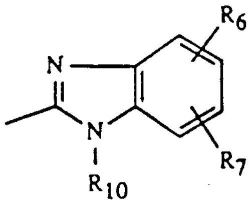 , 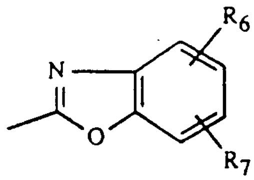 , 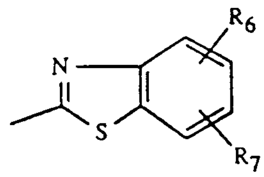 ,

b') 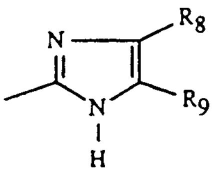 , 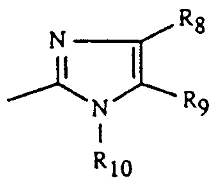 , 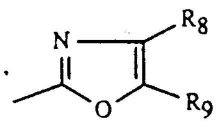 , 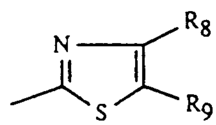 ,

c') 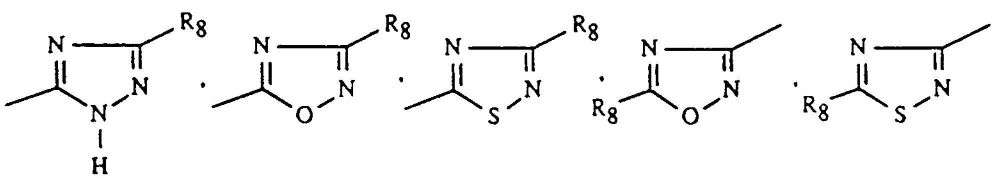

d') 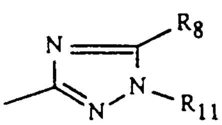

e') 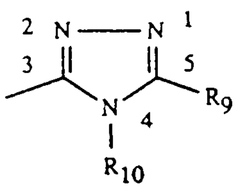 , 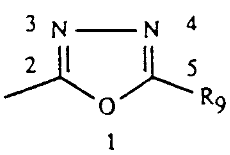 , 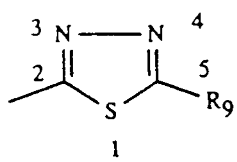 ,

f') 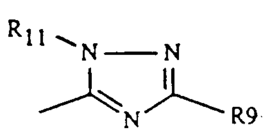 ,

et les autres substituants sont comme définis dans la revendication 2.

6. Composé de formule I selon l'une des revendications 2 à 5, caractérisé en ce que $R_2$ représente le méthyle ou l'éthyle.

7. Composé de formule I selon la revendication 1, caractérisé en ce que $R_2$ et $R_3$ représentent, indépendamment l'un de l'autre, le méthyle, l'éthyle ou l'isopropyle et X a les significations données pour la formule I.

8. Composé de formule I selon la revendication 1, caractérisé en ce que $R_2$ représente le méthyle, l'éthyle, l'isopropyle ou le sec-butyle;
$R_1$ représente le groupe

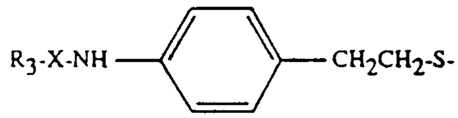

X représentant l'un des groupes -C(O)-, -OC(O)-, -NHC(O)-, -NHC(S)- ou $-SO_2-$;
et
$R_3$ représentant un alkyle en $C_1$-$C_8$ ou un cycloalkyle en $C_3$-$C_6$.

**9.** Composé de formule I selon la revendication 1, caractérisé en ce que $R_2$ représente le méthyle, l'éthyle, l'isopropyle ou le sec-butyle;
$R_1$ représente le groupe

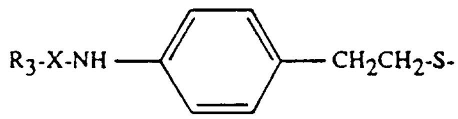

X représentant l'un des groupes -C(O)-, -OC(O)-, -NHC(O)-, -NHC(S)- ou $-SO_2-$;
et
$R_3$ représentant le groupe

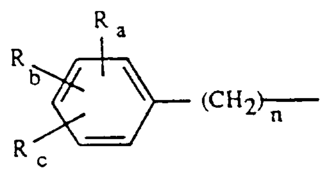

n étant égal à 0 ou 1 et
$R_a$, $R_b$ et $R_c$ représentant, indépendamment les uns des autres, l'hydrogène, un alkyle en $C_1$-$C_2$, un alcoxy en $C_1$-$C_2$, un alkyle en $C_1$-$C_2$ thio, un haloalkyle en $C_1$-$C_2$, un haloalcoxy en $C_1$-$C_2$, un haloalkyle en $C_1$-$C_2$ thio, un halogène ou $NO_2$ et $R_c$ pouvant représenter en plus le groupe $Het_2$-O, $Het_2$ représentant les groupes données pour la formule I.

**10.** Composé de formule I selon la revendication 2, caractérisé en ce que $R_2$ représente le méthyle, l'éthyle, l'isopropyle ou le sec-butyle:
$R_1$ représente le groupe

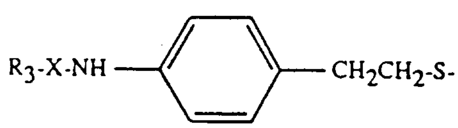

X représentant l'un des groupes -C(O)-, -OC(O)-, ou -NHC(O)-; et
$R_3$ représentant $Het_1$, un alkyle en $C_1$-$C_6$ non substitué ou substitué par un alcoxy en $C_1$-$C_4$, un alkyle en $C_1$-$C_4$ thio ou un halogène; un cycloalkyle en $C_3$-$C_6$ non substitué ou substitué par un alcoxy en $C_1$-$C_2$, un alkyle en $C_1$-$C_2$ thio, ou un halogène ou le groupe

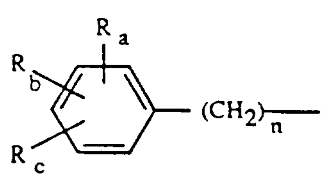

n étant égal à 0 ou 1,
$R_a$, $R_b$ et $R_c$ représentant, indépendamment les uns des autres, l'hydrogène, le fluor, le chlore, le brome, le méthyle, le méthoxy, le méthylthio, $CF_3$ ou $NO_2$, $R_c$ pouvant représenter en plus le groupe

$Het_2$-O-

$Het_1$ et $Het_2$ représentant l'un des groupes a') à f') définis dans la revendication 5 pour Het et les autres substituants étant définis comme dans la revendication 2.

**11.** Composé de formule I selon la revendication 1, caractérisé en ce que $R_2$ représente le méthyle, l'éthyle, l'isopropyle ou le sec-butyle;
$R_1$ représente le groupe

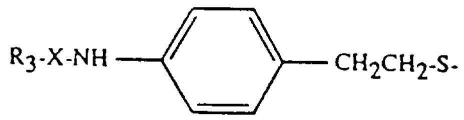

X représentant -OC(O)-; et
$R_3$ représentant un alkyle en $C_1$-$C_6$ non substitué ou substitué par un alcoxy en $C_1$-$C_4$, un alkyle en $C_1$-$C_4$ thio ou un halogène.

**12.** Composé de formule I selon la revendication 1, caractérisé en ce que $R_2$ représente le méthyle, l'éthyle, l'isopropyle ou le sec-butyle;
$R_1$ représente le groupe

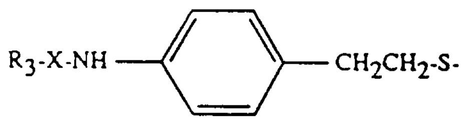

X représentant -C(O)-, -OC(O)- ou -NHC(O)-; et
$R_3$ représentant l'hydrogène; un alkyle en $C_1$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$; un cycloalkyle en $C_3$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$.

**13.** Composé de formule I selon la revendication 2, caractérisé en ce que $R_2$ représente le méthyle, l'éthyle, l'isopropyle ou le sec-butyle;
$R_1$ représente le groupe

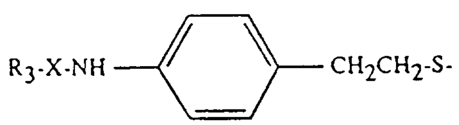

X représentant -C(O)-, -OC(O)- ou -NHC(O)-; et
$R_3$ représentant le groupe

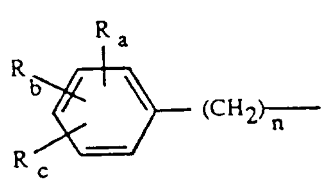

n étant égal à 0 ou 1 et
$R_a$, $R_b$ et $R_c$ représentant, indépendamment les uns des autres, l'hydrogène, un alkyle en $C_1$-$C_2$, un alcoxy en $C_1$-$C_2$, un alkyle en $C_1$-$C_2$ thio, un haloalkyle en $C_1$-$C_2$, un haloalcoxy en $C_1$-$C_2$, un haloalkyle en $C_1$-$C_2$ thio, un halogène ou $NO_2$ et
$R_c$ représentant en plus le groupe

$Het_2$-O-

$Het_2$ représentant l'un des groupes a') à f') définis dans la revendication 5 pour Het et les autres substituants étant définis comme dans la revendication 2.

**14.** Composé de formule I selon la revendication 1, caractérisé en ce que $R_2$ représente le méthyle, l'éthyle, l'isopropyle ou le sec-butyle;
$R_1$ représente le groupe

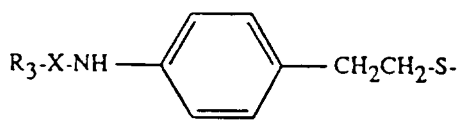

X représentant -C(O)-, -OC(O)- ou -NHC(O)-; et
$R_3$ représentant le groupe

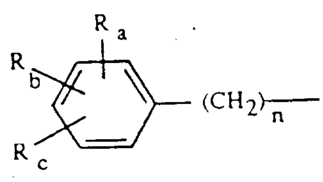

n étant égal à 0 ou 1 et
$R_a$, $R_b$ et $R_c$ représentant, indépendamment les uns des autres, l'hydrogène, un alkyle en $C_1$-$C_2$, un alcoxy en $C_1$-$C_2$, un alkyle en $C_1$-$C_2$ thio, un haloalkyle en $C_1$-$C_2$, un haloalcoxy en $C_1$-$C_2$, un haloalkyle en $C_1$-$C_2$ thio, un halogène ou $NO_2$.

**15.** Composé de formule I selon la revendication 1, caractérisé en ce que $R_2$ représente le méthyle ou l'éthyle;
$R_1$ représente le groupe

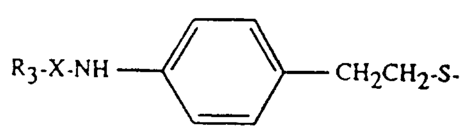

X représentant -OC(O)- et $R_3$ un alkyle en $C_1$-$C_6$.

**16.** Un composé de formule I selon la revendication 1, choisi dans le groupe
la 13$\beta$-[2-(4-acétaminophényl)éthylthio]milbémycine A4;
la 13$\beta$-(2-[4-(4-heptylcarbonylamino)phényl]éthylthio)milbémycine A4;
la 13$\beta$-(2-[4-(2,6-dichloropyridin-4-ylcarbonylamino)phényl]éthylthio)milbémycine A4;
la 13$\beta$-(2-[4-tosylphényl]éthylthio)milbémycine A4;
la 13$\beta$-(2-[4-(méthylaminothiocarbonylamino)phényl]éthylthio)milbémycine A4;
la 13$\beta$-(2-[4-(benzoylaminocarbonylamino)phényl]éthylthio)milbémycine A4;
la 13$\beta$-(2-[4-(cyclohexylaminocarbonylamino)phényl]éthylthio)milbémycine A4;
la 13$\beta$-(2-[4-((4-méthoxyphényl)aminocarbonylamino)phényl]éthylthio)milbémycine A4;
la 13$\beta$-(2-[4-(carboxyméthylaminocarbonylamino)phényl]éthylthio)milbémycine A4; et

la 13β-(2-[4-(méthoxycarbonylamino)phényl]éthylthio)milbémycine A4.

**17.** Procédé pour la préparation d'un composé de formule I selon la revendication 1, caractérisé en ce que l'on fait réagir un composé de formule II

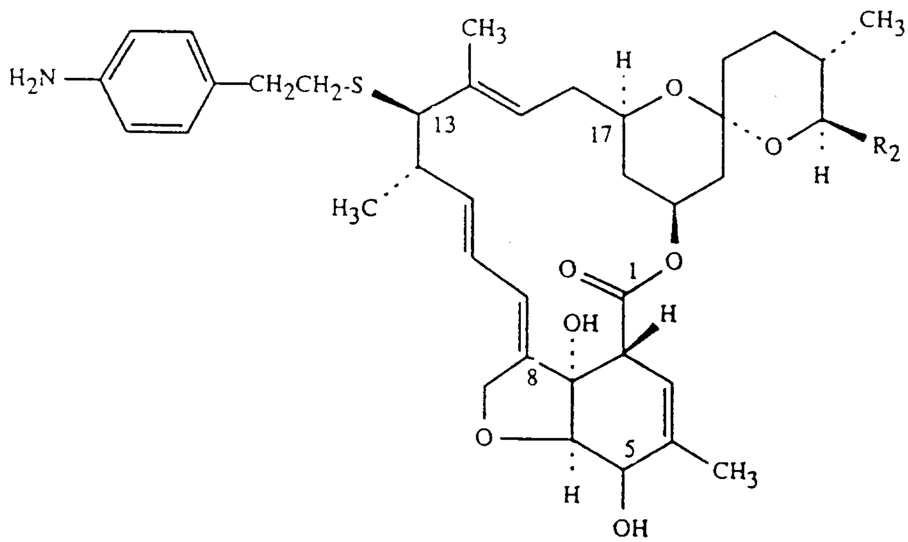

(II),

où $R_2$ a les significations données pour la formule I,

α) dans le cas où dans la formule I X représente -C(O)-ou -OC(O)-, avec un composé de formule III

$R_3$-X-T (III)

en l'absence ou, de préférence, en présence d'un agent neutralisant,

$R_3$ ayant les significations données pour la formule I, X représentant -C(O)- ou -OC(O)- et T un groupe partant;

β) dans le cas où dans la formule I X représente -NH-C(O)-, avec un isocyanate de formule IV

$R_3$-N = C = O (IV)

ou avec un agent d'aminoacylation de formule V

$R_3$-NH-C(O)-D (V)

$R_3$ ayant les significations données pour la formule I et D représentant un groupe partant cité pour T ou, de préférence, le phénoxy non substitué ou substitué;

γ) dans le cas où dans la formule I X représente -NH-C(S)-, avec un isothiocyanate de formule VI

$R_3$-N = C = S (VI)

$R_3$ ayant les significatios données pour la formule I et

δ) dans le cas où dans la formule I X représente $SO_2$, avec un agent de sulfonation de formule VIa

$R_3$-$SO_2$-T (VIa)

en l'absence ou, de préférence, en présence d'un agent neutralisant, $R_3$ ayant les significations données pour la formule I et T étant défini comme sous ).

**18.** Composition insecticide et parasiticide, caractérisée en ce qu'elle contient, à titre de substance active, un composé de formule I selon l'une des revendications 1 à 16.

**19.** Composés de formule I selon l'une des revendications 1 à 16, destinés à être utilisés pour lutter contre les parasites sur ou dans les animaux.

**20.** Composés de formule I selon la revendication 19, destinés à être utilisés pour lutter contre les endo- et ectoparasites chez l'animal utile.

**21.** Utilisation d'un composé de formule I selon l'une des revendications 1 à 16 pour la préparation d'un médicament pour lutter contre les parasites sur ou dans les animaux.

**Revendications pour les Etats contractants suivants : ES, GR**

**1.** Procédé pour la préparation d'un composé de formule I

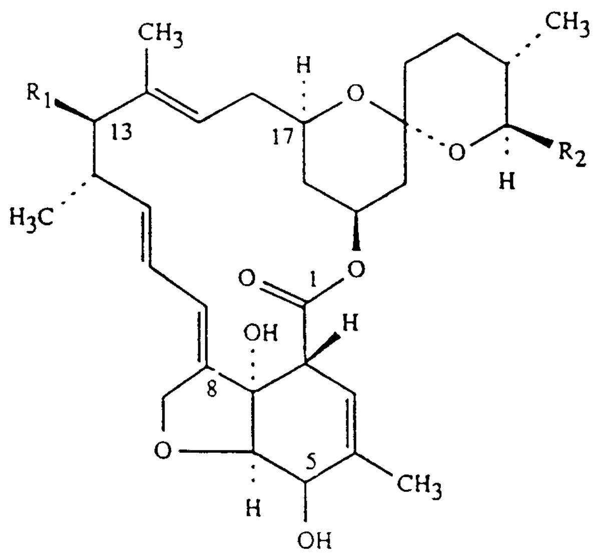

(I),

où

$R_2$ représente le méthyle, l'éthyle, l'isopropyle, le sec-butyle ou le groupe $-C(CH_3)=CHQ$,

Q représentant le méthyle, l'éthyle ou l'isopropyle;

et

$R_1$ représente le groupe

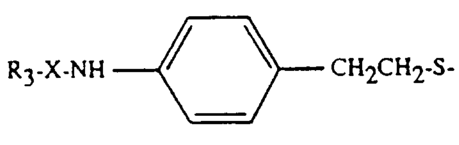

X représentant l'un des groupes -C(O)-, -OC(O)-, -NHC(O)-, -NHC(S)- ou $-SO_2-$;

et

$R_3$ représentant l'hydrogène, $Het_1$, un alkyle en $C_1$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$; un cycloalkyle en $C_3$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$ ou le groupe

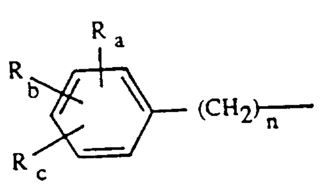

n étant égal à 0, 1 ou 2,
$R_a$, $R_b$ et $R_c$ représentant, indépendamment les uns des autres, l'hydrogène, un alkyle en $C_1$-$C_4$, un alcoxy en $C_1$-$C_4$, un alkyle en $C_1$-$C_4$ thio, un haloalkyle en $C_1$-$C_4$, un haloalcoxy en $C_1$-$C_4$, un haloalkyle en $C_1$-$C_4$ thio, un alcoxyalcoxy en $C_2$-$C_6$, un alcanoyle en $C_1$-$C_5$ oxy, un alcoxy en $C_1$-$C_5$ carbonyle, un halogène, CN, $NO_2$ ou $NH_2$, $R_c$ pouvant représenter en plus un cycloalkyle en $C_3$-$C_8$ ou le groupe $Het_2$-O-, $Het_1$ et $Het_2$ représentant un hétérocycle non substitué ou substitué, lié par l'intermédiaire du carbone, choisi dans le groupe constitué par le benzimidazole, le benzoxazole, le benzothiazole, l'imidazole, l'oxazole, le thiazole, l'oxadiazole, le thiadiazole, le triazole, la pyridine, la pyridazine, la pyrimidine, la pyrazine, la triazine, la quinoléine, l'isoquinoléine, la quinoxaline, la phtalazine, la quinazoline et la benzotriazine; y compris ses sels d'addition avec des acides physiologiquement compatibles, caractérisé en ce que l'on fait réagir un composé de formule II

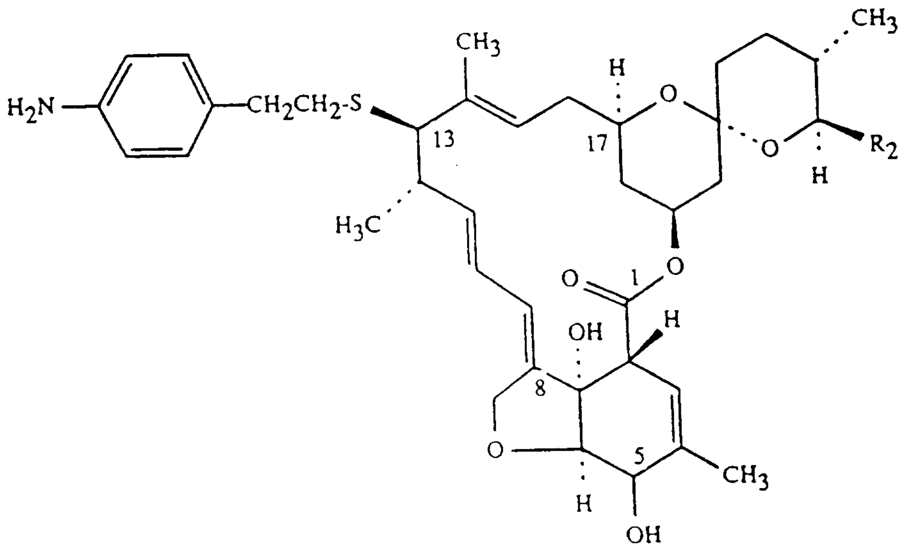
(II),

où $R_2$ a les significations données pour la formule I
α) dans le cas où dans la formule I X représente -C(O)-ou -OC(O)-, avec un composé de formule III

$R_3$-X-T (III)

en l'absence ou, de préférence, en présence d'un agent neutralisant,
$R_3$ ayant les significations données pour la formule I, X représentant -C(O)- ou -OC(O)- et T un groupe partant;
β) dans le cas où dans la formule I X représente -NH-C(O)-, avec un isocyanate de formule IV

$R_3$-N = C = O (IV)

ou avec un agent d'aminoacylation de formule V

$R_3$-NH-C(O)-D (V)

$R_3$ ayant les significations données pour la formule I et D représentant un groupe partant cité pour T ou, de préférence, le phénoxy non substitué ou substitué;
γ) dans le cas où dans la formule I X représente -NH-C(S)-, avec un isothiocyanate de formule VI

$R_3$-N = C = S (VI)

$R_3$ ayant les significations données pour la formule I et
δ) dans le cas où dans la formule I X représente $SO_2$, avec un agent de sulfonation de formule VIa

$R_3$-$SO_2$-T (VIa)

en l'absence ou, de préférence, en présence d'un agent neutralisant, $R_3$ ayant les significations données pour la formule I et T étant défini comme sous $\alpha$).

**2.** Procédé pour la préparation d'un composé de formule I selon la revendication 1, dans laquelle $R_2$ représente le méthyle, l'éthyle, l'isopropyle, le sec-butyle ou le groupe $-C(CH_3)=CHQ$,
Q représentant le méthyle, l'éthyle ou l'isopropyle;
et
$R_1$ représente le groupe

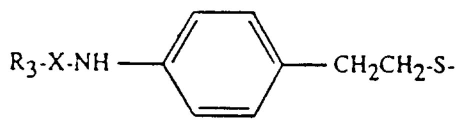

X représentant l'un des groupes -C(O)-, -OC(O)-, -NHC(O)-, -NHC(S)- ou $-SO_2-$;
et
$R_3$ représentant l'hydrogène, $Het_1$, un alkyle en $C_1$-$C_8$ non substitué ou substitué par un alcoxy en $C_1$-$C_4$, un alkyle en $C_1$-$C_4$ thio, un alcoxy en $C_1$-$C_5$ carbonyle, un halogène, CN, $NO_2$, COOH ou $NH_2$; un cycloalkyle en $C_3$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$ ou représentant le groupe

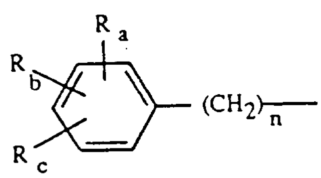

n étant égal à 0, 1 ou 2,
$R_a$, $R_b$ et $R_c$ représentant, indépendamment les uns des autres, l'hydrogène, un alkyle en $C_1$-$C_4$, un alcoxy en $C_1$-$C_4$, un alkyle en $C_1$-$C_4$ thio, un haloalkyle en $C_1$-$C_4$ , un haloalcoxy en $C_1$-$C_4$, un haloalkyle en $C_1$-$C_4$ thio, un alcoxyalcoxy en $C_2$-$C_6$, un alcanoyle en $C_1$-$C_5$ oxy, un alcoxy en $C_1$-$C_5$ carbonyle, un halogène, CN, $NO_2$ ou $NH_2$, $R_c$ représentant en plus un cycloalkyle en $C_3$-$C_8$ ou le groupe

$Het_2$-O-

$Het_1$ et $Het_2$, étant ci-après rassemblés sous la désignation Het représentant un groupe choisi parmi l'une des séries a à u :

| Groupe | Het | Type |
|---|---|---|
| a) | 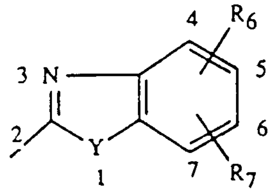 | benzimidazole-2-yle<br>benzoxazol-2-yle<br>benzothiazol-2-yle |
| b) | 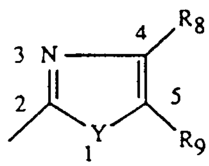 | imidazol-2-yle<br>oxazol-2-yle<br>thiazol-2-yle |
| c) | 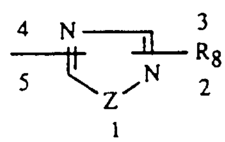 | [1H,1,2,4-triazol]-5-yle<br>[1,2,4-oxadiazol]-5-yle<br>[1,2,4-thiadiazol]-5-yle<br>[1,2,4-oxadiazol]-3-yle<br>[1,2,4-thiadiazol]-3-yle |
| d) | 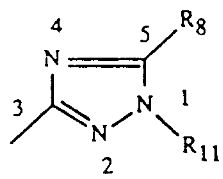 | [1H-1,2,4-triazol]-3-yle |
| e) | 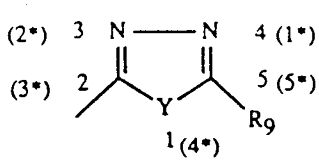 | [1,3,4-oxadiazol]-2-yle<br>[1,3,4-thiadiazol]-2-yle<br>* [4H-1,2,4-triazol]-3-yle |
| f) | 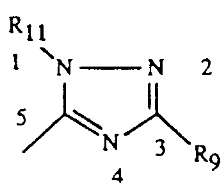 | [1H-1,2,4-triazol]-5-yle |
| g) | 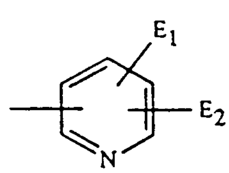 | pyridin-2-yle<br>pyridin-3-yle<br>pyridin-4-yle |

| Groupe | Het | Type |
|---|---|---|
| h) | 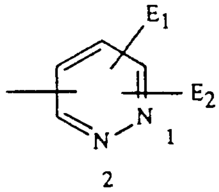 | pyridazin-3-yle<br>pyridazin-4-yle |
| i) | 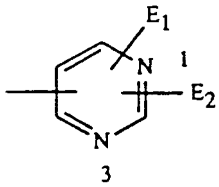 | pyrimidin-2-yle<br>pyrimidin-4-yle<br>pyrimidin-5-yle |
| k) | 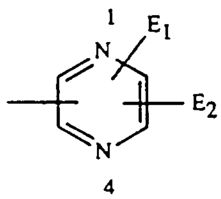 | pyrazin-2-yle |
| l) | 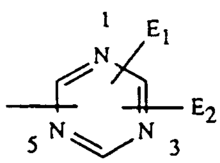 | s-triazin-2-yle |
| m) | 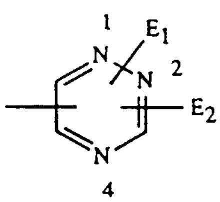 | [1,2,4-triazin]-3-yle<br>[1,2,4-triazin]-5-yle<br>[1,2,4-triazin]-6-yle |
| n) | 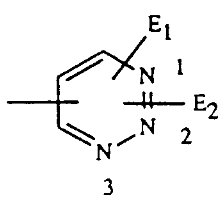 | [1,2,3-triazin]-4-yle<br>[1,2,3-triazin]-5-yle |
| o) | 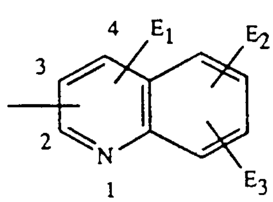 | quinoléin-2-yle<br>quinoléin-3-yle<br>quinoléin-4-yle |

| Groupe | Het | Type |
|---|---|---|
| p) | 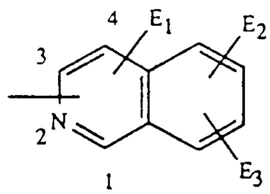 | isoquinoléin-1-yle<br>isoquinoléin-3-yle<br>isoquinoléin-4-yle |
| q) | 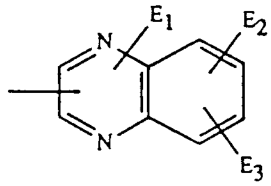 | quinoxalin-2-yle |
| r) | 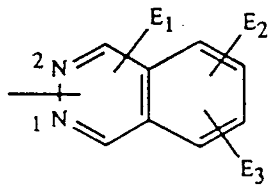 | phtalazin-3-yle |
| s) | 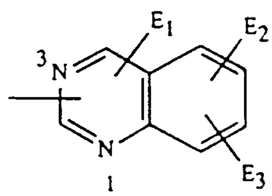 | quinazolin-2-yle<br>quinozolin-4-yle |
| t) | 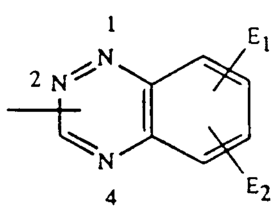 | [1,2,4-benzotriazin]-3-yle |
| u) | 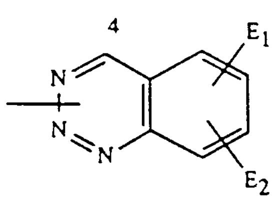 | [1,2,3-benzotriazin]-4-yle |

où

Y représente l'oxygène, le soufre ou $NR_{10}$;

Z représente l'oxygène, le soufre ou NH;

$R_6$ et $R_7$ représentent, indépendamment l'un de l'autre, l'hydrogène, un alkyle en $C_1$-$C_4$, un haloalkyle en $C_1$-$C_4$, un alcoxy en $C_1$-$C_4$, un haloalcoxy en $C_1$-$C_4$, un alkyle en $C_1$-$C_4$ thio, un alkyle en $C_1$-$C_4$ sulfonyle, un alkyle en $C_1$-$C_4$ sulfinyle, un haloalkyle en $C_1$-$C_4$ sulfonyle, un haloalkyle en $C_1$-$C_4$ sulfinyle, un halogène, le nitro ou le cyano;

$R_8$ et $R_9$ représentent, indépendamment l'un de l'autre, l'hydrogène, un alkyle en $C_1$-$C_6$, un haloalkyle en $C_1$-$C_6$, un alcoxy en $C_1$-$C_6$, un alkyle en $C_1$-$C_6$ thio, un halogène ou le nitro; ou indépendamment

l'un de l'autre un cycloalkyle en $C_3$-$C_7$ non substitué ou substitué par un halogène ou par un alkyle en $C_1$-$C_3$;
$R_{10}$ représente l'hydrogène ou un alkyle en $C_1$-$C_6$;
$R_{11}$ représente un alkyle en $C_1$-$C_6$;
et
$E_1$, $E_2$ et $E_3$ , représentent, indépendamment les uns des autres, l'hydrogène, un alkyle en $C_1$-$C_4$, un haloalkyle en $C_1$-$C_4$, un alcoxy en $C_1$-$C_4$, un haloalcoxy en $C_1$-$C_4$, un alkyle en $C_1$-$C_4$ thio, un haloalkyle en $C_1$-$C_4$ thio, un alcoxyalcoxy en $C_2$-$C_6$, un alcanoyle en $C_1$-$C_5$ oxy, un alcoxy en $C_1$-$C_5$ carbonyle, un cycloalkyle en $C_3$-$C_8$, un halogène, CN, $NO_2$ ou $NH_2$.

**3.** Procédé pour la préparation d'un composé de formule I selon la revendication 2, où $E_1$, $E_2$ et $E_3$ représentent, indépendamment les uns des autres, l'hydrogène, le méthyle, l'éthyle, $CF_3$, le méthoxy, $OCF_3$, le méthylthio, $CH_3OCH_2O$, le cyclopropyle, le fluor, le chlore ou le brome et les autres substituants sont définis comme dans la revendication 2.

**4.** Procédé pour la préparation d'un composé de formule I selon la revendication 2, dans lequel dans les groupes b à f $R_8$ et $R_9$ représentent, indépendamment l'un de l'autre, un cycloalkyle en $C_3$-$C_7$ non substitué ou substitué par un halogène ou par un alkyle en $C_1$-$C_3$ et les autres substituants, ainsi que Y et Z sont définis comme dans la revendication 2.

**5.** Procédé pour la préparation d'un composé de formule I selon la revendication 2, dans lequel le substituant Het représente

a') 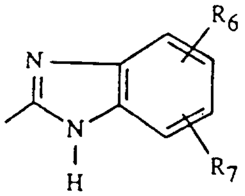 , 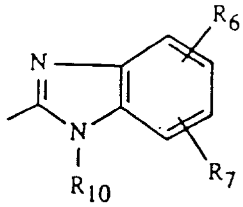 , 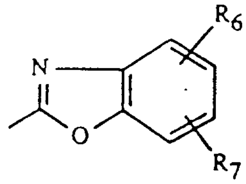 , 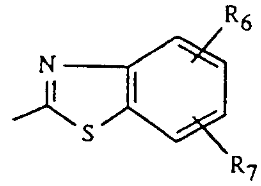

b') 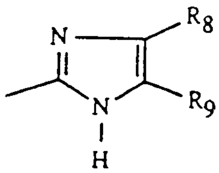 , 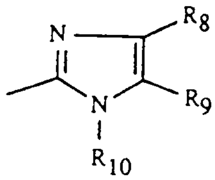 , 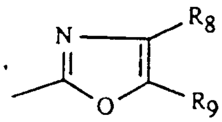 , 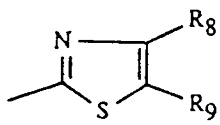 .

c') 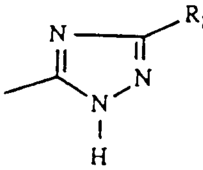 , 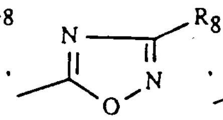 , 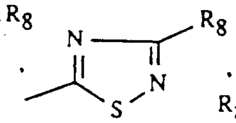 , 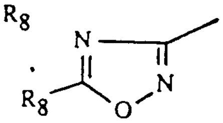 , 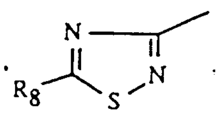 .

d') 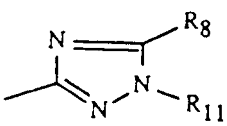 .

e') 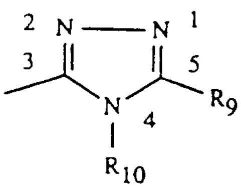 , 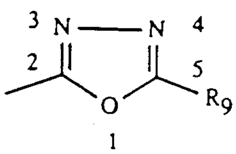 , 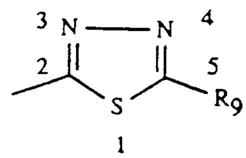 .

f') 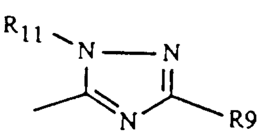 .

et les autres substituants sont définis comme dans la revendication 2.

6. Procédé pour la préparation d'un composé de formule I selon l'une des revendications 2 à 5, dans laquelle $R_2$ représente le méthyle ou l'éthyle.

7. Procédé pour la préparation d'un composé de formule I selon la revendication 1, dans laquelle $R_2$ et $R_3$ représentent, indépendamment l'un de l'autre, le méthyle, l'éthyle ou l'isopropyle et X a les significations données pour la formule I.

8. Procédé pour la préparation d'un composé de formule I selon la revendication 1, dans laquelle $R_2$ représente le méthyle, l'éthyle, l'isopropyle ou le sec-butyle ;
$R_1$ représente le groupe

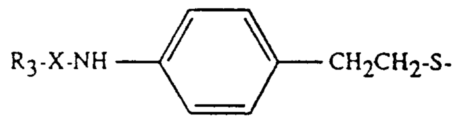

X représentant l'un des groupes -C(O)-, -OC(O)-, -NHC(O)-, -NHC(S)- ou $-SO_2-$;
et
$R_3$ représentant un alkyle en $C_1$-$C_8$ ou un cycloalkyle en $C_3$-$C_6$.

**9.** Procédé pour la préparation d'un composé de formule I selon la revendication 1, dans laquelle $R_2$ représente le méthyle, l'éthyle, l'isopropyle ou le sec-butyle;
$R_1$ représente le groupe

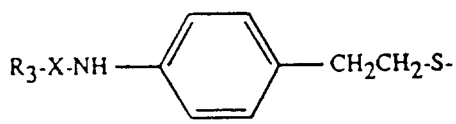

X représentant l'un des groupes -C(O)-, -OC(O)-, -NHC(O)-, -NHC(S)- ou $-SO_2-$;
et
$R_3$ représentant le groupe

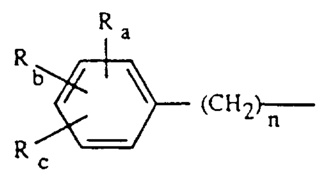

n étant égal à 0 ou 1 et
$R_a$, $R_b$ et $R_c$ représentant, indépendamment les uns des autres, l'hydrogène, un alkyle en $C_1$-$C_2$, un alcoxy en $C_1$-$C_2$, un alkyle en $C_1$-$C_2$ thio, un haloalkyle en $C_1$-$C_2$, un haloalcoxy en $C_1$-$C_2$, un haloalkyle en $C_1$-$C_2$ thio, un halogène ou $NO_2$ et $R_c$ pouvant représenter en plus le groupe $Het_2$-O, $Het_2$ représentant les groupes données pour la formule I.

**10.** Procédé pour la préparation d'un composé de formule I selon la revendication 2, dans laquelle $R_2$ représente le méthyle, l'éthyle, l'isopropyle ou le sec-butyle;
$R_1$ représente le groupe

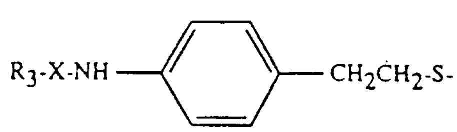

X représentant l'un des groupes -C(O)-, -OC(O)- ou -NHC(O)-; et
$R_3$ représentant $Het_1$, un alkyle en $C_1$-$C_6$ non substitué ou substitué par un alcoxy en $C_1$-$C_4$, un alkyle en $C_1$-$C_4$ thio ou un halogène; un cycloalkyle en $C_3$-$C_6$ non substitué ou substitué par un alcoxy en $C_1$-$C_2$, un alkyle en $C_1$-$C_2$ thio, ou un halogène ou le groupe

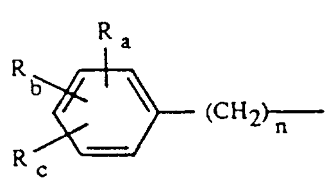

n étant égal à 0 ou 1;
$R_a$, $R_b$ et $R_c$ représentant, indépendamment les uns des autres, l'hydrogène, le fluor, le chlore, le brome, le méthyle, le méthoxy, le méthylthio, $CF_3$ ou $NO_2$, $R_c$ pouvant représenter en plus le groupe

$Het_2$-O-

$Het_1$ et $Het_2$ représentant l'un des groupes a') à f') définis dans la revendication 5 pour Het et les autres substituants étant définis comme dans la revendication 2.

**11.** Procédé pour la préparation d'un composé de formule I selon la revendication 1, dans laquelle $R_2$ représente le méthyle, l'éthyle, l'isopropyle ou le sec-butyle;
$R_1$ représente le groupe

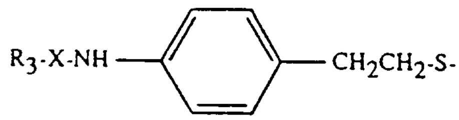

X représentant -OC(O)-; et
$R_3$ représentant un alkyle en $C_1$-$C_6$ non substitué ou substitué par un alcoxy en $C_1$-$C_4$, un alkyle en $C_1$-$C_4$ thio ou un halogène.

**12.** Procédé pour la préparation d'un composé de formule I selon la revendication 1, dans laquelle $R_2$ représente le méthyle, l'éthyle, l'isopropyle ou le sec-butyle;
$R_1$ représente le groupe

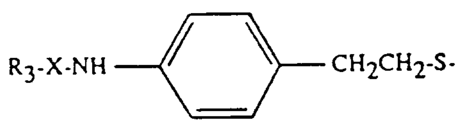

X représentant -C(O)-, -OC(O)- ou -NHC(O)-; et
$R_3$ représentant l'hydrogène; un alkyle en $C_1$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$; un cycloalkyle en $C_3$-$C_8$ non substitué ou substitué par un alcoxy en $C_1$-$C_4$, un alkyle en $C_1$-$C_4$ thio, un alcoxy en $C_2$-$C_5$ carbonyle, un halogène, CN, $NO_2$, COOH ou $NH_2$.

**13.** Procédé pour la préparation d'un composé de formule I selon la revendication 2, dans laquelle $R_2$ représente le méthyle, l'éthyle, l'isopropyle ou le sec-butyle;
$R_1$ représente le groupe

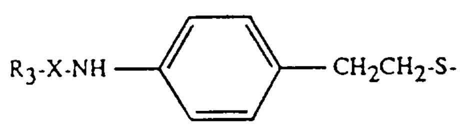

X représentant -C(O)-, -OC(O)- ou -NHC(O)-; et
$R_3$ représentant le groupe

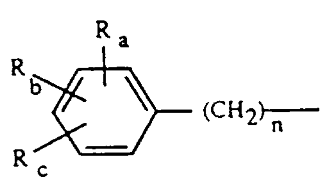

n étant égal à 0 ou 1 et
$R_a$, $R_b$ et $R_c$ représentant, indépendamment les uns des autres, l'hydrogène, un alkyle en $C_1$-$C_2$, un alcoxy en $C_1$-$C_2$, un alkyle en $C_1$-$C_2$ thio, un haloalkyle en $C_1$-$C_2$, un haloalcoxy en $C_1$-$C_2$, un haloalkyle en $C_1$-$C_2$ thio, un halogène ou $NO_2$ et
$R_c$ représentant en plus le groupe

$Het_2$-O-

$Het_2$ représentant l'un des groupes a') à f') définis dans la revendication 5 pour Het et les autres substituants étant définis comme dans la revendication 2.

**14.** Procédé pour la préparation d'un composé de formule I selon la revendication 1, dans laquelle $R_2$ représente le méthyle, l'éthyle, l'isopropyle ou le sec-butyle;
$R_1$ représente le groupe

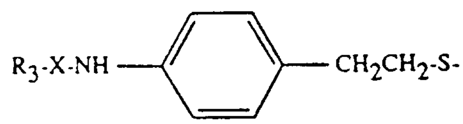

X représentant -C(O)-, -OC(O)- ou -NHC(O)-; et $R_3$ représentant le groupe

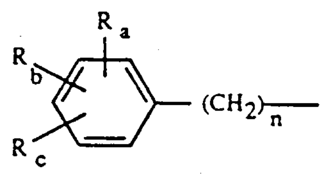

n étant égal à 0 ou 1 et
$R_a$, $R_b$ et $R_c$ représentant, indépendamment les uns des autres, l'hydrogène, un alkyle en $C_1$-$C_2$, un alcoxy en $C_1$-$C_2$, un alkyle en $C_1$-$C_2$ thio, un haloalkyle en $C_1$-$C_2$, un haloalcoxy en $C_1$-$C_2$, un haloalkyle en $C_1$-$C_2$ thio, un halogène ou $NO_2$.

**15.** Procédé pour la préparation d'un composé de formule I selon la revendication 1, dans laquelle $R_2$ représente le méthyle ou l'éthyle;
$R_1$ le groupe

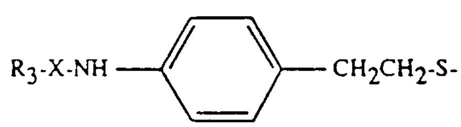

X représentant -OC(O)- et $R_3$ un alkyle en $C_1$-$C_6$.

**16.** Procédé pour la préparation d'un composé de formule I selon la revendication 1, choisi dans le groupe
la 13$\beta$-[2-(4-acétaminophényl)éthylthio]milbémycine A4;
la 13$\beta$-(2-[4-(4-heptylcarbonylamino)phényl]éthylthio)milbémycine A4;
la 13$\beta$-(2-[4-(2,6-dichloropyridin-4-ylcarbonylamino)phényl]éthylthio)milbémycine A4;
la 13$\beta$-(2-[4-tosylphényl]éthylthio)milbémycine A4;
la 13$\beta$-(2-[4-(méthylaminothiocarbonylamino)phényl]éthylthio)milbémycine A4;
la 13$\beta$-(2-[4-(benzoylaminocarbonylamino)phényl]éthylthio)milbémycine A4;
la 13$\beta$-(2-[4-(cyclohexylaminocarbonylamino)phényl]éthylthio)milbémycine A4;
la 13$\beta$-(2-[4-((4-méthoxyphényl)aminocarbonylamino)phényl]éthylthio)milbémycine A4;
la 13$\beta$-(2-[4-(carboxyméthylaminocarbonylamino)-phényl]éthylthio)milbémycine A4; et
la 13$\beta$-(2-[4-(méthoxycarbonylamino)phényl]éthylthio)milbémycine A4.